# EUROPEAN PATENT SPECIFICATION

(11) **EP 1 343 561 B1**
(45) Date of publication and mention of the grant of the patent: **28.02.2007**
(21) Application number: 01270360.9
(22) Date of filing: 12.12.2001
(51) Int. Cl.: A61P 7/02, A61K 31/428, A61K 31/381, C07D 277/46, C07D 277/82, C07D 333/78, C07D 333/66, C07D 409/12, C07D 333/68, C07D 495/04, C07D 417/12, C07D 409/04, C07D 417/14

(54) **SERINE PROTEASE INHIBITORS**
INHIBITOREN VON SERIN PROTEASEN
INHIBITEURS DE SERINE PROTEASE

(30) Priority: 13.12.2000 WO PCT/GB00/04764; 12.06.2001 GB 0114185
(43) Date of publication of application: 17.09.2003
(73) Proprietor: Tularik Limited, London, EC4A 1JP (GB)
(72) Inventor: LIVELY, Sarah, Elizabeth, Congleton, Cheshire CW12 3PJ (GB); HARRISON, Martin, James, Manchester M20 6TG (GB); NAYLOR, Neil, Jason, Macclesfield, Cheshire SK10 2DS (GB); FARTHING, Christopher, Neil, Macclesfield, Cheshire SK8 6TU (GB); WASZKOWYCZ, Bohdan, Wilmslow, Cheshire SK9 4AL (GB)
(74) Representative: Hay, Martin Alexander
(86) International application number: PCT/GB2001/005526
(87) International publication number: WO 2002/047762

(56) References cited:
- WO-A-00/76970
- WO-A-00/77027
- WO-A-01/44226
- WO-A-99/55661
- WO-A1-01/96305
- WO-A1-99/11658
- DATABASE CA [Online] CHEMICAL ABSTRACTS SERVICE, COLUMBUS, OHIO, US; DORMAN, DOUGLAS E. ET AL: "Isolation and Structure Elucidation of the Major Degradation Products of Cefaclor in the Solid State" retrieved from STN Database accession no. 126:282631 XP002190059 & J. PHARM. SCI. (1997), 86(5), 540-549 ,
- CHEMICAL ABSTRACTS, vol. 62, no. 1, 4 January 1965 (1965-01-04) Columbus, Ohio, US; abstract no. 473c, MOSES ET AL: "Hydrogenation of alpha-oximino and alpha--phenylhydrazonophenylacetamides" page 473; XP002190058 & ARKIV KEMI, vol. 22, no. 33, 1964, pages 451-467,
- DATABASE CA [Online] CHEMICAL ABSTRACTS SERVICE, COLUMBUS, OHIO, US; AL-OBAID, A. M. ET AL: "Synthesis and biological evaluation of new cyclopenteno[b]thiophene derivatives as local anesthetic and antiarrhythmic agents" retrieved from STN Database accession no. 128:180295 XP002190060 & PHARMAZIE (1998), 53(1), 24-28 ,

## Description

This invention relates to compounds which are inhibitors of the serine protease, tryptase, to pharmaceutical compositions thereof and to their use in the treatment of the human or animal body. More particularly it relates to compounds for use in the treatment of mast cell mediated diseases such as asthma and other allergic and inflammatory conditions, to pharmaceutical compositions thereof and to their use in the treatment of the human or animal body.

Asthma, the most prevalent of all mast cell mediated conditions affects about 5% of the population in industrialised countries and there is evidence that its incidence and severity are on the increase. Furthermore, the incidence of childhood asthma is rising and there are suggestions of a link between environmental pollutants and the onset of the disease.

Initially, it was believed that bronchoconstriction, i.e. the narrowing of the airways in the lungs, was the major feature of asthma. However, it is now recognised that inflammation in the lungs is an integral part of the development of the disease.

The inhalation of an allergen by an asthmatic generates a strong immune system response which triggers release of various inflammatory mediators, including histamine and leukotrienes from inflammatory cells. These increase the permeability of the blood vessel walls, attract inflammatory cells into the tissues and contract the smooth muscle around the airways. As a result, fluid leaks from the blood and the tissues swell, further narrowing the airways. The inflammatory cells cause damage to the epithelial cells lining the airways exposing nerve endings which stimulates secretion of mucous as well as augmenting the inflammation by causing the release of neurokinins.

Thus asthma is a complex disease frequently characterised by progressive developments of hyper-responsiveness of the trachea and bronchi as a result of chronic inflammation reactions which irritate the epithelium lining the airway and cause pathological thickening of the underlying tissues.

Leukocytes and mast cells are present in the epithelium and smooth muscle tissue of the bronchi where they are activated initially by binding of specific inhaled antigens to IgE receptors. Activated mast cells release a number of preformed or primary chemical mediators of the inflammatory response in asthma as well as enzymes. Moreover, secondary mediators of inflammation are generated by enzymatic reactions of activated mast cells and a number of large molecules are released by degranulation of mast cells.

It has therefore been proposed that chemical release from mast cells probably accounts for the early bronchiolar constriction response that occurs in susceptible individuals after exposure to airborne allergens. The early asthmatic reaction is maximal at around 15 minutes after allergen exposure, recovery occurring over the ensuing 1 to 2 hours. In approximately 30% of individuals, the early asthmatic reaction is followed by a further decline in respiratory function which normally begins within a few hours and is maximal between 6 and 12 hours after exposure. This late asthmatic reaction is accompanied by a marked increase in the number of inflammatory cells infiltrating bronchiolar smooth muscle and epithelial tissues, and spilling into the airways. These cells are attracted to the site by release of mast cell derived chemotactic agents.

The most straightforward way of dealing with an asthma attack is with a bronchodilator drug which causes airways to expand. The most effective bronchodilators are the β-adrenergic agonists which mimic the actions of adrenalin. These are widely used and are simply administered to the lungs by inhalers. However, bronchoconstrictor drugs are primarily of use in short term symptomatic relief, and do not prevent asthma attacks nor deterioration of lung function over the long term.

Anti-inflammatory drugs such as cromoglycate and the corticosteroids are also widely used in asthma therapy. Cromoglycate has anti-inflammatory activity and has been found to be extremely safe. Although such cromolyns have minimal side effects and are currently preferred for initial preventive therapy in children, it is well known that they are of limited efficacy.

The use of corticosteroids in asthma therapy was a major advance since they are very effective anti-inflammatory agents, however, steroids are very powerful, broad spectrum anti-inflammatory agents and their potency and non-specificity means that they are seriously limited by adverse side effects. Localising steroid treatment to the lungs using inhaler technology has reduced side effects but the reduced systemic exposure following inhalation still results in some undesirable effects. Hence, there is a reluctance to use steroids early in the course of the disease.

There therefore still remains a need for an alternative asthma therapy which is a safe, effective, anti-inflammatory or immunomodulatory agent which can be taken to treat chronic asthma.

Tryptase is the major secretory protease of human mast cells and is proposed to be involved in neuropeptide processing and tissue inflammation. Tryptase is one of a large number of serine protease enzymes which play a central role in the regulation of a wide variety of physiological processes including coagulation, fibrinolysis, fertilization, development, malignancy, neuromuscular patterning and inflammation. Although a large number of serine proteases have been widely investigated, tryptase still remains relatively unexplored.

Mature human tryptase is a glycosylated, heparin-associated tetramer of catalytically active subunits. Its amino-acid structure appears to have no close counterpart among the other serine proteases which have been characterised. Tryptase is stored in mast cell secretory granules and after mast cell activation, human tryptase can be measured readily in a variety of biological fluids. For example, after anaphylaxis, tryptase appears in the blood stream where it is readily detectable for several hours. Tryptase also appears in samples of nasal and lung lavage fluid from atopic subjects challenged with specific antigen.

Tryptase has been implicated in a variety of biological processes where activation and degranulation of mast cells occur. Accordingly, mast cell tryptase inhibition may be of great value in the prophylaxis and treatment of a variety of mast cell mediated conditions. Mast cells can degranulate by both IgE-dependent and independent mechanisms thereby implicating tryptase in both atopic and non-atopic inflammatory conditions. Tryptase can activate proteases such as pro-urokinase and pro-MMP3 (pro-matrix metalloprotease 3, pro-stromelysin), thereby indicating a pathological role in tissue inflammation and remodelling. Furthermore, the recent evidence that tryptase can activate certain G-protein coupled receptors (eg PAR2) and induce neurogenic inflammation points to a broader physiological role, for example in modulating pain mechanisms. Given tryptase's multiple mechanisms of action, it has been proposed that tryptase inhibitors may be beneficial in a broad range of diseases. These include conditions such as: asthma (specifically influencing the inflammatory component, the underlying hyperreactivity, and the chronic fibrotic damage due to smooth muscle thickening); chronic obstructive pulmonary disease (COPD) and pulmonary fibrotic diseases; rhinitis; psoriasis; urticaria; dermatitis; arthritis; Crohn's disease; colitis; angiogenesis; atherosclerosis; multiple sclerosis; interstitial cystitis; migraine headache; neurogenic inflammation and pain mechanisms; wound healing; cirrhosis of the liver; Kimura's disease; pre-eclampsia; bleeding problems associated with menstruation and the menopause; cancer (particularly melanoma and tumour metastasis); pancreatitis; and certain viral infections (Yong, Exp. Toxic Pathol, 1997, 49, 409; Steinhoff et al., Nat. Med., 2000, 6, 151; Downing and Miyan, Immunol. Today, 2000, 21, 281; Tetlow and Wooley, Ann. Rheum. Dis., 1995, 54, 549; Jeziorska, Salamonsen and Wooley, Biol. Reprod., 1995, 53, 312; Brain, Nat. Med., 2000, 6, 134; Olness et al., Headache, 1999, 39, 101.) The underlying principle is that a tryptase inhibitor should have utility where mast cells have being induced to degranulate by whatever mechanism, including anaphylactic reactions due to exogenous substances, e.g. morphine-induced bronchoconstriction (Bowman and Rand, Textbook of Pharmacology, 2^{nd} edt., 1980.)

In WO96/09297, WO95/32945, WO94/20527 and US 5,525,623 a variety of peptide based compounds are suggested as potential inhibitors of the mast cell protease tryptase. In WO95/03333 a tryptase inhibitor is provided by a polypeptide obtainable from the leech *hirudo medicinalis.* In WO96/08275 secretory leukocyte protease inhibitor (SLPI) and active fragments thereof have been found to inhibit the proteolytic activity of tryptase. In WO99/55661 certain 4-aminomethylbenzoic ester derivatives are proposed as potential tryptase inhibitors.

WO 99/11658 discloses at page 2, lines 20 to 25 that certain meta-benzamidine derivatives have been found to be particularly effective as inhibitors of serine proteases, especially proteases with negatively charged P1 specificity pockets, and most especially the serine proteases thrombin, trypsin, urokinase and Factor Xa. It further states, at page 5, lines 19 to 22, that "In the compounds of formula (I) it is envisaged that the unsubstituted or substituted amidino group could be replaced by a substituted or unsubstituted aminomethyl group although an amidino group is preferred.

We have now found that certain aromatic compounds carrying certain lipophilic side chains are particularly effective as inhibitors of the serine protease, tryptase. Certain of these compounds have further been found to have good bioavailability.

It is envisaged that the compounds of the invention will be useful not only in the treatment and prophylaxis of asthma but also of other allergic and inflammatory conditions mediated by tryptase such as allergic rhinitis, skin conditions such as eczema, psoriasis, atopic dermatitis and urticaria, rheumatoid arthritis, conjunctivitis, inflammatory bowel disease, neurogenic inflammation, atherosclerosis and cancer.

Thus viewed from one aspect the invention provides a tryptase inhibitor compound of formula (I) 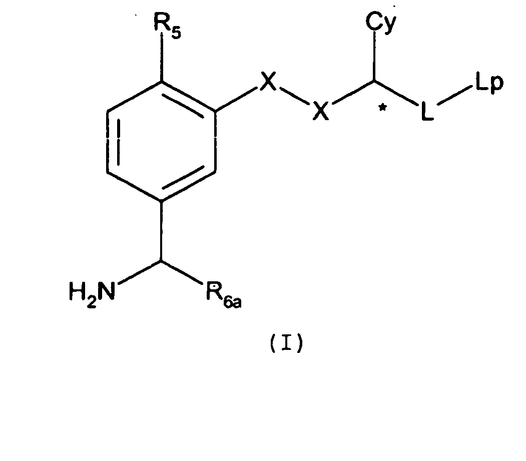 where:
R₅ represents amino, hydroxy, aminomethyl, hydroxymethyl or hydrogen;
R₆ₐ represents hydrogen or methyl;
X-X is -CONR₁ₐ-;
R₁ₐ represents hydrogen, (1-6C)alkyl or phenyl(1-6C)alkyl;
L is CONR_{1d}(CH₂)ₘ in which m is 0 or 1 and R_{1d} is hydrogen, (1-6C)alkyl or phenyl(1-6C)alkyl;
Cy is a saturated or unsaturated, mono or poly cyclic, homo or heterocyclic group, preferably containing 5 to 10 ring atoms and optionally substituted by one or more groups R₃ₐ- or R₃ᵢXᵢ-;
each R₃ₐ independently is hydrogen, hydroxyl, (1-6C) alkoxy, (1-6C) alkyl, (2-6C)alkenyl, (2-6C)alkynyl, (1-6C)alkanoyl, (1-6C) alkylaminoalkyl, hydroxy(1-6C)alkyl, carboxy, (1-6C) alkoxyalkyl, (1-6C) alkoxycarbonyl, (1-6C) alkylaminocarbonyl, amino (1-6C)alkyl, CONH₂, CH₂CONH₂, aminoacetyl, (1-6C)alkanoylamino, hydroxy(1-6C)alkanoylamino, amino(1-6C)alkanoylamino, (1-6C)alkylamino(1-6C)alkanoylamino, di(1-6C)alkylamino(1-6C)alkanoylamino, (1-6C) alkoxycarbonylamino, amino, halo, cyano, nitro, thiol,(1-6C) alkylthio, (1-6C) alkylsulphonyl, (1-6C) alkylsulphenyl, imidazolyl, hydrazido, (1-6C)alkylimidazolyl, (1-6C) alkylsulphonamido, (1-6C) alkylaminosulphonyl, aminosulphonyl, (1-6C) haloalkoxy, or (1-6C) haloalkyl;
Xᵢ is a bond, O, NR₁ₚ, CH₂, CO, CONR₁ₚ, NR₁ₚCO, SO₂, NR₁ₚSO₂ or SO₂NR₁ₚ;
R₃ᵢ is phenyl or pyridyl;
R₁ₚ is as defined for R₁ₐ;
Lp is selected from 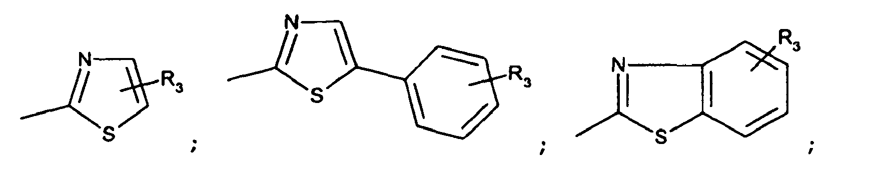 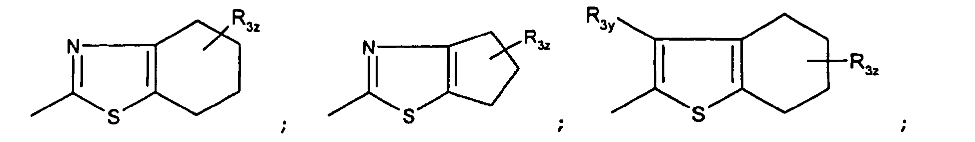 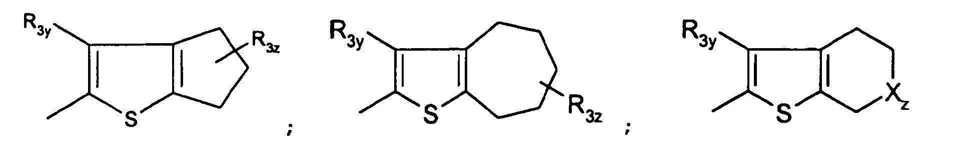 and 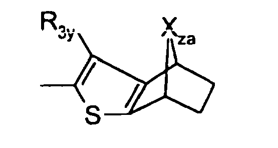 in which
   in which R₃ is an amino acid residue, N-(1-6C)alkylaminocarbonyl,N,N-di(1-6C)alkylaminocarbonyl, N-(1-6C)alkylaminoalkanoyl, N-(1-6C)alkanoylamino(1-6C)alkanonyl, C-hydroxyamino(1-6C)alkanoyl, hydroxy(2-6C)alkanoylamino(1-6C)alkanoyl, di(1-6C)alkylaminosulfonyl, hydrogen, hydroxyl, (1-6C)alkoxy,(1-6C)alkanoyloxy,(1-6C)alkyl,(2-6C)alkenyl (2-6C)alkynyl,(3-6C)alkenyloxycarbonyl,(1-6C)alkanoyl, amino(1-6C)alkyl, amido (CONH₂), amino(1-6C)alkanoyl, aminocarbonyl(1-5C)alkanoyl, hydroxy(1-6C)alkyl, carboxy, hydroxy(1-6C)alkanoyl,(1-6C)alkoxy(1-6C)alkanoyl, (1-6C)alkoxy(1-6C)alkyl, (1-6C)alkoxycarbonyl(1-5C)alkyl, (1-6C)alkoxycarbonyl,(1-6C)alkanoylamino, amino, halo, cyano, nitro, thiol,(1-6C) alkylthio, (1-6C)alkylsulfonyl,(1-6C)alkylsulphenyl or hydrazido;
R_{3y} represents R₃ or a group of formula

   Rₖ-G₂-Xₐ-

   in which G₂ is absent or represents (1-3C)alkanediyl, Xₐ is absent or represents O, S, SO, SO₂ NR_{L}, CO, OCO, COO, CONR_{L}, NR_{L}CO, SO₂NR_{L} or NR_{L}SO₂; Rₖ represents a carbocyclic or heterocyclic group, optionally substituted by R₃; and R_{L} represents hydrogen or (1-6C)alkyl.
R_{3z} is oxo or is as defined for R_{3y}, X_{za} is CH₂ and X_{z} is O, S or NR_{z} in which R_{z} has a value independently selected from a value for R_{3y};
or a physiologically tolerable salt thereof, e.g. a halide, phosphate or sulphate salt or a salt with ammonium or an organic amine such as ethylamine or meglumine.

Compounds of formula I have surprisingly been found to be particularly effective as inhibitors of tryptase and to show a surprising selectivity for tryptase over other serine proteases.

According to another aspect, the present invention provides a compound of formula (I) as defined hereinabove, or a physiologically acceptable salt thereof, in which:
each R₃ₐ independently is hydrogen, hydroxyl, (1-6C) alkoxy, (1-6C) alkyl, (2-6C)alkenyl, (2-6C)alkynyl, (1-6C)alkanoyl, (1-6C) alkylaminoalkyl, hydroxy(1-6C)alkyl, carboxy, (1-6C) alkoxyalkyl,(1-6C) alkoxycarbonyl, (1-6C) alkylaminocarbonyl, amino(1-6C)alkyl, CONH₂, CH₂CONH₂, aminoacetyl, (1-6C)alkanoylamino, (1-6C) alkoxycarbonylamino, amino, halo, cyano, nitro, thiol, 1-6C) alkylthio, (1-6C) alkylsulphonyl, (1-6C) alkylsulphenyl, imidazolyl, hydrazido, (1-6C)alkylimidazolyl, (1-6C) alkylsulphonamido, (1-6C) alkylaminosulphonyl, aminosulphonyl, (1-6C) haloalkoxy, or (1-6C) haloalkyl;
Xᵢ is a bond, O, NH or CH₂; and R₃ᵢ is phenyl optionally substituted by R₃ₐ;
Lp is selected from 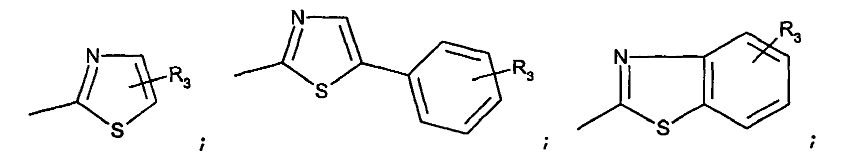 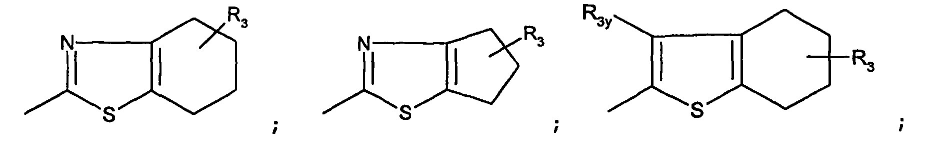 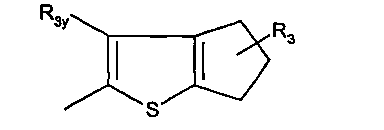
R₃ is an amino acid residue, N-(1-6C)alkylaminocarbonyl, N,N-di(1-6C)alkylaminocarbonyl, N-(1-6C)alkylaminoalkanoyl, N-(1-6C)alkanoylamino(1-6C)alkanonyl, C-hydroxyamino(1-6C)alkanoyl, hydroxy(2-6C)alkanoylamino(1-6C)alkanoyl, di(1-6C)alkylaminosulfonyl, hydrogen, hydroxyl, (1-6C)alkoxy, (1-6C)alkanoyloxy, (1-6C) alkyl, (2-6C)alkenyl (2-6C)alkynyl, (3-6C)alkenyloxycarbonyl, (1-6C)alkanoyl, amino(1-6C) alkyl, amido (CONH₂), amino(1-6C)alkanoyl, aminocarbonyl(1-5C)alkanoyl, hydroxy(1-6C)alkyl, carboxy, hydroxy(1-6C)alkanoyl, (1-6C)alkoxy(1-6C)alkyl, (1-6C)alkoxycarbonyl(1-5C)alkyl, (1-6C)alkoxycarbonyl, (1-6C)alkanoylamino, amino, halo, cyano, nitro, thiol, (1-6C) alkylthio, (1-6C)alkylsulfonyl, (1-6C)alkylsulphenyl or hydrazido; and
R_{3y} represents R₃ or a group of formula

   Rₖ-G₂-Xₐ-

   in which G₂ represents a bond or (1-3C)alkanediyl, Xₐ represents a bond, CO, OCO, COO or NHCO, and Rₖ represents a carbocyclic or heterocyclic group, optionally substituted by R₃.

It will be appreciated that when Xₐ and G₂ each represents a bond, then -G₂-Xₐ- represents a bond. Thus the terms absent and a bond are used interchangeably in this specification.

In the compounds of the invention, R₅ preferably represents amino or hydrogen, more preferably hydrogen.

R₆ₐ preferably represents hydrogen.

In the compounds of the invention, the alpha atom (*) preferably has the conformation that would result from construction from a D-α-aminoacid NH₂-CH(Cy)-COOH where the NH₂ represents part of X-X. It will be appreciated that in this specification, the designation D refers to the R configuration.

In the compounds of the invention, unless otherwise indicated, aryl groups preferably contain 5 to 10 ring atoms optionally including 1, 2 or 3 heteroatoms selected from O, N and S; alkyl, alkenyl or alkynyl groups or alkylene moieties preferably contain up to 6 carbons, e.g. C₁₋₆ or C₁₋₃; cyclic groups preferably have ring sizes of 3 to 8 atoms; and fused multicyclic groups preferably contain 8 to 16 ring atoms.

R₁ₐ is preferably hydrogen.

Preferably, the X moiety nearest to the alpha atom is an NH group. The X moiety alpha to the aromatic ring is CO. Thus a particularly preferred linker X-X is -CONH-.

Examples of particular values for R_{1d} are:
hydrogen;
for (1-6C)alkyl: methyl or ethyl; and
for phenyl(1-6C)alkyl: benzyl or phenylethyl.

R_{1d} is preferably hydrogen.

Examples of particular values for L are CONH, CON(CH₃) and CONHCH₂, more preferably CONH or CON(CH₃).

When Lp comprises a carbocyclic Rₖ group, this may be, for example, a non-aromatic or aromatic, mono or polycyclic hydrocarbon group containing up to 25, more preferably up to 10 carbon atoms. The carbocyclic group may thus be, for example, a cycloalkyl, polycycloalkyl, phenyl or naphthyl group, or a cycloalkyl group fused with a phenyl group.

Examples of particular values for a cycloalkyl group are (3-6C) cycloalkyl groups, such as cyclopentyl and cyclohexyl. A cycloalkyl group is preferably unsubstituted or substituted by one group R₃, preferably an amino or alkyl group.

Examples of particular values for a polycycloalkyl group are (6-10C) polycycloalkyl groups, such as bicycloalkyl, for example decalinyl or norbornyl. A polycycloalkyl group is preferably unsubstituted or substituted by one, two or three R₃ groups, for example alkyl such as methyl. An example of a polycycloalkyl group substituted by alkyl is isopinocampheyl.

A phenyl group is preferably unsubstituted or substituted by one or two R₃ groups.

A naphthyl group is preferably unsubstituted or substituted by one R₃ group.

Examples of a cycloalkyl or cycloalkenyl group fused with a phenyl group are indanyl and tetrahydronaphthyl. This group is preferably unsubstituted or substituted by oxo or one or two R₃ groups. Examples of groups substituted by oxo are 1-oxoindan-5-yl, 1-oxo-1,2,3,4-tetrahydronaphth-7-yl and 1-oxo-1,2,3,4-tetrahydro-naphth-6-yl.

When Lp comprises a heterocyclic Rₖ group, this may be, for example, a non-aromatic or aromatic, mono or polycyclic group containing one or two oxygen, nitrogen or sulfur atoms in the ring system, and in total up to 25, more preferably up to 10 ring system atoms.

Examples of a heterocyclic group when it is a non-aromatic monocyclic group are azacycloalkyl groups, such as pyrrolidinyl and piperidinyl; azacycloalkenyl groups, such as pyrrolinyl; diazacycloalkyl groups, such as piperazinyl; oxacycloalkyl groups, such as tetrahydropyranyl; oxaazacycloalkyl groups, such as morpholino; and thiacycloalkyl groups, such as tetrahydrothiopyranyl. A non-aromatic monocyclic group preferably contains 5, 6 or 7 ring atoms and is preferably unsubstituted or substituted by one group R₃.

Examples of a heterocyclic group when it is a non-aromatic polycyclic group are bicyclic groups, such as azacycloalkyl fused with phenyl, for example dihydroindolyl, dihydroisoindolyl, tetrahydroquinolinyl and tetrahydroisoquinolinyl; azacycloalkyl fused with cycloalkyl, such as decahydroisoquinolinyl; and thienyl fused with cycloalkyl, such as tetrahydrobenzo[b]thienyl or 4H-cyclopenta(b)thienyl. Other examples of thienyl fused with cycloalkyl are 4H-cyclohepta(b)thienyl and tetrahydro-4,7-methanobenzo(b)thiophenyl. Further examples of bicyclic groups are thienyl fused with a heteracycloalkyl group, such as 4,5-dihydro-5H-thieno[2,3-c]pyranyl, 4,5-dihydro-5H-thieno[2,3-c]thiopyranyl and tetrahydrothieno[2,3-b]pyridyl.

Examples of a heterocyclic group when it is an aromatic monocyclic group are furyl, pyrrolyl, thienyl, imidazolyl, thiazolyl, pyridyl, pyridazinyl, pyrimidinyl, pyrazinyl and triazinyl, preferably unsubstituted or substituted by one or two R₃ groups.

Examples of a heterocyclic group when it is an aromatic polycyclic group are bicyclic groups such as benzofuryl, quinolinyl, isoquinolinyl, benzothienyl, indolyl and benzothiazolyl.

Examples of particular values for R₃ are:-
for an amino acid residue: N-acetylalaninoyl, serinoyl, threoninoyl, aspartoyl or glutamoyl;
for N-(1-6C)alkylaminocarbonyl: N-(1,3-dimethyl)butylaminocarbonyl;
for N,N-di(1-6C)alkylaminocarbonyl: N-methyl-N-ethylaminocarbonyl;
for N-(1-6C)alkylaminoalkanoyl: N-methylacetyl;
for N-(1-6C)alkanoylamino(1-6C)alkanonyl: 2-N-acetylaminoacetyl, 2-N-acetylaminopropanoyl or 2-N-(2-methylpropanoyl)aminoacetyl;
for C-hydroxyamino(1-6C)alkanoyl: 2-amino-3-hydroxypropanoyl or 2-amino-3-hydroxybutanoyl;
for hydroxy(2-6C)alkanoylamino(1-6C)alkanoyl: 2-hydroxyacetylaminoacetyl;
for di(1-6C)alkylaminosulfonyl: dimethylaminosulfonyl; hydrogen;
hydroxyl;
for (1-6C)alkoxy: methoxy;
for (1-6C)alkanoyloxy: acetoxy;
for (1-6C) alkyl: methyl, ethyl, propyl, 2-propyl or 2,2-dimethylethyl;
for (2-6C)alkenyl: allyl;
for (2-6C)alkynyl: propynyl;
for (3-6C)alkenyloxycarbonyl: allyloxycarbonyl;
for (1-6C)alkanoyl: acetyl, propionyl or isobutyryl;
for amino(1-6C)alkyl: aminomethyl;
amido (CONH₂);
for amino(1-6C)alkanoyl: aminoacetyl (COCH₂NH₂), aminopropionyl (COCH₂CH₂NH₂) or 2-aminopropionyl (COCH(CH₃)NH₂);
for aminocarbonyl(1-5C)alkanoyl: aminocarbonylacetyl;
for hydroxy(1-6C)alkyl: hydroxymethyl or 1-hydroxyethyl; carboxy;
for hydroxy(1-6C)alkanoyl: 2-hydroxyacetyl or 2-hydroxypropanoyl;
for (1-6C)alkoxy(1-6C)alkanoyl: (1-6C)alkoxyacetyl, such as methoxyacetyl;
for (1-6C)alkoxy(1-6C)alkyl: methoxymethyl;
for (1-6C)alkoxycarbonyl(1-5C)alkyl: methoxycarbonylmethyl;
for (1-6C)alkoxycarbonyl: methoxycarbonyl or ethoxycarbonyl;
for (1-6C)alkanoylamino: formylamino or acetylamino;
amino;
for halo: chloro;
cyano;
nitro;
thiol;
for (1-6C)alkylthio: methylthio;
for (1-6C)alkylsulfonyl: methylsulphonyl or ethylsulfonyl;
for (1-6C)alkylsulphenyl((1-6C)alkylSO-): methylsulphenyl; and hydrazido.

Further examples of particular values for R₃ are:-
for N-(1-6C)alkylaminocarbonyl: N-methylaminocarbonyl or N-isobutylaminocarbonyl; and
for N,N-di(1-6C)alkylaminocarbonyl: N,N-dimethylaminocarbonyl
or N,N-diethylaminocarbonyl.

For example, the lipophilic group Lp is selected from 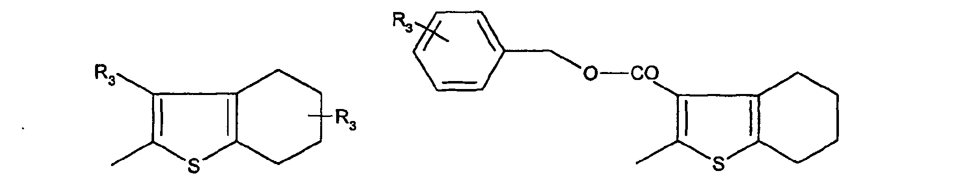 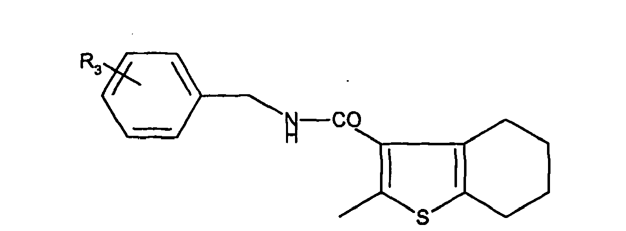 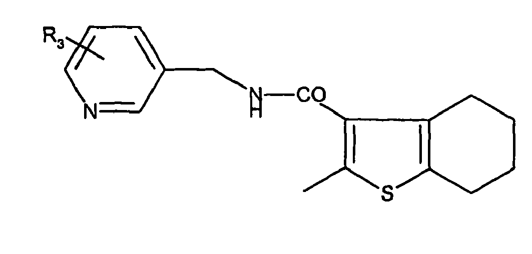 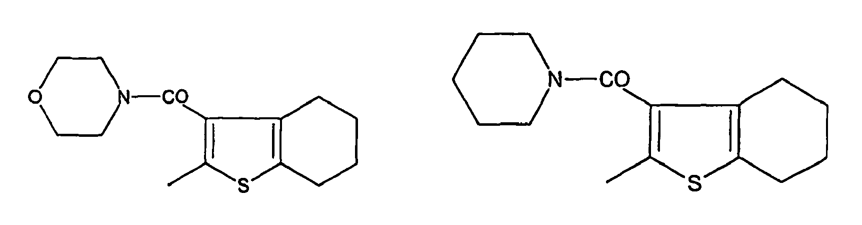 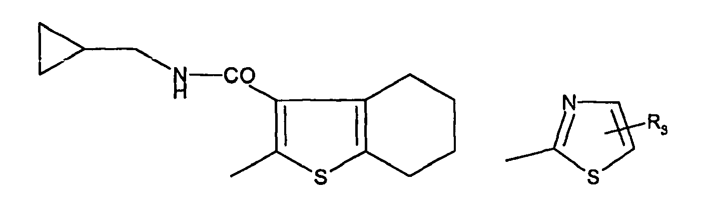 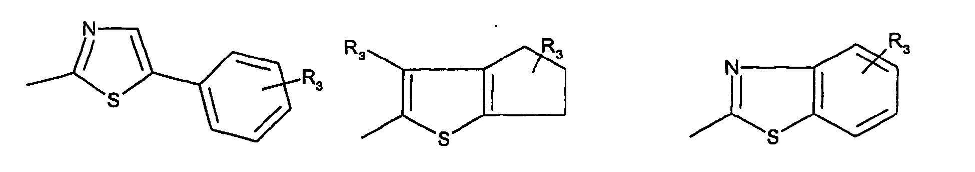 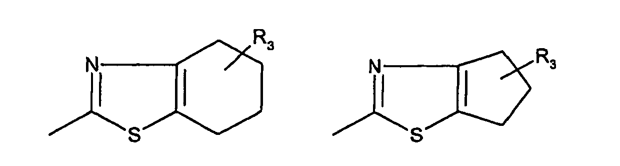 or additionally from 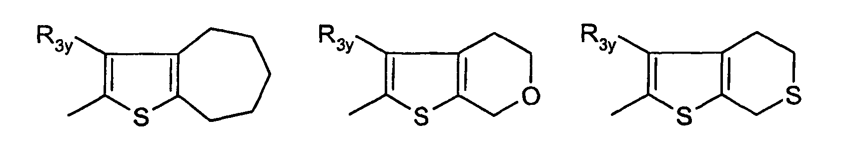 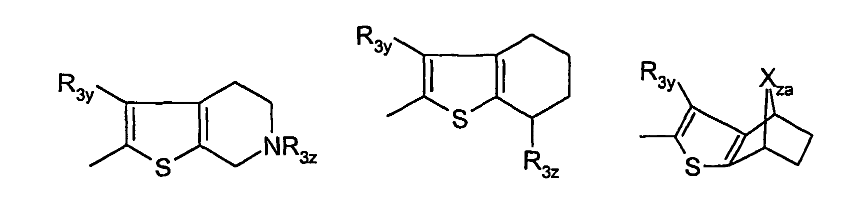 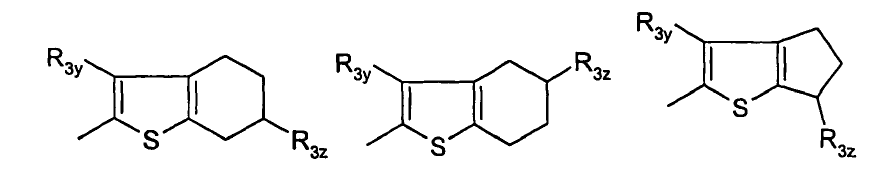 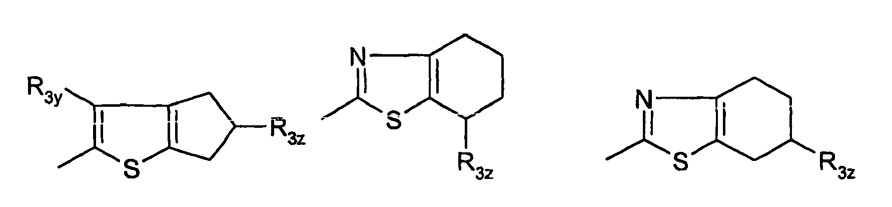 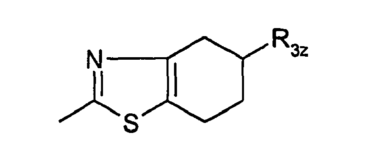 wherein R₃, R_{3y} and R_{3z} are as hereinbefore defined.

Examples of particular values for Lp when it represents a thiazolyl group are: 4-methyl-5-acetylthiazol-2-yl, 4,5-dimethylthiazol-2-yl, 4-methyl-5-ethoxycarbonylthiazol-2-yl, 3-cyano-4-methyl-5-ethoxycarbonylthiazol-2-yl, 4-methoxycarbonylmethyl-5-methylthiazol-2-yl and 5-phenylthiazol-2-yl.

Another particular group of compounds of formula I is that in which L represents CONR_{1d} (such as CONH or CONCH₃) and Lp represents

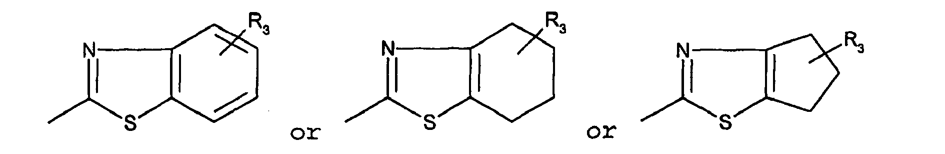

In this group of compounds, the heterocyclic group is preferably substituted by one or two R₃ groups. Each R₃ group is preferably selected from hydrogen, halogen such as chlorine, (1-6C)alkyl, such as methyl, and (1-6C)alkoxy, such as methoxy.

Accordingly, examples of particular values for Lp are: benzothiazol-2-yl, 4-chlorobenzothiazol-2-yl, 4-methylbenzothiazol-2-yl, 6-methylbenzothiazol-2-yl, 4-methoxybenzothiazol-2-yl and 5,6-dimethylbenzothiazol-2-yl. Further examples are 4-methoxy-7-methylbenzothiazol-2-yl, 6-nitrobenzothiazol-2-yl, 4,7-dimethoxybenzothiazol-2-yl, 4,5,6,7-tetrahydrobenzothiazol-2-yl, 5-methyl-4,5,6,7-tetrahydrobenzothiazol-2-yl, 6-methyl-4,5,6,7-tetrahydrobenzothiazol-2-yl, 5-ethyl-4,5,6,7-tetrahydrobenzothiazol-2-yl and 7-ethyl-4,5,6,7-tetrahydrobenzothiazol-2-yl. Further examples are 4-methoxy-7-methylbenzothiazol-2-yl, 6-nitrobenzothiazol-2-yl, 4,7-dimethoxybenzothiazol-2-yl, 4,5,6,7-tetrahydrobenzothiazol-2-yl, 5-methyl-4,5,6,7-tetrahydrobenzothiazol-2-yl, 6-methyl-4,5,6,7-tetrahydrobenzothiazol-2-yl, 5-ethyl-4,5,6,7-tetrahydrobenzothiazol-2-yl and 7-ethyl-4,5,6,7-tetrahydrobenzothiazol-2-yl. Yet further examples are 5-hydroxymethyl-4,5,6,7-tetrahydrobenzothiazol-2-yl, 6-hydroxymethyl-4,5,6,7-tetrahydrobenzothiazol-2-yl and 7-oxo-4,5,6,7-tetrahydrobenzothiazol-2-yl.

Another particular group of compounds of formula I is that in which L represents CONR_{1d} (such as CONH or CONCH₃) and Lp represents 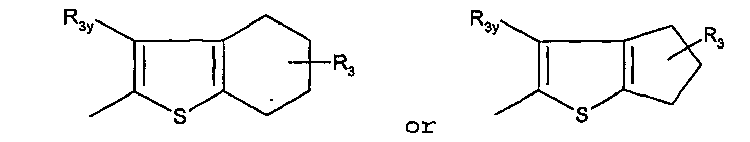 in which R_{3y} represents R₃ or a group of formula

Rₖ-G₂-Xₐ-

in which G₂ represents a bond or (1-3C)alkanediyl, Xₐ represents a bond, CO, OCO, COO or NHCO, and Rₖ represents a carbocyclic or heterocyclic group, optionally substituted by R₃.

Examples of particular values for Rₖ are the examples given above for a carbocyclic or heterocyclic group forming part of Lp. Particular mention may be made of phenyl; cycloalkyl, such as cyclopropyl; azacycloalkyl, such as piperidin-1-yl; oxazacycloalkyl, such as morpholino; and pyridyl, such as pyrid-3-yl. Further particular mention may be made of diazacycloalkyl, such as piperazin-1-yl; furyl, such as fur-2-yl; thienyl, such as thien-2-yl; thiazolyl, such as thiazol-4-yl or thiazol-5-yl, pyrrolidin-1-yl and pyrid-2-yl. Another example is pyrid-4-yl.

Examples of values for G₂ are a bond, -CH₂-, and CH₂CH₂.

Examples of structures of groups comprising a 4,5,6,7-tetrahydrobenzothienyl group as described above are:

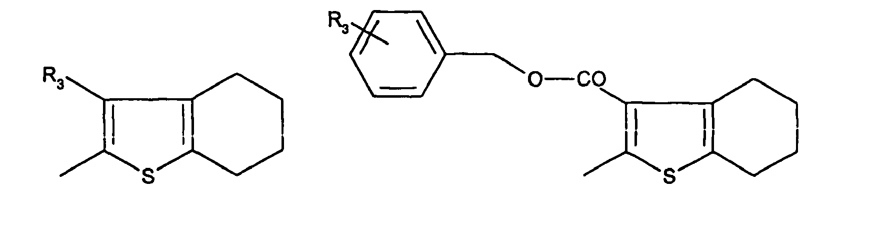

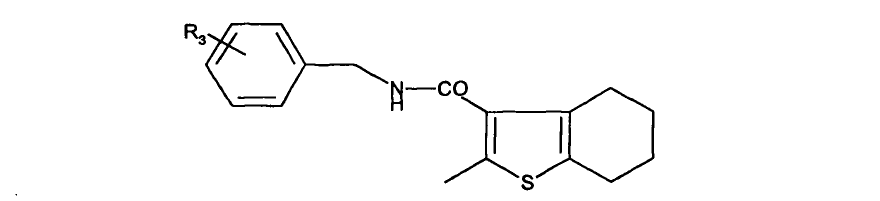

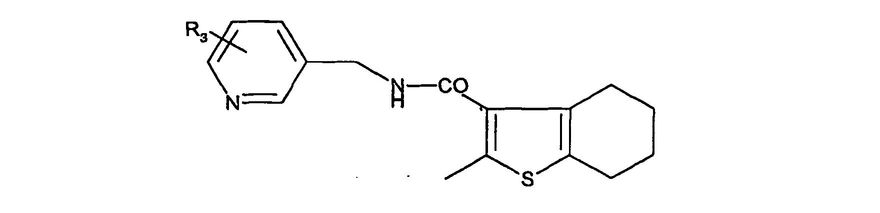

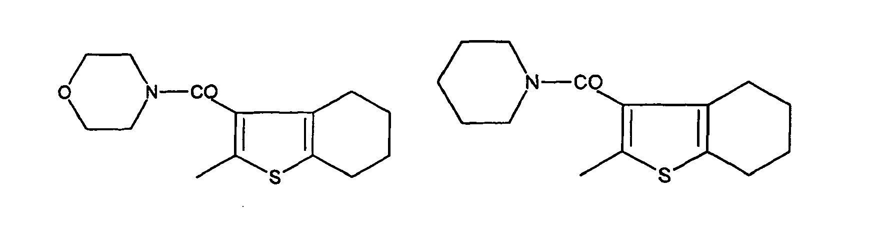

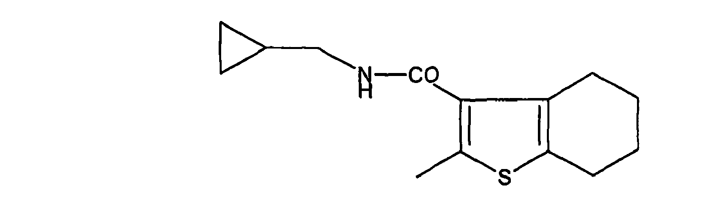

Further examples of structures of groups comprising a 4,5,6,7-tetrahydrobenzothienyl group as described above are:

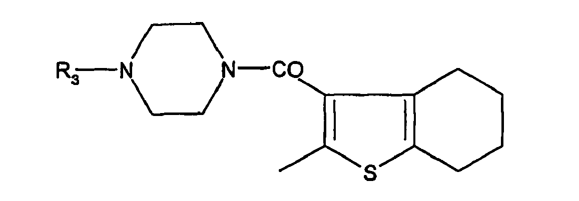

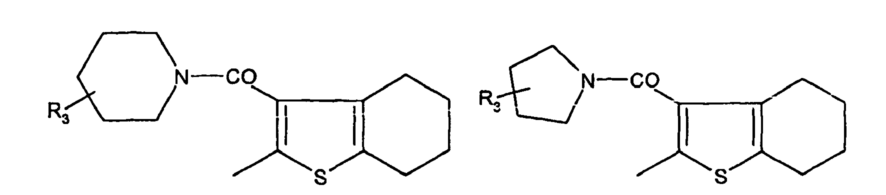

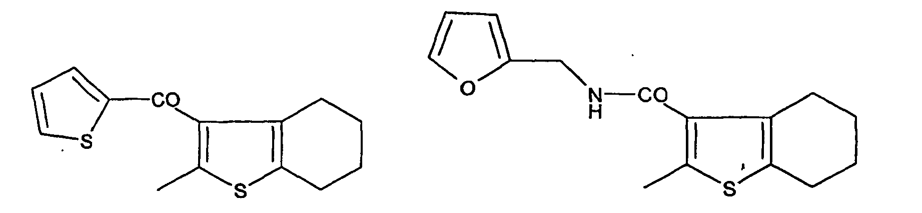

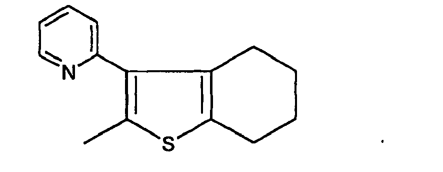

Other examples of structures are:

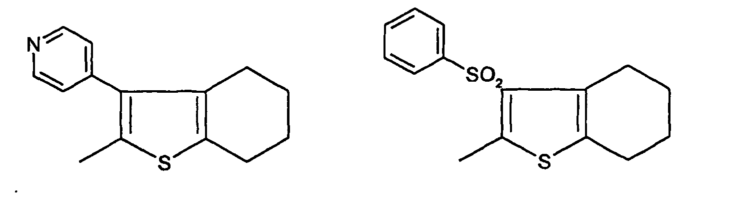

When R₃ is present as a substituent on the 1-position of a piperazinyl group, it is preferably hydrogen, (1-6C)alkanoyl, such as formyl, or (1-6C)alkoxycarbonyl, such as ethoxycarbonyl.

When R₃ is present as a substituent on a piperidin-1-yl group, it is preferably at the 3- or 4-position and is preferably hydrogen, (1-6C)alkyl, such as methyl; amido or (1-6C)alkoxycarbonyl, such as ethoxycarbonyl.

When R₃ is present as a substituent at the 3-position of a 4,5,6,7-tetrahydrobenzothiophene group, it preferably represents a carboxy group; a (1-6C)alkoxycarbonyl group, such as methoxycarbonyl or ethoxycarbonyl; or a (1-6C)alkylaminocarbonyl group, such as N-1,3-dimethylbutylaminocarbonyl. Other examples of values for a (1-6C)alkylaminocarbonyl group are methylaminocarbonyl and isobutylaminocarbonyl.

Accordingly, examples of particular values for Lp are: 3-carboxy-4,5,6,7-tetrahydrobenzothien-2-yl, 3-ethoxy-carbonyl-4,5,6,7-tetrahydrobenzothien-2-yl and 3-N-(1,3-dimethylbutyl)aminocarbonyl-4,5,6,7-tetrahydrobenzothien-2-yl. Further examples are 3-N-methylaminocarbonyl-4,5,6,7-tetrahydrobenzothien-2-yl and 3-N-isobutylaminocarbonyl-4,5,6,7-tetrahydrobenzothien-2-yl.

Further examples of R₃ when it is present as a substituent at the 3-position of a 4,5,6,7-tetrahydrobenzothiophene group are N,N-dialkylaminocarbonyl, such as dimethylaminocarbonyl or diethylaminocarbonyl; amido; (1-6C)alkoxycarbonyl, such a s methoxycarbonyl or ethoxycarbonyl; cyano and (1-6C)alkylsulfonyl, such as methylsulfonyl. Another example of a (1-6C)alkylsulfonyl group is t-butylsulfonyl.

Accordingly, further examples of Lp are 3-N,N-dimethylaminocarbonyl-4,5,6,7-tetrahydrobenzothien-2-yl, 3-N,N-diethylaminocarbonyl-4,5,6,7-tetrahydrobenzothien-2-yl, 3-ethoxycarbonyl-4,5,6,7-tetrahydrobenzothien-2-yl, 3-amido-4,5,6,7-tetrahydrobenzothien-2-yl, 3-methylsulfonyl-4,5,6,7-tetrahydrobenzothien-2-yl, 3-cyano-4,5,6,7-tetrahydrobenzothien-2-yl and 3-ethoxycarbonyl-4H-cyclopenta(b)thienyl. Another example is 3-t-butylsulfonyl-4,5,6,7-tetrahydrobenzothien-2-yl

When R₃ is present as a substituent on a phenyl or pyridyl group, it is preferably a hydrogen atom.

Accordingly, examples of particular values for Lp are: 3-benzyloxycarbonyl-4,5,6,7-tetrahydrobenzothien-2-yl, 3-benzylaminocarbonyl-4,5,6,7-tetrahydrobenzothien-2-yl, 3-(3-pyridyl)methylaminocarbonyl-4,5,6,7-tetrahydro-benzothien-2-yl, 3-cyclopropylmethylaminocarbonyl-4,5,6,7-tetrahydrobenzothien-2-yl, 3-morpholinocarbonyl-4,5,6,7-tetrahydrobenzothien-2-yl and 3-piperidinocarbonyl-4,5,6,7-tetrahydrobenzothien-2-yl. Further examples are: 3-piperazin-1-ylcarbonyl-4,5,6,7-tetrahydrobenzothien-2-yl, 3-(4-formyl)piperazin-1-ylcarbonyl-4,5,6,7-tetrahydrobenzothien-2-yl, 3-(4-ethoxycarbonyl)piperazin-1-ylcarbonyl-4,5,6,7-tetrahydrobenzothien-2-yl, 3-(4-methoxybenzyl)aminocarbonyl-4,5,6,7-tetrahydrobenzothien-2-yl, 3-(4-ethoxycarbonyl)-piperidin-1-ylcarbonyl-4,5,6,7-tetrahydrobenzothien-2-yl, 3-(3-amido)-piperidin-1-ylcarbonyl-4,5,6,7-tetrahydrobenzothien-2-yl, 3-(4-amido)-piperidin-1-ylcarbonyl-4,5,6,7-tetrahydrobenzothien-2-yl, 3-methylpiperidin-1-ylcarbonyl-4,5,6,7-tetrahydrobenzothien-2-yl, 3-(2-thienyl)carbonyl-4,5,6,7-tetrahydrobenzothien-2-yl, 3-(2-furylmethylamino-carbonyl-4,5,6,7-tetrahydrobenzothien-2-yl, 3-(1-pyrrolidinyl)carbonyl-4,5,6,7-tetrahydrobenzothien-2-yl and 3-(2-pyridyl)-4,5,6,7-tetrahydrobenzothien-2-yl.

When R₃ is present as a substituent at the 4,5,6 and/or 7 position of a 4,5,6,7-tetrahydrobenzothien-2-yl group or the 4,5 and/or 6 position of a 4H-cyclopenta(b)thienyl group, it is preferably a hydrogen atom or a (1-6C)alkyl group, such as methyl.

Examples of particular values for Lp are accordingly 3-ethoxycarbonyl-4-methyl-4,5,6,7-tetrahydrobenzothien-2-yl, 3-ethoxycarbonyl-5-methyl-4,5,6,7-tetrahydrobenzothien-2-yl and 3-ethoxycarbonyl-6-methyl-4,5,6,7-tetrahydrobenzothien-2-yl.

Another particular group of compounds of formula I is that in which L represents CONR_{1d} (such as CONH or CONCH₃) and Lp represents: 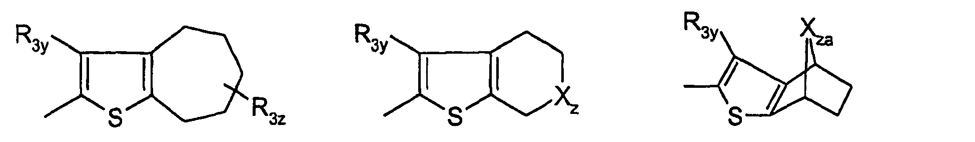 in which R_{3y}, R_{3z}, X_{z} and X_{za} are as defined previously.

Examples of particular values for R_{3y} are (1-6C)alkoxycarbonyl, such as ethoxycarbonyl, N,N-dialkylaminocarbonyl, such as N,N-dimethylaminocarbonyl and cyano.

R_{3z} is preferably hydrogen.

R_{z} in X_{z} is preferably hydrogen, (1-6C) alkanoyl, amino(1-6C)alkanoyl, (1-6C)alkoxy(1-6C)alkanoyl (such as (1-6C)alkoxyacetyl) or benzyloxycarbonyl. Examples of particular values for R_{z} are hydrogen, acetyl, aminoacetyl, methoxyacetyl and benzyloxycarbonyl.

X_{za} is preferably CH₂.

Accordingly, examples of particular values for Lp are: 3-ethoxycarbonyl-tetrahydro-4H-cyclohepta(b)thien-2-yl, 3-ethoxycarbonyl-4,5-dihydro-5H-thieno[2,3-c]pyranyl, 3-ethoxycarbonyl-4,5-dihydro-5H-thieno[2,3-c]thiopyranyl, 3-dimethylaminocarbonyl-6-benzyloxycarbonyltetrahydrothieno[2,3-b]pyridin-2-yl, 3-dimethylaminocarbonyltetrahydrothieno[2,3-b]pyridin-2-yl, 3-dimethylaminocarbonyl-6-acetyltetrahydrothieno[2,3-b]pyridin-2-yl, 3-dimethylaminocarbonyl-6-aminoacetyltetrahydrothieno-[2,3-b]pyridin-2-yl, 3-dimethylaminocarbonyl-6-methoxyacetyl-tetrahydrothieno[2,3-b]pyridin-2-yl and 3-ethoxycarbonyl-tetrahydro-4,7-methanobenzo(b)-thiophen-2-yl.

A preferred sub-group of compounds of formula (I) is that of which L is CONH and Lp is 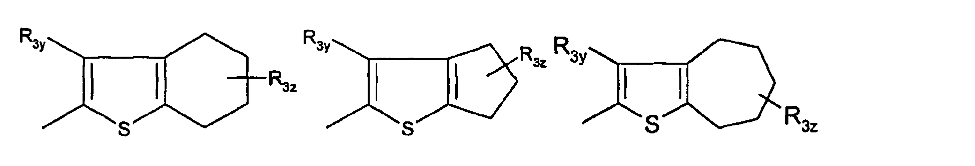 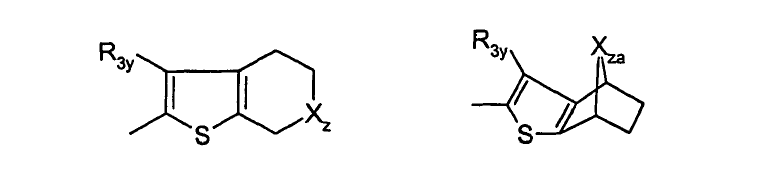 in which R_{3y} is selected from N,N-di-(1-6C)alkylaminocarbonyl, di(1-6C)alkylaminosulfonyl, (3-6C)alkenyloxycarbonyl, (1-6C)alkanoyl, hydroxy(1-6C)alkanoyl, (1-6C)alkoxy(1-6C)alkanoyl, (1-6C)alkoxycarbonyl, (1-6C)alkylsulfonyl, (1-6C)alkylsulphenyl, and Rₖ-G₂-Xₐ in which either Xₐ is CO, OCO, NR_{L}CO, (where R_{L} is (1-6C)alkyl), SO₂ or NR_{L}SO₂ and R_{K} and G₂ are as defined previously, or Xₐ and G₂ are both absent and Rₖ is pyrid-2-yl, thiazol-2-yl, thiazol-4-yl, pyrazin-2-yl, pyrazin-3-yl, pyrimidin-2-yl or pyrimidin-4-yl.

Compounds belonging to this sub-group have been found to exhibit good bioavailability.

The cyclic group attached to the alpha carbon (Cy) preferably represents cycloalkyl (such as cyclohexyl), piperidinyl (such as piperidin-4-yl), phenyl, 3,4-methylenedioxyphenyl, furyl (such as fur-2-yl), thienyl (such as thien-2-yl or thien-3-yl), imidazolyl (such as imidazol-4-yl), oxazolyl (such as oxazol-4-yl or oxazol-5-yl, thiazolyl (such as thiazol-4-yl or thiazol-5-yl), pyridyl (such as pyrid-2-yl, pyrid-3-yl or pyrid-4-yl), pyrimidinyl (such as pyrimidin-2-yl, pyrimidin-4-yl or pyrimidin-5-yl, pyrazinyl (such as pyrazin-2-yl or pyrazin-3-yl), naphthyl (such as naphth-1-yl or naphth-2-yl), indolyl (such as indol-5-yl), indanyl (such as indan-5-yl), 3,4-dihydrobenzofuryl (such as 3,4-dihydrobenzofur-5-yl), benzofuryl (such as benzofur-2-yl) or benzo[b]thienyl group (such as benzo[b]thien-2-yl), optionally substituted by R₃ₐ or R₃ᵢXᵢ.

Examples of particular values for R₃ₐ are:-
hydrogen;
hydroxyl;
for (1-6C)alkoxy: methoxy, ethoxy or isopropoxy;
for (1-6C) alkyl: methyl, ethyl or isopropyl;
for: (1-6C)alkanoyl: acetyl, propanoyl or isopropanoyl,
for (1-6C)alkylaminoalkyl: methylaminomethyl or dimethylaminomethyl;
for (1-6C)hydroxyalkyl: hydroxymethyl;
carboxy;
for (1-6C)alkoxyalkyl: methoxymethyl;
for (1-6C)alkoxycarbonyl: methoxycarbonyl or ethoxycarbonyl; for (1-6C) alkylaminocarbonyl: methylaminocarbonyl or dimethylaminocarbonyl;
for (1-6C) aminoalkyl: aminomethyl;
CONH₂;
CH₂CONH₂;
aminoacetyl;
for (1-6C)alkanoylamino: formylamino or acetylamino;
for hydroxy(1-6C)alkanoylamino: hydroxyacetylamino;
for amino(1-6C)alkanoylamino: aminoacetylamino;
for (1-6C)alkylamino(1-6C)alkanoylamino:(1-6C)alkylacetylamino, such as methylacetylamino;
for di(1-6C)alkylamino(1-6C)alkanoylamino: dimethylaminoacetylamino;
for (1-6C) alkoxycarbonylamino: methoxycarbonylamino, ethoxycarbonylamino or t-butoxycarbonylamino;
amino;
for halo: fluoro, chloro or bromo;
cyano;
nitro;
thiol;
for (1-6C) alkylthio: methylthio;
for (1-6C) alkylsulphonyl: methylsulphonyl or ethylsulphonyl;
for (1-6C)alkylsulphenyl: methylsulphenyl;
for imidazolyl: imidazol-4-yl;
hydrazido;
for (1-6C) alkylimidazolyl: 2-methylimidazol-4-yl;
for (1-6C) alkylsulphonamido: methylsulphonylamido or ethylsulphonylamido;
for (1-6C)alkylaminosulphonyl: methylaminosulphonyl or ethylaminosulphonyl;
aminosulphonyl;
for (1-6C) haloalkoxy: trifluoromethoxy; and
for (1-6C) haloalkyl: trifluoromethyl.

An example of a particular value for R₃ᵢ is phenyl.

Examples of particular values for R₃ᵢXᵢ are phenyl, phenoxy, phenylamino and benzyl.

R₁ₚ is preferably hydrogen.

Cy is preferably unsubstituted or substituted by one or two R₃ₐ groups.

Preferably R₃ₐ is hydrogen, hydroxyl, methyl, ethyl, isopropyl, acetyl, propanoyl, isopropanoyl, isopropoxy, amino, aminomethyl, hydroxymethyl, carboxy, amido, chloro, formylamino, acetylamino, aminoacetyl or carboxy.

Examples of particular values for Cy are cyclohexyl, piperidin-4-yl, 1-acetylpiperidin-4-yl, 1-propanoylpiperidin-4-yl, 1-isobutyrylpiperidin-4-yl, 1-aminoacetylpiperidin-4-yl, phenyl, 2-aminophenyl, 4-aminophenyl, 3-hydroxyphenyl, 4-methylphenyl, 2,4-dimethylphenyl, 3,6-dimethylphenyl, 4-ethylphenyl, 4-isopropylphenyl, 4-hydroxphenyl, 3-aminomethylphenyl, 4-aminomethylphenyl, 4-(H₂NCO)phenyl, 4-hydroxymethylphenyl, 3-hydroxymethylphenyl, 2-hydroxymethylphenyl, 4-carboxyphenyl, 4-isopropoxyphenyl, 2-chlorophenyl, 3,4-methylenedioxyphenyl, 4-phenylphenyl, 4-phenoxyphenyl, 5-methylfur-2-yl, imidazol-4-yl, 2-methylthiazol-4-yl, 2-aminothiazol-4-yl, 2-formylaminothiazol-4-yl, 2-aminothiazol-5-yl, 2-formylaminothiazol-5-yl, 2-phenylthiazol-4-yl, 4-aminopyrid-3-yl, 6-methylpyrid-2-yl, 3-amino-pyrid-4-yl, naphth-1-yl, naphth-2-yl, benzofur-2-yl, 2-methoxyphenyl, 3-methoxyphenyl, 4-methoxyphenyl, indan-5-yl, 2-methylthiophenyl, 3-methylthiophenyl, 3-methylsulfinylphenyl, 2-methylsulphonylphenyl, 3-methylsulphonylphenyl, 3-N,N-dimethylaminophenyl, 2,3-dihydrobenzofuran-5-yl, 3-bromophenyl, 3-cyanophenyl, 2-methoxycarbonylphenyl, 2-ethoxycarbonylphenyl, 2-methylphenyl, 2-fluorophenyl, 6-aminopyrid-3-yl, 2-ethylthiazol-4-yl, 2-benzylthiazol-4-yl, 2-methylsulfonamidothiazol-4-yl, 2-chloropyrid-3-yl, 2-hydroxyacetylaminothiazol-4-yl, 2-N,N-dimethylaminoacetyl-aminothiazol-4-yl, 2-trifluoromethoxyphenyl, 2-trifluoromethylphenyl, 3-chloropyrid-2-yl, 3-methylpyrid-2-yl, pyrazin-2-yl, pyrazin-3-yl, pyrimidin-2-yl and pyrimidin-4-yl.

In one sub-group of compounds of formula (I), the cyclic group attached to the alpha carbon is cycloalkyl (such as cyclohexyl), piperidinyl (such as piperidin-4-yl), phenyl, 3,4-methylenedioxyphenyl, furyl, (such as fur-2-yl), thienyl (such as thien-2-yl or thien-3-yl), imidazolyl (such as imidazol-4-yl), thiazolyl (such as thiazol-4-yl or thiazol-5-yl), pyridyl (such as pyrid-2-yl, pyrid-3-yl or pyrid-4-yl), naphthyl (such as naphth-1-yl or naphth-2-yl), benzofuryl (such as benzofur-2-yl), or benzo[b]thienyl (such as benzo [b] thien-2-yl) group, optionally substituted by R₃ₐ or R₃ᵢXᵢ in which Xᵢ is a bond, O, NH or CH₂ and R₃ᵢ is phenyl optionally substituted by R₃ₐ and each R₃ₐ independently is hydrogen, hydroxyl, (1-6C) alkoxy, (1-6C) alkyl, (1-6C)alkanoyl, (1-6C) alkylaminoalkyl, hydroxy(1-6C)alkyl, carboxy, (1-6C) alkoxyalkyl,(1-6C) alkoxycarbonyl, (1-6C) alkylaminocarbonyl, amino (1-6C) alkyl CONH₂, CH₂CONH₂, aminoacetyl, (1-6C)alkanoylamino, (1-6C) alkoxycarbonylamino, amino, halo, cyano, nitro, thiol, (1-6C) alkylthio, (1-6C) alkylsulphonyl, (1-6C) alkylsulphenyl, imidazolyl, hydrazido, (1-6C)alkylimidazolyl,(1-6C) alkylsulphonamido, (1-6C) alkylaminosulphonyl, aminosulphonyl, (1-6C) haloalkoxy, or (1-6C) haloalkyl.

Within this group, examples of values for R₃ₐ are hydrogen; hydroxyl; methoxy; ethoxy; isopropoxy; methyl; ethyl; isopropyl; acetyl; propanoyl; isopropanoy; methylaminomethyl; dimethylaminomethyl; hydroxymethyl; carboxy; methoxymethyl; methoxycarbonyl; ethoxycarbonyl; methylaminocarbonyl; dimethylaminocarbonyl; aminomethyl; CONH₂; CH₂CONH₂; aminoacetyl; formylamino; acetylamino; methoxycarbonylamino; ethoxycarbonylamino; t-butoxycarbonylamino; amino; fluoro; chloro; cyano; nitro; thiol; methylthio; methylsulphonyl; ethylsulphonyl; methylsulphenyl; imidazol-4-yl; hydrazido; 2-methylimidazol-4-yl; methylsulphonylamido; ethylsulphonylamido; methylaminosulphonyl; ethylaminosulphonyl; aminosulphonyl; trifluoromethoxy or trifluoromethyl; and for R₃ᵢXᵢ are phenyl, phenoxy, phenylamino or benzyl.

Within this group, examples of values for Cy are cyclohexyl, piperidin-4-yl, 1-acetylpiperidin-4-yl, 1-propanoylpiperidin-4-yl, 1-isobutyrylpiperidin-4-yl, 1-aminoacetylpiperidin-4-yl, phenyl, 4-aminophenyl, 3-hydroxyphenyl, 4-methylphenyl, 2,4-dimethylphenyl, 3,6-dimethylphenyl, 4-ethylphenyl, 4-isopropylphenyl, 4-hydroxphenyl, 3-aminomethylphenyl, 4-aminomethylphenyl, 4-(H₂NCO)phenyl, 4-hydroxymethylphenyl, 3-hydroxymethylphenyl, 2-hydroxymethylphenyl, 4-carboxyphenyl, 4-isopropoxyphenyl, 2-chlorophenyl, 3,4-methylenedioxyphenyl, 4-phenylphenyl, 4-phenoxyphenyl, 5-methylfur-2-yl, imidazol-4-yl, 2-methylthiazol-4-yl, 2-aminothiazol-4-yl, 2-formylaminothiazol-4-yl, 2-aminothiazol-5-yl, 2-formylaminothiazol-5-yl, 2-phenylthiazol-4-yl, 4-aminopyrid-3-yl, 6-methylpyrid-2-yl, 3-amino-pyrid-4-yl, naphth-1-yl, naphth-2-yl, benzofur-2-yl or 3-methylbenzothien-2-yl.

A group of compounds of particular interest is that in which Cy is a group of formula 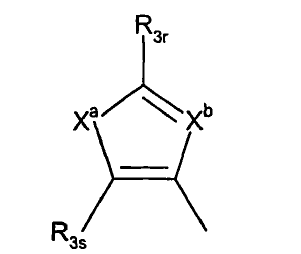 in which one of X^{a} and X^{b} is N and the other is NH or S, and each of R₃ᵣ and R₃ₛ is as defined for R₃ₐ.

Compounds belonging to this sub-group have been found to show good bioavailability.

Preferably X^{a} is S or NH and X^{b} is N.

Preferably R₃ₛ is hydrogen.
Preferably R₃ᵣ is hydrogen, (1-6C)alkyl, amino, (1-6C)alkanoylamino, hydroxy(1-6C)alkanoylamino, N,N-di(1-6C)alkylaminoalkanoylamino, phenyl or benzyl.

Another group of compounds in which good bioavailability has been found are compounds of formula (I) in which Cy is pyrid-2-yl, pyrimidin-2-yl, pyrimidin-4-yl, pyrazin-2-yl, pyrazin-3-yl or oxazol-4-yl optionally substituted by R₃ₐ or R₃ᵢXᵢ.

The compounds of the invention may be prepared by conventional chemical synthetic routes, e.g. by amide bond formation to couple the aromatic function to the alpha atom and to couple the lipophilic function to the alpha atom. The cyclic group-alpha atom combination may conveniently derive from an alpha amino acid (preferably of D (i.e. R) configuration) with the aromatic deriving from for example an acid derivative of a compound based on R₂, e.g. an aminomethylbenzoic acid (which is readily available). Amide formation from such reagents (in which any amino or hydroxyl function (especially in an aminomethyl group) may if desired be protected during some or all of the synthesis steps) yields a compound of formula (V).

R₂-CONH-CH(Cy)-COOH (V)

(where R₂ represents 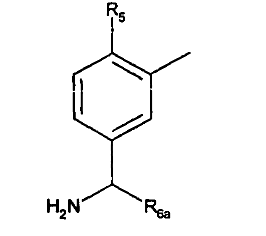 and Cy is as defined above).

Prior to reaction the amino group in an aminoalkyl group should be protected by an appropriate protecting group, PG, e.g. Boc, Z, Fmoc or Bpoc. The use of protecting groups is described in McOmie, "Protective Groups in Organic Chemistry", Plenum, 1973 and Greene, "Protective Groups in Organic Synthesis", Wiley Interscience, 1981.

The lipophilic group may then conveniently be introduced by reaction of a compound of formula (V) (or another analogous carboxylic acid) optionally after transformation into an activated form, e.g. an acid chloride or active ester, with a lipophilic group carrying or containing an amine group to produce a compound with the linkage of -CO- or -CO-NR_{1d}(CH₂)ₘ-from the alpha atom to the lipophilic group. The protecting group, PG, is then removed.

Alternatively a compound of formula V or another analogous carboxylic acid may be transformed into an alcohol by reaction with isobutylchloroformate and reduction with sodium borohydride.

Such an alcohol, e.g. of formula (VI)

R₂ - CONH - CH(Cy)CH₂OH (VI)

can be reacted to introduce the lipophilic group by reactions such as:
oxidation of the alcohol to form a corresponding aldehyde (e.g. by oxidation with manganese dioxide or DMSO/oxalyl chloride or DMSO/SO₃ or Dess-Martin reagent) which may be reacted to introduce the lipophilic group by reactions such as:
   reaction with an organometallic, eg a Grignard reagent, optionally followed by oxidation of the resulting hydroxyl group (e.g. with MnO₂, DMSO/oxalyl chloride or Dess-Martin reagent.

In this way compounds with the linkage of -CO- between the alpha carbon and the lipophilic group may be produced.

An alternative and preferred route to these compounds is to carry out any of the above chemical reactions to incorporate the lipophilic group into a protected intermediate such as a compound of formula (VII). 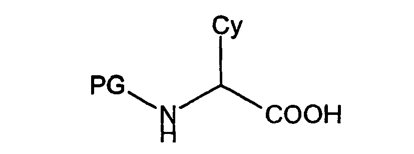 PG = Protecting group

The protecting group may then be removed before coupling of the 3-aminomethylbenzoic acid (optionally protected).

The protection of amino and carboxylic acid groups is described in McOmie, Protecting Groups in Organic Chemistry, Plenum Press, NY, 1973, and Greene and Wuts, Protecting Groups in Organic Synthesis, 2nd. Ed., John Wiley & Sons, NY, 1991. Examples of carboxy protecting groups include C₁-C₆ alkyl groups such as methyl, ethyl, *t*-butyl and *t*-amyl; aryl(C₁-C₄)alkyl groups such as benzyl, 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, benzhydryl and trityl; silyl groups such as trimethylsilyl and *t-*butyldimethylsilyl; and allyl groups such as allyl and 1-(trimethylsilylmethyl)prop-1-en-3-yl.

Examples of amine protecting groups (PG) include acyl groups, such as groups of formula RCO in which R represents C₁₋₆ alkyl, C₃₋₁₀ cycloalkyl, phenyl C₁₋₆ alkyl, phenyl, C₁₋₆ alkoxy, phenyl C₁₋₆ alkoxy, or a C₃₋₁₀ cycloalkoxy, wherein a phenyl group may be optionally substituted, for example by one or two of halogen, C₁-C₄ alkyl and C₁-C₄ alkoxy. Preferred amino protecting groups include t-butoxycarbonyl (Boc) and benzyl.

α-Amino acids of formula (VII) which are not commercially available can be synthesized by methods known in the art, for example as described in "Synthesis of Optically Active α-Amino Acids" by Robert M. Williams (Pergamon Press, 1989) and "Asymmetric Synthesis of ArylGlycines", Chem. Rev. 1992, 889-917.

Compounds of the type (VII) made be prepared (for example) by one or more of the following methods.
(i) from aryl or heteroaryl aldehydes via the Strecker synthesis or modifications thereof, via Bucherer-Bergs hydantoin synthesis, or via the Ugi methodology (Isonitrile Chemistry, Ugi I. Ed.; Academic: New York, 1971; pp145-199) with removal and replacement of protecting groups;
(ii) from styrenes via Sharpless methodology (J. Am. Chem. Soc. 1998,120, 1207-1217)
(iii) from aryl boronic acids via Petasis methodology (Tetrahedron, 1997, 53, 16463-16470) with removal and replacement of protecting groups;
(iv) from aryl and heteroaryl acetic acids - via Evan's azidation (Synthesis, 1997, 536-540) or by oximation, followed by reduction and addition of protecting groups;
(v) from existing aryl glycines by manipulation of functional groups, for example, alkylation of hydroxy groups, palladium assisted carbonylation of triflates derived from hydroxy groups and further manipulation of the carboxylic esters to give carboxylic acids by hydrolysis, carboxamides by activation of the carboxylic acid and coupling with amines, amines via Curtius reaction on the carboxylic acid; or
(vi) from aliphatic, carbocylic and non-aromatic heterocyclic aldehydes and ketones using a Horner-Emmons reaction with N-benzyloxycarbonyl)-α-phosphonoglycine trimethyl ester (Synthesis, 1992, 487-490).

Examples of synthetic schemes are shown below:

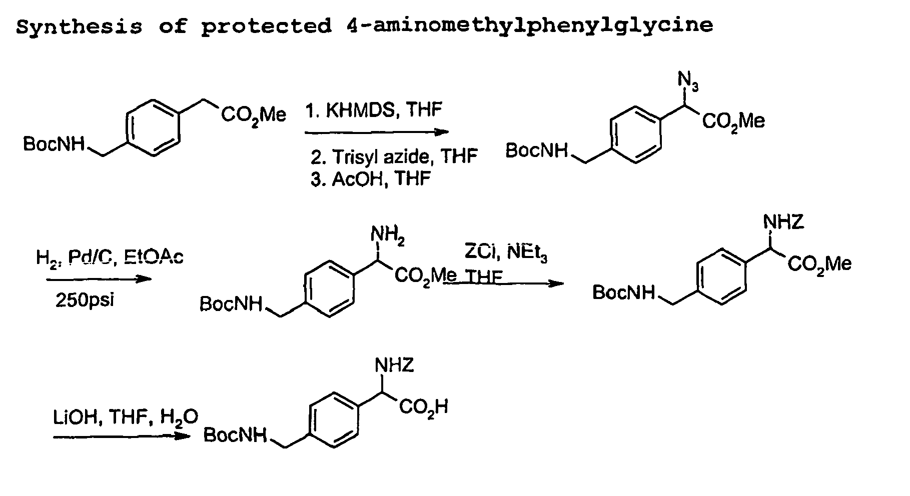

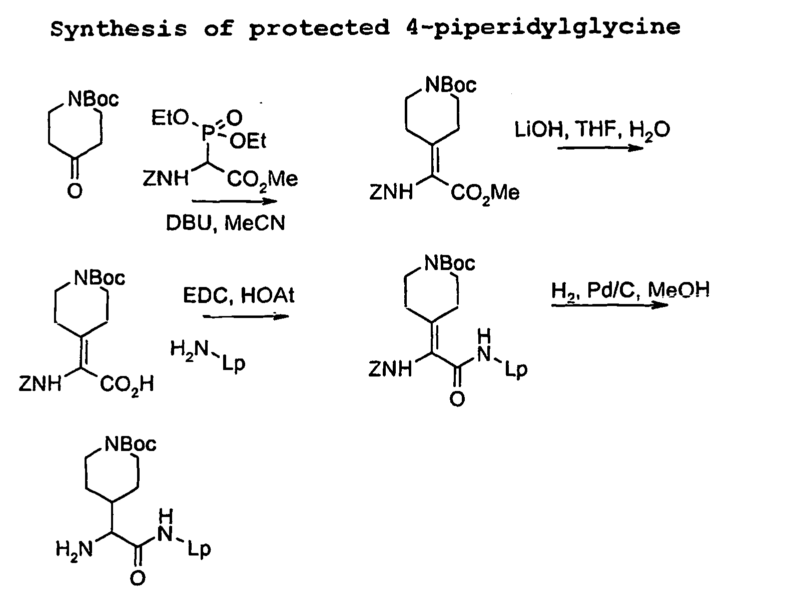

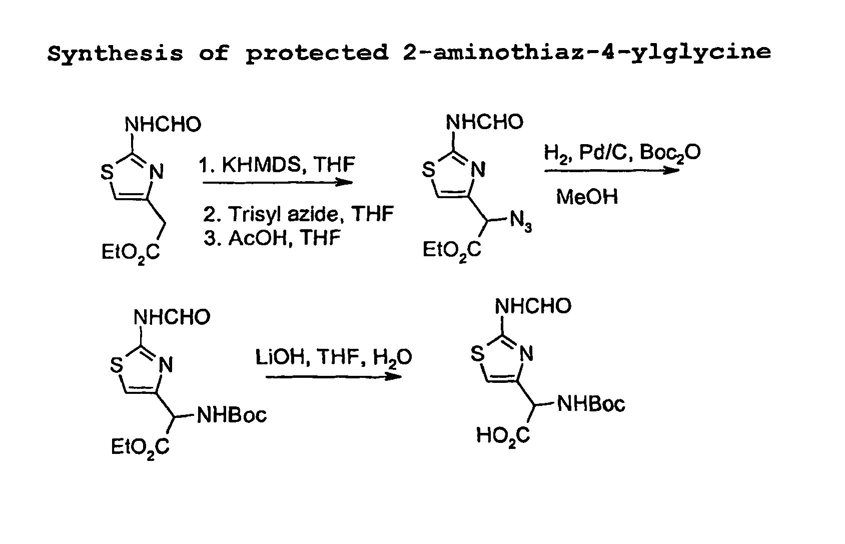

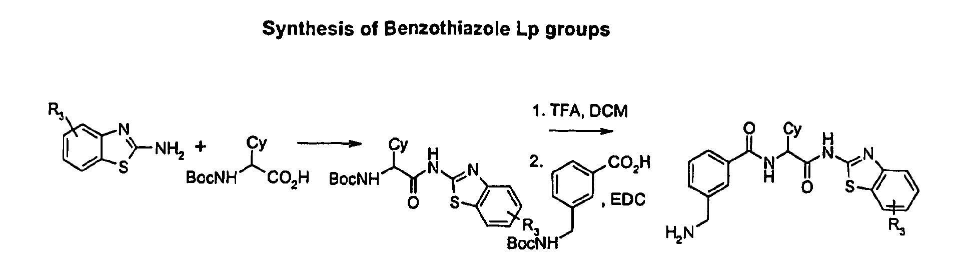

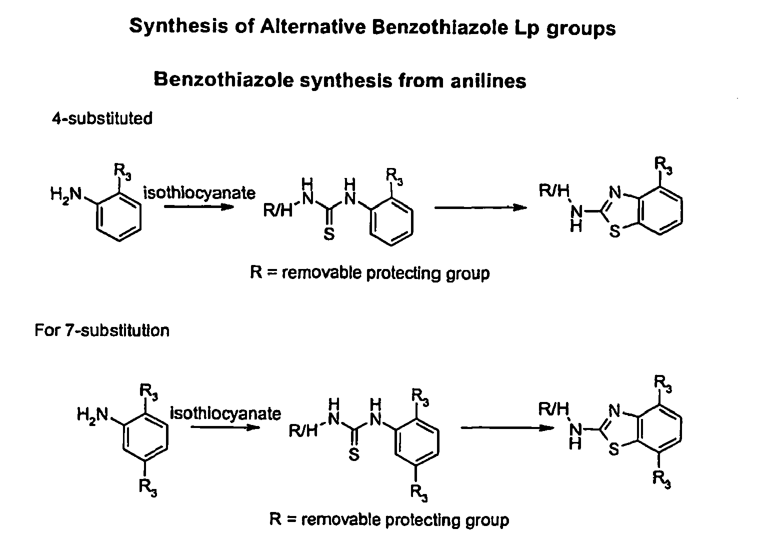

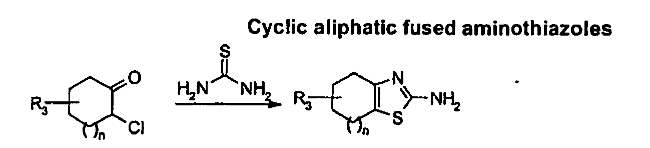

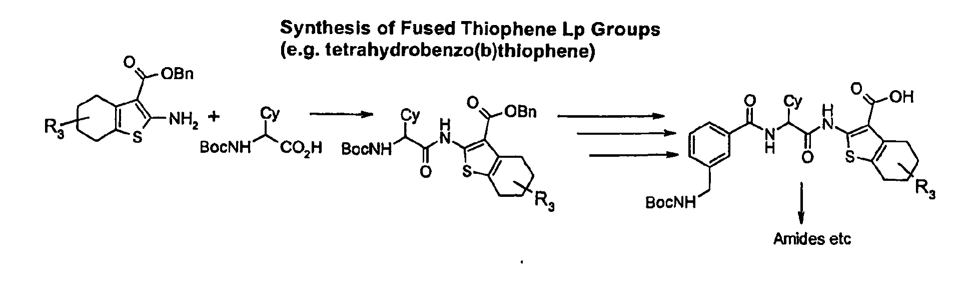

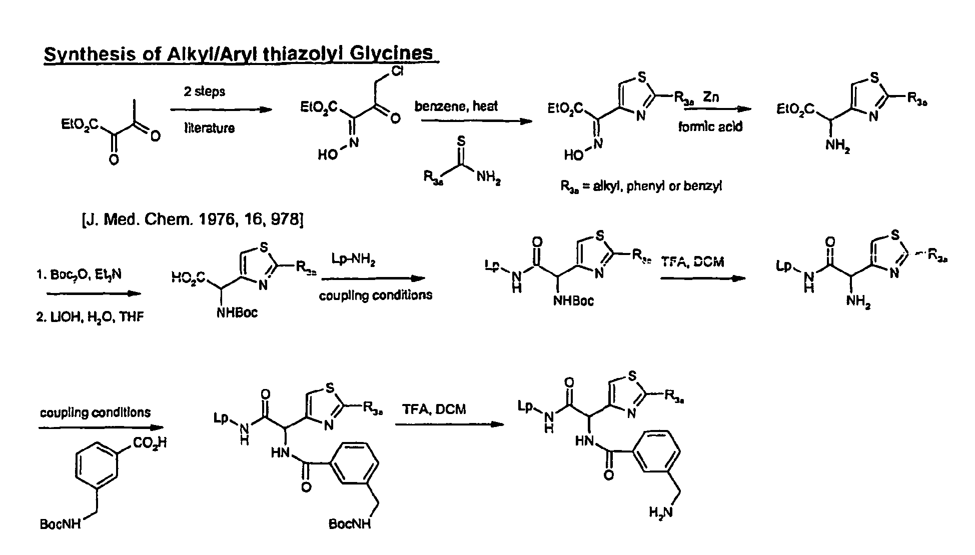

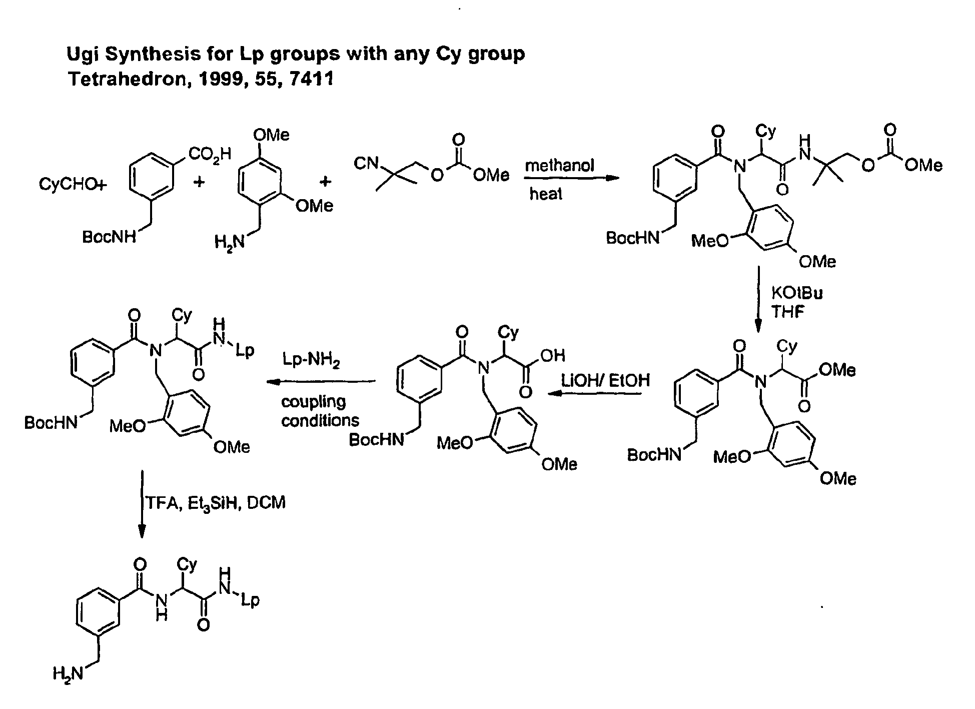

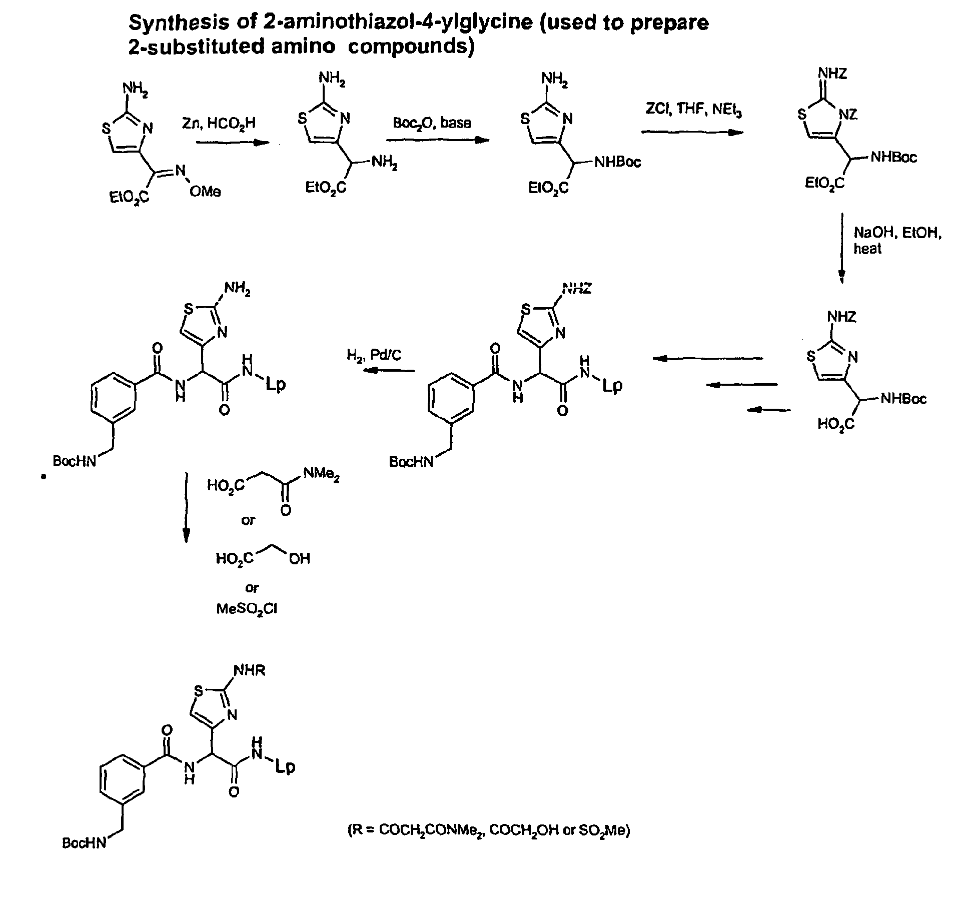

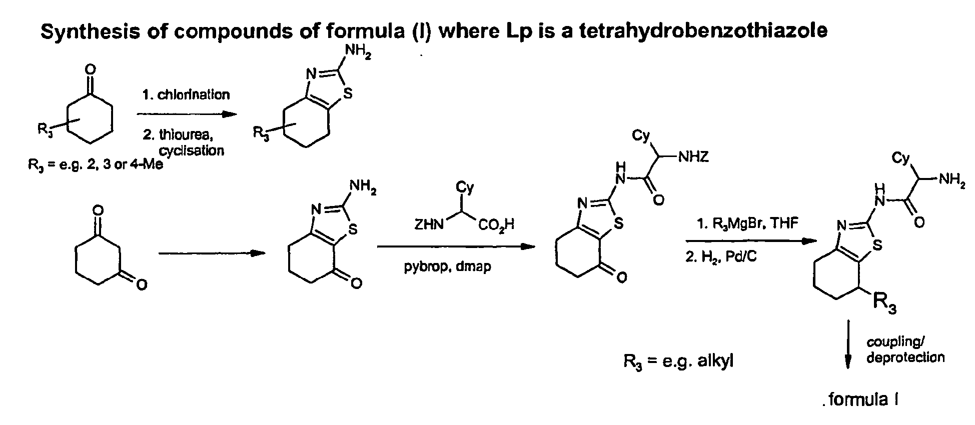

Thus viewed from a further aspect, the invention provides a process for the preparation of a compound according to the invention which process comprises coupling a lipophilic group to a compound of formula (VIII)

R₂-(X)₂-CH(Cy)-Z₁ (VIII)

or a protected derivative thereof (wherein R₂, X and Cy are as defined above and Z₁ is a reactive functional group).

The reactive functional group, Z₁ may be, for example, a carboxyl group; an acid halide, such as a chloride or bromide; an ester, such as a (1-6C)alkoxycarbonyl group, or an activated ester, such as a mixed anhydride or formed *in situ* using a coupling agent such as 1-(3-dimethylaminopropyl)-3-ethylcarbodiimide hydrochloride (EDC). Preferably the lipophilic group is coupled using conditions in which the compound of formula VIII is an acid bromide formed *in situ* by the action of bromo-tris-pyrrolidino-phosphonium hexafluorophosphate (PyBroP). This reaction is conveniently performed under concentrated conditions in dichloromethane using dimethylaminopyridine (DMAP) as catalyst.

Instead of introducing the group L-Lp as the final stage process step, the compounds of formula I may alternatively be prepared by a process in which the group R₂ is introduced in the final process step, which is the preferred route.

Thus viewed from another aspect the invention provides a process for the preparation of a compound according to the invention which process comprises reacting a compound of formula (IX)

Z₂-CH(Cy)-L-Lp (IX)

(wherein Cy, L and Lp are as defined above and Z₂ is HX (e.g. H₂N) or a reactive functional group), or a protected derivative thereof, with a compound of formula (X)

R₂- Z₃ (X)

(wherein R₂ is as defined above and Z₃ is an appropriate reactive group), or a protected derivative thereof, followed if necessary by the removal of any protecting groups. Thus, for a compound of formula I in which X-X represents CONH, a compound of formula (IX) in which Z₂ is H₂N may be reacted with a compounds of formula (X) in which Z₃ is COOH or a reactive derivative thereof, such as an acyl halide or an anhydride, for example as described in the Examples herein.

Certain of the compounds of formula (IX) in which Z₂ is H₂N and their salts are believed to be novel and are provided as a further aspect of the invention.

In another aspect the invention relates to a process for preparing a compound of formula I comprising deprotecting a compound of formula (I') :

R_{2'}-X-X-CH(Cy') -L-Lp (I')

wherein R₂, is R₂ (as hereinabove defined) or protected R₂ (such as 3-N-Boc-aminomethylphenyl), Cy' is Cy (as hereinabove defined) or protected Cy and Lp' is Lp (as hereinabove defined) or protected Lp; providing at least one protecting group is present.

Compounds of formula (I') in which L represents CONH and X-X represents CONR₁ₐ, where R₁ₐ, is an optionally substituted benzyl group, such as 2,4-dimethoxybenzyl, may be prepared using Ugi methodology, starting from an aldehyde of formula CyCHO, an optionally substituted benzylamine of formula R₁ₐ,NH₂, an isonitrile of formula CN-Lp and an N-protected 3-aminomethylbenzoic acid, such as 3-BOC-aminomethylbenzoic acid. The protecting groups may be removed using trifluoroacetic acid. Preferably, rather than starting with an isonitrile of formula CN-Lp, it has been found to be advantageous to start with an isonitrile of formula (XI)

CN-C(CH₃)₂CH₂OCOOR (XI)

in which R represents a (1-6C)alkyl group, such as methyl, to afford a compound of formula (XII)

R^{2'}-CONR^{1a'} -CH (Cy') -CONH-C (CH₃)₂CH₂OCOOR (XII)

The compound of formula (XII) may then be treated with a strong base, such as potassium t-butoxide, to afford a compound of formula (XIII)

R^{2'}-CONR^{1a'}-CH (Cy')-COOR (XIII)

The compound of formula (XIII) may then be hydrolysed, for example by treatment with lithium hydroxide in ethanol, to afford a compound of formula (XIV)

R^{2'}-CONR^{1a'}-CH (Cy') - COOH (XIV)

The compound of formula (XIV) may then be reacted with an amine of formula H₂N-Lp under amide bond forming conditions to afford a compound of formula (I').

Certain of the intemediates described herein are believed to be novel. The present invention also provides the novel intermediates.

If necessary physiologically tolerable salts can be formed using methods known in the art.

Where the lipophilic group Lp comprises more than one group, it may generally be formed by coupling these groups together at an appropriate stage in the preparation of the compound of formula I using conventional methods or as described in the Examples.

The compounds of the invention may be administered by any convenient route, e.g. into the gastrointestinal tract (e.g. rectally or orally), the nose, lungs, musculature or vasculature or transdermally. The compounds may be administered in any convenient administrative form, e.g. tablets, powders, capsules, solutions, dispersions, suspensions, syrups, sprays, suppositories, gels, emulsions, patches etc. Such compositions may contain components conventional in pharmaceutical preparations, e.g. diluents, carriers, pH modifiers, sweeteners, bulking agents, and further active agents. Preferably the compositions will be sterile and in a solution or suspension form suitable for injection or infusion. Such compositions form a further aspect of the invention.

The following are examples of pharmaceutical compositions of compounds according to the invention.

### Formulation 1

### Hard gelatin capsules are prepared using the following ingredients:

| | Quantity (mg/capsule) |
|---|---|
| Active Ingredient | 250 |
| Starch, dried | 200 |
| Magnesium stearate | 10 |
| Total | 460 mg |

The above ingredients are mixed and filled into hard gelatin capsules in 460 mg quantities.

### Formulation 2

### Tablets each containing 60 mg of active ingredient are made as follows:

| | |
|---|---|
| Active Ingredient | 60 mg |
| Starch | 45 mg |
| Microcrystalline cellulose | 35 mg |
| Polyvinylpyrrolidone | 4 mg |
| Sodium carboxymethyl starch | 4.5 mg |
| Magnesium stearate | 0.5 mg |
| Talc | 1 mg |
| Total | 150 mg |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate, and talc, previously passed through a No. 60 mesh U.S. sieve, are then added to the granules which, after mixing, are compressed on a tablet machine to yield tablets each weighing 150 mg.

Viewed from this aspect the invention provides a pharmaceutical composition comprising a serine protease (tryptase) inhibitor according to the invention together with at least one pharmaceutically acceptable carrier or excipient. The pharmaceutical composition may also optionally comprise at least one further anti-inflammatory

Viewed from a further aspect the invention provides the use of a tryptase inhibitor according to the invention for the manufacture of a medicament for use in a method of treatment of the human or non-human animal body (e.g. a mammalian, avian or reptilian body) to combat (i.e. treat or prevent) a condition responsive to said inhibitor which comprises administering an effective amount of a compound according to the invention.

The dosage of the inhibitor compound of the invention will depend upon the nature and severity of the condition being treated, the administration route and the size and species of the patient. However in general, quantities of from 0.01 to 100 µmol/kg bodyweight will be administered.

The invention will now be described further with reference to the following Examples.

### Experimental:

Abbreviations used herein in the reaction schemes and examples follow IUPAC-IUB nomenclature. Additional abbreviations are BOC, t-butyloxycarbonyl; HPLC, high performance liquid chromatography; LC, liquid chromatography; MS, mass spectrometry; Rt, retention time; NMR, nuclear magnetic resonance; DMF, dimethylformamide; Quant, quantitative; DMAP, dimethylaminopyridine; TFA, trifluoroacetic acid; Sat., saturated; Aq., aqueous; DCM, dichloromethane; PyBroP, bromo-tris-pyrrolidino-phosphonium hexafluorophosphate; Phg, phenylglycine; Chex, cyclohexyl; THF, tetrahydrofuran; DiBal, diisobutylaluminium hydride; KHMDS, potassium bis(trimethylsilyl)amide; DBU, 1,8-diazabicyclo[5.4.0]undec-7-ene; Trisyl, tri-isopropylbenzenesulphonyl; Z, benzyloxycarbonyl; and EDC, 1-(3-dimethylaminopropyl)-3-ethylcarbodiimide hydrochloride. Starting materials were purchased from Aldrich (Gillingham, UK), Lancaster (Morecambe, UK), Avocado (Heysham, UK), Maybridge (Tintagel, UK), Nova Biochem (Nottingham, UK) or Bachem.

### Purification:

Flash column chromatography was carried out using Merck silica gel Si60 (40-63 µm, 230-400 mesh). Purification of final products was by crystallisation, flash column chromatography or gradient reverse phase HPLC on a Waters Deltaprep 4000 at a flow rate of 50 mL/minute using a Deltapak C18 radial compression column (40 mm x 210 mm, 10-15 mm particle size). Eluant A consisted of aqueous trifluoroacetic acid (0.1 %) and eluant B 90% acetonitrile in aqueous trifluoroacetic acid (0.1 %) with gradient elution (Gradient, 0 minutes 5 % B for 1 minutes, then 5 % B to 20 % B over 4 minutes, then 20 % B to 60 % B over 32 minutes). Fractions were analysed by analytical HPLC and LC/MS before pooling those with >95 % purity for lyophilisation.

### Analysis:

Proton nuclear magnetic resonance (¹H NMR) spectra were recorded on a Bruker DPX300 (300 MHz). Analytical HPLC's were performed on a Shimadzu LC6 gradient system equipped with an autosampler. Eluant A consisted of aqueous trifluoroacetic acid (0.1 %) and eluant B consisted of 90 % acetonitrile and 10 % water, containing trifluoroacetic acid (0.1 %). Gradient 1 elution began at 5 % B and increased to 100 % B over seven minutes. Gradient 2 elution began at 5 % B and increased to 100 % B over ten minutes. Gradient 3 elution began at 5 % B for one minute, increasing to 20 % B after the fourth minute, 40 % B after the 14^{th} minute and then 100 % B after the 15^{th} minute. The columns used were Luna 2 C18 (3 µ, 30 mm x 4.6 mm), Luna 2 C18 (5 µ, 150 mm x 2 mm) and a Symmetry Rp8 (3.5 µ, 50 x 2.1 mm).

LC/MS were performed on a PESCIEX single quadrupole API-150EX instrument, equipped with a Luna 2 C18 column (3 µ, 30 mm x 4.6 mm) eluting with 20 % to 100 % acetonitrile in water over five minutes (gradient 4).

### Method 1

### 3-(Aminomethyl)benzoyl-D-phenylglycine 2-aminobenzothiazol-6-amide bis(trifluoroacetate) salt.

### 2,6-Diaminobenzothiazole

2-Amino-6-nitrobenzothiazole (500 mg, 2.56 mmol) was dissolved in methanol (20 mL) and 10 % palladium on carbon (50 mg) was added as a slurry in methanol (1 mL). The atmosphere was replaced with hydrogen and the suspension was stirred overnight. The catalyst was removed by suction filtration and the solvent evaporated to afford 2,6-diaminobenzothiazole (420 mg, 99 %) as a pale yellow solid.

### N-BOC-D-Phenylglycine 2-aminobenzothiazol-6-amide

*N*-BOC-D-Phenylglycine (250 mg, 1.0 mmol), 1-(3-dimethylaminopropyl)-3-ethylcarbodiimide hydrochloride (190 mg, 1.0 mmol) and 7-aza-1-hydroxybenzotriazole (140 mg, 1.0 mmol) were stirred in dimethylformamide (3 mL) for ten minutes. 2,6-Diaminobenzothiazole (160 mg, 1.0 mmol) was then added and the solution was stirred overnight at room temperature. Ethyl acetate (15 mL) was added and the solution was washed with water (5 mL), saturated citric acid solution (5 mL), saturated NaHCO₃ (5 mL) and water (5 mL), and dried over MgSO₄. The solvent was removed under reduced pressure to afford N-BOC-D-phenylglycine 2-aminobenzothiazol-6-amide.
¹H NMR (CDCl₃): 8.93 (1 H, br s, C(O)NHAr); 7.72 (1 H, s, benzothiazole C(7)H); 7.35 (2 H, br s, Ph); 7.23 - 7.05 (3 H, m, Ph); 6.93 (1 H, d, *J* = 10 Hz, benzothiazole C(4)H or C(5)H); 6.72 (1 H, d, *J* = 10 Hz, benzothiazole C(4)H or C(5)H); 6.05 (1 H, d, *J* = 7 Hz, CHPh); 5.92 (2 H, br s, NH₂); 5.45 (1 H, br s, BOCNH); 1.27 (9 H, s, ^{t}Bu).

### D-Phenylglycine 2-aminobenzothiazol-6-amide

A solution of *N*-BOC-D-phenylglycine 2-aminobenzothiazol-6-amide in dichloromethane (5 mL) was treated with trifluoroacetic acid (5 mL) and stirred for 30 minutes. The dichloromethane and excess trifluoroacetic acid were removed under reduced pressure and the residue was triturated with diethyl ether to afford D-phenylglycine 2-aminobenzothiazol-6-amide as its trifluoroacetate salt (350 mg, 89 %).

### 3-(Aminomethyl)benzoyl-D-phenylglycine 2-aminobenzothiazol-6-amide trifluoroacetate salt

*N*-BOC-3-aminomethylbenzoic acid (250 mg, 1.0 mmol), 1-(3-dimethylaminopropyl)-3-ethylcarbodiimide hydrochloride (190 mg, 1.0 mmol) and 7-aza-1-hydroxybenzotriazole (140 mg, 1.0 mmol) were stirred in dimethylformamide (10 mL) for five minutes. D-Phenylglycine 2-aminobenzothiazol-6-amide trifluoroacetate salt (350 mg, 0.85 mmol) was then added and the mixture was stirred overnight. The solution was poured into ethyl acetate (20 mL) and washed with 5 % HCl (5 mL), saturated NaHCO₃ (5 mL) and water (5 mL), dried over MgSO₄ and the solvent removed under reduced pressure. The crude product was purified by flash column chromatography on silica gel (60 % ethyl acetate / 40 % hexane to 100 % ethyl acetate) to afford *N*-BOC-3-(aminomethyl)benzoyl-D-phenylglycine 2-aminobenzothiazol-6-amide. This was dissolved in dichloromethane (5 mL) and trifluoroacetic acid (5 mL) was added. The solution was stirred at room temperature for 30 minutes before the dichloromethane and excess trifluoroacetic acid were removed under reduced pressure. The residue was triturated with diethyl ether to afford 3-(aminomethyl)benzoyl-D-phenylglycine 2-aminobenzothiazol-6-amide as its trifluoroacetate salt (150 mg, 32 %).
¹H NMR (d₄ MeOH): 8.21 ppm (1 H, s, benzothiazole C(7)H); 7.97 (1 H, s, aminomethylbenzoyl C(2)H); 7.94 (1 H, d, J = 5 Hz, 3-(aminomethyl)benzoyl C(6)H); 7.80 - 7.48 (5 H, m, Ar); 7.47 - 7.32 (4 H, m, Ar); 5.81 (1 H, s, CHPh); 4.22 (2 H, s, CH₂NH₂).

HPLC (Luna 2, Gradient 1): rt = 2.80 minutes.

LC/MS (Luna 2, Gradient 4): rt = 1.40 minutes, 432 (MH)⁺.

Examples 1 - 8 were synthesised in the same way as the compound of Method 1 using the the indicated amine in place of 2,6-Diaminobenzothiazole.

### Example 1

### 3-(Aminomethyl)benzoyl-D-phenylglycine 5-acetyl-4-methylthiazol-2-amide trifluoroacetate salt.

From 5-acetyl-4-methylthiazol-2-amine.
¹H NMR (d₃ acetonitrile): 8.92 (1 H, d, *J* = 6 Hz, NHCH); 7.95 (2 H, br s, NH₂); 7.78 - 7.65 (2 H, m, Ar); 7.40-7.14 (7 H, m, Ar); 5.61 (1 H, d, *J* = 6 Hz, α-CH); 3.87 (2 H, br d, *J* = 5 Hz, CH₂NH₂); 2.32 (3 H, s, CH₃); additional methyl group concealed by solvent peak between 2.20 and 2.30ppm.
HPLC (Luna 2, Gradient 1): rt = 3.55 minutes.
LC/MS (Luna 2, Gradient 4): rt = 1.78 minutes, 423 (MH)⁺.

### Example 2

### 3-(Aminomethyl)benzoyl-D-phenylglycine 5-phenylthiazol-2-amide trifluoroacetate salt.

From 5-phenylthiazol-2-amine.
¹H NMR (d₃ acetonitrile): 12.81 (1 H, s, H-bonded NHAr); 9.07 (1 H, d, *J* = 6 Hz, NHCO); 8.12 (2 H, br s, CH₂NH₂); 8. 07 - 7.84 (3 H, m, Ar); 7.62 - 7.28 (12 H, m, Ar); 5.88 (1 H, d, J = 6 Hz, α-CH); 4.10 (2 H, m, CH₂NH₂).
HPLC (Luna 2, Gradient 1): rt = 4.36 minutes.
LC/MS (Luna 2, Gradient 4): rt = 2.23 minutes, 443 (MH)⁺.

### Example 3

### 3-(Aminomethyl)benzoyl-D-phenylglycine 4,5-dimethyl-thiazol-2-amide trifluoroacetate salt.

From 9,5-dimethylthiazol-2-amine.
¹H NMR (d₄ methanol): 9.01 (0.5 H due to partial exchange, d, *J* = 6 Hz, NHCH); 7.99 - 7.93 (2 H, m, Ar); 7.61 - 7.43 (7 H, m, Ar); 5.85 (1 H, s superimposed upon d, J 6 Hz, α-CH); 4.18 (2 H, s, CH₂NH₂) ; 2.29 & 2.18 (2 x 3 H, 2 x s, 2 x CH₃).
HPLC (Luna 2, Gradient 1): rt = 3.67 minutes.
LC/MS (Luna 2, Gradient 4): rt = 1.96 minutes, 395 (MH)⁺.

### Example 4

### 3-(Aminomethyl)benzoyl-D-phenylglycine 4-methyl-5-ethoxycarbonylthiazol-2-amide trifluoroacetate salt.

From 4-methyl-5-ethoxycarbonylthiazol-2-amine.
¹H NMR (d₃ acetonitrile): 8.20 (1 H, d, *J =* 6 Hz, NHCH) ; 8.02 (1 H, s, Ar) ; 7.76 (1 H, d, *J =* 7 Hz, Ar) ; 7.52 - 7.30 (7 H, m, Ar) ; 5.78 (1 H, d, J 6 Hz, α-CH); 4.16 (2 H, q, *J =* 6 Hz, CH₂CH₃); 4.11 (2 H, s, CH₂NH₂); 2.45 (3 H, s, ArCH₃); 1.21 (3 H, t, *J =* 6 Hz, CH₂CH₃).
HPLC (Luna 2, Gradient 1): rt = 3.73 minutes.
LC/MS (Luna 2, Gradient 4): rt = 2.13 minutes, 453 (MH)⁺.

### Example 5

### 3-(Aminomethyl)benzoyl-D-phenylglycine 4-(methoxycarbonylmethyl)-5-methylthiazol-2-amide trifluoroacetate salt.

From 4-methoxycarbonylmethyl-5-methylthiazol-2-amine.
¹H NMR (d₃ acetonitrile): 8.02 - 7.97 (2 H, m, Ar & NHCH); 7.87 (1 H, d, *J* = 8 Hz, Ar); 7.59 - 7.33 (7 H, m, Ar); 7.21 (2 H, br s, NH₂); 5.81 (1 h, d, *J* = 6 Hz, α-CH); 4.18 (2 H, s, CH₂NH-₂); 3.58 (3 H, s, CO₂CH₃); 3.55 (2 H, s, CH₂CO); 2.31 (3 H, s, ArCH₃).
HPLC (Luna 2, Gradient 1): rt = 3.54 minutes.
LC/MS (Luna 2, Gradient 4): rt = 2.05 minutes, 453 (MH)⁺.

### Example 6

### 3-(Aminomethyl)benzoyl-D-phenylglycine 5,6-dihydro-3-methoxycarbonyl-4H-cyclopenta(b)thiophen-2-amide trifluoroacetate salt.

From 5,6-dihydro-3-methoxycarbonyl-4*H*-cyclopenta(b)thiophen-2-amine.
¹H NMR (d₃ acetonitrile): 8.30 (1 H, d, *J* = 6 Hz, NHCH); 8.04 (1 H, Ar); 7.85 (1H, d, *J* = 8 Hz, Ar) ; 7.62 - 7.30 (7 H, m, Ar) ; 5.80 (1 H, d, *J* = 6 Hz, α-CH) ; 4.16 (2 H, s, CH₂NH₂) ; 3.72 (3 H, s, CH₃) ; 2.87 & 2.84 (2 x 2 H, 2 x t, 2 x *J* = 6 Hz, CH₂CH₂CH₂) ; 2.30 (2 H, pentet, *J* = 6 Hz, CH₂CH₂CH₂).
HPLC (Symmetry, Gradient 2): rt = 6.56 minutes.
LC/MS (Luna 2, Gradient 4): rt = 2.26 minutes, 464 (MH)⁺.

### Example 7

### 3-(Aminomethyl)benzoyl-D-phenylglycine 3-ethoxycarbonyl-4,5,6,7-tetrahydrobenzo(b)thiophene-2-amide trifluoroacetate salt.

From 3-ethoxycarbonyl-4,5,6,7-tetrahydrobenzo(b)thiophene-2-amine.
¹H NMR (d₃ acetonitrile): 8.25 (0.6 H due to partial exchange, d, *J* = 6 Hz, NHCH); 8.06 (1 H, s, Ar); 7.86 (1 H, d, *J* = 7 Hz, Ar); 7.52 - 7.23 (7 H, m, Ar); 5.79 (1 H, s superimposed upon d, *J* = 6 Hz, α-CH); 4.18 - 4.04 (4 H, m, CH₂NH₂ & CH₂CH₃); 2.65 & 2.50 (2 x 2 H, 2 x br s, CH₂CH₂CH₂CH₂); 1.82 - 1.70 (4 H, m, CH₂CH₂CH₂CH₂).
HPLC (Luna 2, Gradient 1): rt = 5.15 minutes.
LC/MS (Luna 2, Gradient 4): rt = 2.42 minutes, 492 (MH)⁺.

### Example 8

### 3-(Aminomethyl)benzoyl-D-phenylglycine 3-benzyloxycarbonyl-9,5,6,7-tetrahydrobenzo(b)thiophene-2-amide trifluoroacetate salt.

From 3-benzyloxycarbonyl- 4,5,6,7-tetrahydrobenzo(b)thiophene-2-amine.
¹H NMR (d₃ acetonitrile): 8.02 - 7.95 (2 H, m, 1 x Ar & NHCH); 7.92 (1 H, d, *J =* 7 Hz, Ar); 7.72 - 7.32 (12 H, m, Ar); 5.81 (1 H, d, *J =* 6 Hz, α-CH); 5.22 (2 H, s, CH₂Ar) ; 4.18 (2 H, s, CH₂NH₂) ; 2.70 & 2.59 (2 x 2 H, 2 x t, 2 x *J* = 5 Hz CH₂CH₂CH₂CH₂) ; 1. 82 - 1.68 (4 H, m, CH₂CH₂CH₂CH₂).
HPLC (Luna 2, Gradient 1): rt = 5.40 minutes.
LC/MS (Luna 2, Gradient 4): rt = 2.75 minutes, 554 (MH)⁺.

### Example 9

### 3-(Aminomethyl)benzoyl-D/L-phenylglycine 4,5,6,7-tetrahydro-3-(4-morpholinecarbonyl)benzo (b)thiophene-2-amide trifluoroacetate salt.

### 3-(BOC-Aminomethyl)benzoyl-D-phenylglycine 4,5,6,7-tetrahydro-3-(hydroxycarbonyl)benzo(b)thiophen-2-amide.

A solution of 3-(BOC-aminomethyl)benzoyl-D/L-phenylglycine 3-benzyloxycarbonyl-4,5,6,7-tetrahydrobenzo(b)thiophen-2-amide (a protected form of example 8 and an intermediate in its synthesis) (1.00 g, 1.6 mmol) in methanol (20 mL) was stirred over 10% palladium on carbon under a hydrogen atmosphere for two hours. The mixture was filtered and the methanol evaporated under reduced pressure to afford the acid (0.86 g, quant.) which was used without further purification.
¹H NMR (d₆ DMSO): 11.91 (1 H, br s CO₂H); 9.46 (1 H, d, *J* = 6 Hz, NHCH); 7.90 (1 H, d, *J* = 6 Hz, NHBoc); 7.86 (1 H, s, Ar); 7.55 - 7.32 (7 H, m, Ar); 5.92 (1 H, d, *J* = 6 Hz, α-CH); 4.20 (2 H, d, *J* = 6 Hz, CH₂NH₂); 2.71 & 2.59 (2 x 2 H, 2 x br s, CH₂CH₂CH₂CH₂); 1.72 - 1.60 (4 H, m, CH₂CH₂CH₂CH₂); 1.34 (9 H, s, C(CH₃)₃).

### 3-(Aminomethyl)benzoyl-D-phenylglycine 4,5,6,7-tetrahydro-3-(4-morpholinecarbonyl) benzo(b)thiophene-2-amide trifluoroacetate salt.

A solution of the carboxylic acid (55 mg, 0.10 mmol) in DMF (2 mL) was stirred at room temperature and diisopropylethylamine (52 µL, 39 mg, 0.30 mmol), morpholine (87 µL, 87 mg, 1.00 mmol) and *O*-(7-azabenzotriazol-1-yl)-*N*',*N*',*N*',*N*'-tetramethyluronium hexafluorophosphate (114 mg, 0.30 mmol) were added. The reaction was allowed to stir at room temperature until HPLC indicated complete consumption of starting material (5 days). Ethyl acetate (20 mL) was then added, and the solution extracted with 1N HCl (20 mL); sodium bicarbonate (sat., aq., 20 mL) and water (20 mL); dried over MgSO₄, concentrated under reduced pressure, and purified by flash column chromatography. A solution of the amide in dichloromethane (2 mL) was treated with trifluoroacetic acid (2 mL) for an hour. The excess TFA and dichloromethane were evaporated and the residue purified by trituration with diethyl ether to afford 3-(Aminomethyl)benzoyl-D-phenylglycine 4,5,6,7-tetrahydro-3-(4-morpholinecarbonyl) benzo(b)thiophene-2-amide as its trifluoroacetate salt.
¹H NMR (d₃ acetonitrile): 9.02 ppm (1 H, s, NHAr); 7.85 - 7.70 (3 H, m, NHCH & 2 x Ar); 7.50 - 7.25 (7 H, m, Ar); 5.64 (1 H, d, *J* = 6 Hz, α-CH) ; 4.03 (2 H, br s, CH₂NH₂) ; 3.40 - 2.89 (8 H, m, 2 x CH₂O & CH₂CH₂CH₂CH₂); 2.52 - 2.45 & 2.30 - 2.15 (2 x 2 H, 2 x m, 2 x morpholine CH₂N); 1.75 - 1. 60 (4 H, m, CH₂CH₂CH₂CH₂).
HPLC (Luna 2, Gradient 1): rt = 3.68 minutes.
LC/MS (Luna 2, Gradient 4): rt = 1.91 minutes, 533 (MH)⁺.

Examples 10 - 14 were prepared from 3-(*N*-BOC-aminomethyl)benzoyl-D/L-phenylglycine) 4,5,6,7-tetrahydro-3-(hydroxycarbonyl)benzo(b)thiophen-2-amide in the same manner as example 9, using the indicated amine.

### Example 10

### 3-(Aminomethyl)benzoyl-D-phenylglycine 3-benzylaminocarbonyl 4,5,6,7-tetrahydrobenzo(b)thiophen-2-amide trifluoroacetate salt.

Prepared from benzylamine.
¹H NMR (d₃ acetonitrile): 8.02 - 7.91 (3 H, m, 2 x Ar & NHCH); 7.60 - 7.25 (12 H, m, Ar); 6.73 (1 H, t, *J* = 5 Hz, NHBn); 5.79 (1 H, d, *J* = 6 Hz, α-CH); 4.41 (2 H, d, *J* = 5 Hz, CH₂Ar); 4.16 (2 H, s, CH₂NH₂); 2.71 & 2.62 (2 x 2 H, 2 x br s, CH₂CH₂CH₂CH₂); 1. 8 5 - 1.76 (4 H, m, CH₂CH₂CH₂CH₂).
HPLC (Luna 2, Gradient 1): rt = 4.47 minutes.
LC/MS (Luna 2, Gradient 4): rt = 2.40 minutes, 553 (MH)⁺.

### Example 11

### 3-(Aminomethyl)benzoyl-D-phenylglycine 4,5,6,7-tetrahydro-3-(3-pyridylmethylaminocarbonyl) benzo(b)thiophen-2-amide bis-trifluoroacetate salt.

Prepared from 3-pyridylmethylamine.
¹H NMR (d₃ acetonitrile): 8.37 (1H, s, Ar); 8.34 (1 H, d, *J* = 5 Hz, NHCH); 8.25 (1 H, d, *J* = 6 Hz, Ar); 7.78 - 7.14 (11 H, m, Ar); 6.81 (1 H, t, *J* = 5 Hz, NHBn); 5.58 (1 H, d, *J* = 5 Hz, α-CH); 4.92 (2 x 1 H, 2 x dd, 2 x *J* = 8 Hz, 5 Hz, NHCH₂Ar); 4.04 (2 H, s, CH₂NH₂) : 2.58 & 2.46 (2 x 2 H, 2 x br s, CH₂CH₂CH₂CH₂); 1. 62-1. 53 (4 H, m, CH₂CH₂CH₂CH₂).
HPLC (Luna 2, Gradient 1): rt = 3.32 minutes.
LC/MS (Luna 2, Gradient 4): rt = 1.59 minutes, 554 (MH)⁺.

### Example 12

### 3-(Aminomethyl)benzoyl-D-phenylglycine 4,5,6,7-tetrahydro-3-(1-piperidinecarbonyl)benzo (b) thiophen-2-amide trifluoroacetate salt.

Prepared from piperidine.
¹H NMR (d₃ acetonitrile): 9.20 (1 H, s, NHAr); 8.15 - 7.26 (9 H, m, Ar); 5.82 (1 H, br s, α-CH); 4.06 (2 H, br s, CH₂NH₂); 3.20 - 1.15 (18 H, m, piperidyl and cyclohexyl protons)
HPLC (Luna 2, Gradient 1): rt = 3.94 minutes.
LC/MS (Luna 2, Gradient 4): rt = 2.02 minutes, 531 (MH)⁺.

### Example 13

### 3-(Aminomethyl)benzoyl-D-phenylglycine 3-(cyclopropylmethylaminocarbonyl)-4,5,6,7-tetrahydrobenzo(b)thiophen-2-amide trifluoroacetate salt.

Prepared from cyclopropylmethylamine.
¹H NMR (d₃ acetonitrile): 7.76 - 7.58 (3 H, m, 2 x Ar & NHCH); 7.40 - 7.12 (7 H, m, Ar); 6.18 (1 H, t, J = 5 Hz, NHBn); 5.50 (1 H, d, J = 5 Hz, α-CH); 4.00 (2 H, br s, CH₂NH₂); 2.92 (2H, t, J = 5 Hz, CH₂cPr); 2.48 & 2.40 (2 x 2 H, 2 x br s, CH₂CH₂CH₂CH₂); 1.65 - 1.55 (4 H, m, CH₂CH₂CH₂CH₂); 0.81 - 0.65, 0.28 - 0.17 & 0.05 to -0.05 (1 H, 2 H & 2 H, 3 x m, cPr protons).
HPLC (Luna 2, Gradient 1): rt = 4.19 minutes.
LC/MS (Luna 2, Gradient 4): rt = 2.18 minutes, 517 (MH)⁺.

### Example 14

### 3-(Aminomethyl)benzoyl-D-phenylglycine 4,5,6,7-tetrahydro-3-(1,3-dimethylbutylaminocarbonyl) benzo (b)thiophen-2-amide trifluoroacetate salt.

Prepared from racemic 1,3-dimethylbutylamine.
¹H NMR (d₃ acetonitrile): 7.88 - 7.75 (3 H, m, 2 x Ar & NHCH); 7.48 - 7.25 (7 H, m, Ar); 5.97 (1 H, d, *J* = 5 Hz, α-CH); 5.68 & 5.65 (2 x 0.5 H, 2 x d, 2 x *J* = 4 Hz, NHHex); 4.12 - 4.02 (2 H, m, CH₂NH₂); 4.01 - 3.90 (1 H, m, CH₃CHNH); 2.59 & 2.47 (4 H, m, CH₂CH₂CH₂CH₂); 1.73 - 1.62 (4 H, m, CH₂CH₂CH₂CH₂); 1.53 - 0.70 (12 H, m, remaining hexyl protons).
HPLC (Luna 2, Gradient 1): rt = 4.63 minutes.
LC/MS (Luna 2, Gradient 4): rt = 2.53 minutes, 547 (MH)⁺.

### Example 15

### 3-(Aminomethyl)benzoyl-D-phenylglycine 4,5,6,7-tetrahydro-3-(hydroxycarbonyl)benzo(b)thiophen-2-amide trifluoroacetate salt.

A solution of 3-(BOC-aminomethyl)benzoyl-D/L-phenylglycine 4,5,6,7-tetrahydro-3-(hydroxycarbonyl)benzo(b)thiophen-2-amide (50 mg, 0.9 mmol), the compound from which examples 13 - 17 were made, in dichloromethane (2 mL) was treated with trifluoroacetic acid (2 mL) for an hour at room temperature. The solvents were evaporated and the residue triturated with diethyl ether to afford the title compound as an off-white solid.
¹H NMR (d₃ acetonitrile): 8.25 - 8.17 (2 H, m, Ar) ; 8.01 (1 H, d, *J =* 6 Hz, NHCH); 7.75 - 7.52 (7 H, m, Ar); 6.00 (1 H, d, *J* = 6 Hz, α-CH); 4.35 (2 H, br s, CH₂NH₂); 2.85 & 2.71 (2 x 2 H, 2 x br s, CH₂CH₂CH₂CH₂) ; 1. 92 - 1.80 (4 H, m, CH₂CH₂CH₂CH₂.
HPLC (Luna 2, Gradient 1): rt = 4.31 minutes.
LC/MS (Luna 2, Gradient 4): rt = 2.07 minutes, 464 (MH)⁺.

### Example 16

### 3-(Aminomethyl)benzoyl-D/L-phenylglycine benzothiazol-2-amide trifluoroacetate salt.

### α-N-BOC-D/L-Phenylglycine benzothiazol-2-amide.

A solution of *N*-BOC-D-phenylglycine (750 mg, 3.0 mmol) in anhydrous tetrahydrofuran (20 mL) was stirred at room temperature under argon. Isobutyl chloroformate (0.52 mL, 4.0 mmol) and diisopropylethylamine (0.81 mL, 4.7 mmol) were added and the solution was stirred for 30 minutes. A solution of 2-aminobenzothiazole (500 mg, 3.3 mmol) in tetrahydrofuran (10 mL) was added to the mixed anhydride solution and stirred overnight at room temperature. Ethyl acetate (50 mL) was added and the organic phase was washed with water (25 mL), 5 % HCl solution (25 mL), saturated aqueous NaHCO₃ (25 mL) and water (25 mL), before being dried (MgSO₄) and concentrated under reduced pressure. The residue was purified by flash chromatography using ethyl acetate / hexane 1/1 as eluant to afford the coupled product as a yellow oil (785 mg, 68 %).
¹H NMR (CDCl₃) : 7.71 ppm (1 H, d, *J* = 7.2 Hz, Ar); 7.42 (1 H, d, *J* = 7.2 Hz, Ar); 7.36 (8 H, m, Ar and NH); 6.29 (1 H, br s, CH); 5.60 (1 H, br s, NH); 1.30 (9 H, s, C₄H₉).

### 3-(N-BOC-Aminomethyl)benzoyl-D/L-phenylglycine benzothiazol-2-amide.

A solution of the α-*N*-BOC-D/L-phenylglycine benzothiazol-2-amide (785 mg, 2.24 mmol) in dichloromethane (5 mL) was treated with trifluoroacetic acid (2 mL) and stirred for 1 hour at room temperature. The solution was concentrated under reduced pressure and the residual TFA salt was taken up in dimethylformamide (15 mL). This solution was treated with triethylamine (0.92 mL, 6.6 mmol), 1-(3-dimethylaminopropyl)-3-ethylcarbodiimide hydrochloride (478 mg, 2.5 mmol), 3-(*N-*BOC-aminomethyl)benzoic acid (562 mg, 2.24 mmol) and DMAP (50 mg) and stirred overnight at room temperature. The solution was partitioned between ethyl acetate (25 mL) and water (25 mL) and the organic phase was washed with 5 % HCl solution (25 mL), saturated aqueous NaHCO₃ (25 mL) and water (25 mL) before being dried (MgSO₄) and concentrated under reduced pressure to afford a yellow oil. The residue was purified by flash chromatography using ethyl acetate / hexane 1/1 as eluant to afford a colourless solid (185 mg, 16 %).
¹H NMR (CDCl₃): 7.87 - 7.73 ppm (2 H, m, Ar and NH); 7.66 - 7.45 (3 H, m, Ar and NH); 7.40 - 7.30 (3 H, m, Ar); 7.26 - 7.00 (6 H, m, Ar); 6.11 (1 H, d, *J =* 6.9 Hz, CHPh); 4.98 (1 H, br s, NH); 4.02 (2 H, d, *J* = 6.5 Hz, CH₂NH₂).

### 3-(Aminomethyl)benzoyl-D/L-phenylglycine benzothiazol-2-amide trifluoroacetate salt.

A solution of 3-(*N*-BOC-aminomethyl)benzoyl-D/L-phenyl-glycine benzothiazol-2-amide (156 mg, 0.3 mmol) in dichloromethane (3 mL) was stirred at room temperature and trifluoroacetic acid (2 mL) was added. Stirring was continued for a further hour and the solvents were removed under reduced pressure to afford a yellow oil which was triturated with diethyl ether to give the trifluoroacetate salt as a colourless solid (120 mg, 96 %) .
¹H NMR (d₄ methanol): 7.82 - 7.67 (3 H, m, Ar); 7.56 (1 H, d, *J* = 7.2 Hz, Ar); 7.53 - 7.21 (8 H, m, Ar); 7.19 - 7.09 (1 H, m, CHPh); 3.89 (2 H, s, CH₂NH₂).
HPLC (Luna 2, Gradient 1): rt = 3.95 minutes.
LC/MS (Luna 2, Gradient 4): rt = 1.88 minutes, 417 (MH)⁺.

Examples 17 - 23 were prepared in a manner analogous to Example 16 except that the indicated amine was used in place of 2-aminobenzothiazole.

### Example 17

### 3-(Aminomethyl)benzoyl-D/L-phenylglycine 5,6-dimethylbenzothiazol-2-amide trifluoroacetate salt.

From 2-amino-5,6-dimethylbenzothiazole.
¹H NMR (d₄ methanol): 7.80 - 7.63 ppm (2 H, m, Ar); 7.47 - 7.07 (9 H, m, Ar); 5.71 (1 H, s, CHPh) ; 3.92 (2 H, s, CH₂NH₂); 2.12 (6 H, s, 2 x CH₃).
HPLC (Luna 2, Gradient 1): rt = 4.39 minutes.
LC/MS (Luna 2, Gradient 4): rt = 2.10 minutes, 445 (MH)⁺.

### Example 18

### 3-(Aminomethyl)benzoyl-D/L-phenylglycine 6-methoxy benzothiazol-2-amide trifluoroacetate salt.

From 2-amino-6-methoxybenzothiazole.
¹H NMR (CDCl₃): 8.18 ppm (3 H, br s, Ar and NH); 7.90 (1 H, s, Ar); 7.79 (1 H, s, Ar); 7.71 (1 H, d, *J* = 7.2 Hz, Ar); 7.63 - 7.28 (8 H, m, Ar and NH); 7.24 (1 H, s, Ar); 7.10 (1 H, d, *J* = 7.2 Hz, Ar); 5.72 (1 H, d, *J* = 6.5 Hz, CHPh); 4.09 (2 H, s, CH₂NH₂); 3.88 (3 H, s, OCH₃).
HPLC (Luna 2, Gradient 1): rt = 4.26 minutes.
LC/MS (Luna 2, Gradient 4): rt = 1.94 minutes, 447 (MH)⁺.

### Example 19

### 3-(Aminomethyl)benzoyl-D/L-phenylglycine 6-methylbenzothiazol-2-amide trifluoroacetate salt.

From 2-amino-6-methylbenzothiazole.
¹H NMR (d₄ methanol): 8.02 - 7.90 ppm (2 H, m, Ar); 7.70 - 7.54 (6 H, m, Ar); 7.48 - 7.37 (3 H, m, Ar); 7.25 (1 H, d, *J* = 7.2 Hz, Ar); 5.92 (1 H, s, CHPh); 4.19 (2 H, s, CH₂NH₂); 2.46 (3 H, s, CH₃).
HPLC (Luna 2, Gradient 1): rt = 4.21 minutes.
LC/MS (Luna 2, Gradient 4): rt = 2.26 minutes, 431 (MH)⁺.

### Example 20

### 3-(Aminomethyl)benzoyl-D/L-phenylglycine 4-methoxybenzothiazol-2-amide trifluoroacetate salt.

From 2-amino-4-methoxybenzothiazole.
¹H NMR (d₄ methanol): 7.88 ppm (1 H, d, *J* = 7.2 Hz, Ar); 7.79 (1 H, d, *J* = 7.2 Hz, Ar); 7.64 - 7.14 (9 H, m, Ar); 6.94 (1 H, d, *J* = 7.2 Hz, Ar); 5.89 (1 H, s, CHPh); 4.03 (2 H, s, CH₂NH₂) ; 3.93 (3 H, s, OCH₃).
HPLC (Luna 2, Gradient 1): rt = 3.95 minutes.
LC/MS (Luna 2, Gradient 4): rt = 1.88 minutes, 447 (MH)⁺.

### Example 21

### 3-(Aminomethyl)benzoyl-D/L-phenylglycine 4-methylbenzothiazol-2-amide trifluoroacetate salt.

From 2-amino-4-methylbenzothiazole.
¹H NMR (d₄ methanol): 7.95 ppm (1 H, s, Ar); 7.89 (1 H, d, *J* = 7.2 Hz, Ar); 7.69 - 7.33 (8 H, m, Ar); 7.27 - 7.12 (2 H, m, Ar); 5.91 (1 H, s, CHPh); 4.03 (2 H, s, CH₂NH₂); 2.60 (3 H, s, CH₃).
HPLC (Luna 2, Gradient 1): rt = 4.31 minutes.
LC/MS (Luna 2, Gradient 4): rt = 2.18 minutes, 431 (MH)⁺.

### Example 22

### 3-(Aminomethyl)benzoyl-D/L-phenylglycine 4-chlorobenzothiazol-2-amide trifluoroacetate salt.

From 2-amino-4-chlorobenzothiazole.
¹H NMR (d₄ methanol): 8.00 - 7.85 ppm (2 H, m, Ar); 7.82 - 7.74 (1 H, d, *J =* 7.2 Hz, Ar); 7.67 - 7.35 (8 H, m, Ar); 7.25 (1 H, t, *J =* 7.2 Hz, Ar); 5.89 (1 H, s, CHPh) ; 4.10 (2 H, s, CH₂NH₂). HPLC (Luna 2, Gradient 1): rt = 4.29 minutes.
LC/MS (Luna 2, Gradient 4): rt = 2.05 minutes, 451 (MH)⁺.

### Example 23

### 3-(Aminomethyl)benzoyl-D/L-phenylglycine 4,5,6,7-tetrahydrobenzothiazol-2-amide.

From 4,5,6,7-tetrahydrobenzothiaz-2-amine, the synthesis of which is described below.

### 4,5,6,7-Tetrahydro-1,3-benzothiazol-2-amine

A stirred mixture of 2-chlorocyclohexanone (200 mg, 1.5 mmol) and thiourea (114 mg, 1.5 mmol) in tetrahydrofuran (20 ml) was heated at reflux for 6 hours. The solution was concentrated under reduced pressure and the amine was purified by flash column chromatography using ethylacetate / hexane 1/1 as eluent to afford a colourless oil (169 mg, 74 %).
¹H NMR (CDCl₃): 5.06 (2 H, br s, NH₂); 2.40 - 2.23 (4 H, m, 2 x CH₂); 1. 64 - 1.51 (4 H, m, 2 x CH₂).

### 3-(Aminomethyl)benzoyl-D/L-phenylglycine 4,5,6,7-tetrahydrobenzothiazol-2-amide.

¹H NMR (d₄ methanol): 8.01 - 7.92 (2 H, m, Ar); 7.68 (1 H, d, *J* = 7.2 Hz, Ar); 7.61 - 7.51 (3 H, m, Ar); 7.47 - 7.34 (3 H, m, Ar); 5.90 (1 H, s, CH); 4.20 (2 H, s, CH₂NH₂); 2.69 (2 H, br s, CH₂); 2.60 (2 H, br s, CH₂); 1.98 (4 H, br s, 2 x CH₂).
HPLC (Luna 2, Gradient 1): rt = 3.55 minutes.
LCMS (Luna 2, Gradient 4): rt =1.88 minutes, 421 (M + H)⁺.

Examples 24 - 37 were synthesised using the same method described for example 9 using 3-(*N*-BOC-aminomethyl)benzoyl-D-phenylglycine 4,5,6,7-tetrahydro-3-(hydroxycarbonyl)benzo(b)thiophen-2-amide and the indicated amine.

### Example 24

### 3-(Aminomethyl)benzoyl-D/L-phenylglycine 3-furfurylaminocarbonyl-4,5,6,7-tetrahydrobenzo(b)thiophen-2-amide trifluoroacetate salt

Prepared from furfurylamine.
¹H NMR (d₃ acetonitrile): 7.88-7.74 & 7.50-7.23 (4 H & 11 H, 2 x m, 10 x Ar & 5 x NH), 6.22 & 6.14 (2 x 1 H, 2 x s, furyl CH's), 5.65 (1 H, d, J 6 Hz, α-CH), 4.32 (2 H, d, J 5 Hz, CH₂furyl), 4.04 (2 H, br s, CH₂NH₂), 2.58-2.44 (4 H, m, CH₂CH₂CH₂CH₂), 1.70-1.58 (4 H, m, CH₂CH₂CH₂CH₂).

Hplc (Luna 2, Gradient 1): rt = 4.20 minutes.
LC/MS (Luna 2, Gradient 4): rt = 2.18 minutes, 543 (MH⁺).

### Example 25

### 3-(Aminomethyl)benzoyl-D/L-phenylglycine 4,5,6,7-tetrahydro-3-(para-methoxybenzylaminocarbonyl)benzo (b)thiophen-2-amide trifluoroacetate salt

Prepared from para-methoxybenzylamine.
¹H NMR (d₃ acetonitrile): 8.01-7.35 (9 H, m, Ar), 7.27 & 6.80 (2 x 2 H, 2 x d, 2 x J 7 Hz para-subs Ar), 5.78 (1 H, d, J 6 Hz, α-CH), 4.48-4.35 (2 H, m, CH₂pMB), 4.24 (2 H, br s, CH₂NH₂), 3.78 (3 H, s, OCH₃), 2.72-2.60 (4 H, m, CH₂CH₂CH₂CH₂), 1.85-1.73 (4 H, m, CH₂CH₂CH₂CH₂).

Hplc (Luna 2, Gradient 1): rt = 4.36 minutes.
LC/MS (Luna 2, Gradient 4): rt = 2.33 minutes, 543 (MH⁺).

### Example 26

### 3-(Aminomethyl)benzoyl-D/L-phenylglycine 3-diethylaminocarbonyl-4,5,6,7-tetrahydrobenzo(b)thiophen-2-amide trifluoroacetate salt

Prepared from diethylamine.
¹H NMR (d₃ acetonitrile): Most peaks broadened by a rotomer effect. 9.02 (1 H, br s, NH), 7.91-6.86 (13 H, m, 9 x Ar, 3 x NH), 5.68 (1 H, d, J 6 Hz, α-CH), 4.02 (2 H, br s, ArCH₂NH₂), 3.07-2.82 (4 H, m, N(CH₂)₂) 2.56-2.47 & 2.28-2.16 (2 x 2H, 2 x m, CH₂CH₂CH₂CH₂), 1.72-1.52 (4 H, m, CH₂CH₂CH₂CH₂), 0.99-0.88 & 0.82-0.69 (2 x 3 H, 2 x m, 2 x CH₃).
Hplc (Luna 2, Gradient 1): rt = 4.02 minutes.
LC/MS (Luna 2, Gradient 4): rt = 2.03 minutes, 519 (MH⁺).

### Example 27

### 3-(Aminomethyl)benzoyl-D/L-phenylglycine 4,5,6,7-tetrahydro-3-(methylaminocarbonyl)benzo(b)thiophen-2-amide trifluoroacetate salt

Prepared from methylamine.
¹H NMR (d₃ acetonitrile): 7.95-7.82 & 7.51-7.28 (3 H & 6 H, 2 x m, Ar), 6.85-6.30 (5 H, m, 5 x NH), 5.71 (1 H, d, J 6 Hz, α-CH), 4.15 & 4.10 (2 H, ABq, J 5 Hz, ArCH₂NH₂), 2.71 (3 H, d, J 4 Hz, CH₃), 2.65-2.56 (4 H, m, CH₂CH₂CH₂CH₂), 1.78-1.69 (4 H, m, CH₂CH₂CH₂CH₂).
Hplc (Luna 2, Gradient 1): rt = 3.99 minutes.
LC/MS (Luna 2, Gradient 4): rt = 1.88 minutes, 477 (MH⁺).

### Example 28

### 3-(Aminomethyl)benzoyl-D/L-phenylglycine 3-isobutylaminocarbonyl-4,5,6,7-tetrahydrobenzo(b)thiophen-2-amide trifluoroacetate salt

Prepared from *iso*-butylamine.
¹H NMR (d₃ acetonitrile): 7.91-7.80 & 7.52-7.20 (3 H & 6 H, 2 x m, Ar), 6.84-6.25 (5 H, m, 5 x NH), 5.72 (1 H, d, J 6 Hz, α-CH), 4.16 & 4.11 (2 H, ABq, J 5 Hz, ArCH₂NH₂), 3.01 (2 H, t, J 7 Hz, CH₂ⁱPr), 2.65-2.51 (4 H, m, CH₂CH₂CH₂CH₂), 1.78-1. 67 (4 H, m, CH₂CH₂CH₂CH₂) 0.88-0.76 (6 H, m, 2 x CH₃) CHMe₂ missing, obscured by solvent peaks around 2ppm.
Hplc (Luna 2, Gradient 1): rt = 4.65 minutes.
LC/MS (Luna 2, Gradient 4): rt = 2.23 minutes, 519 (MH⁺).

### Example 29

### 3-(Aminomethyl)benzoyl-DL-phenylglycine 3-(3-aminocarbonylpiperidine-1-carbonyl)-4,5,6,7-tetrahydrobenzo(b)thiophen-2-amide trifluoroacetate salt

Prepared from 3-(aminocarbonyl)piperidine.
¹H NMR (d₃ acetonitrile): (Spectrum complicated by rotomers and diastereomers) 8.15-5.72 (various Ar, NH & α-CH), 4.20-4.06 (2 H, m, ArCH₂NH₂), 3.50-1.20 (various aliphatic).
Hplc (Luna 2, Gradient 1): rt = 3.58, 3.67 minutes (ratio 1:1).
LC/MS (Luna 2, Gradient 4): rt = 1.70 minutes, 574 (MH⁺).

### Example 30

### 3-(Aminomethyl)benzoyl-D/L-phenylglycine 3-(4-ethoxycarbonylpiperidine-1-carbonyl)-4,5,6,7-tetrahydrobenzo(b)thiophen-2-amide trifluoroacetate salt

Prepared from 4-ethoxycarbonylpiperidine.
¹H NMR (d₃ acetonitrile): (Spectrum complicated by rotomers) 9.55-9.35 & 8.10-7.25 (1 H & 13 H, various Ar, NH) 5.82-5.72 (1 H, m, α-CH), 4.15-4.00 (2 H, m, ArCH₂NH₂), 3.80-1.20 (various aliphatic).
Hplc (Luna 2, Gradient 1): rt = 3.982 minutes.
LC/MS (Luna 2, Gradient 4): rt = 2.10 minutes, 603 (MH⁺).

### Example 31

### 3-(Aminomethyl)benzoyl-D/L-phenylglycine 3-(4-formyl)piperazinecarbonyl-4,5,6,7-tetrahydrobenzo(b)thiophen-2-amide trifluoroacetate salt

Prepared from 4-formylpiperazine.
¹H NMR (d₃ acetonitrile): (Spectrum complicated by rotomers) 9.40-9.28 & 8.15-7.22 (1 H & 13 H, various) 5.85-5.78 (1 H, m, α-CH), 4.18-3.97 (2 H, m, ArCH₂NH₂), 3.40-1.62 (various aliphatic).
Hplc (Luna 2, Gradient 1): rt = 3.30 minutes.
LC/MS (Luna 2, Gradient 4): rt = 1.94 minutes, 560 (MH⁺).

### Example 32

### 3-(Aminomethyl)benzoyl-D/L-phenylglycine 3-(4-methylpiperidine)carbonyl-4,5,6,7-tetrahydrobenzo(b)thiophen-2-amide trifluoroacetate salt

Prepared from 4-methylpiperidine.
¹H NMR (d₃ acetonitrile): (Spectrum complicated by rotomers) 9.22-9.02 & 7.86-6.70 (various) 5.75-5.58 (1 H, m, α-CH), 4.30-0.65 (various aliphatic).
Hplc (Luna 2, Gradient 1): rt = 4.14 minutes.
LC/MS (Luna 2, Gradient 4): rt = 2.37 minutes, 545 (MH⁺).

### Example 33

### 3-(Aminomethyl)benzoyl-D/L-phenylglycine 3-(4-aminocarbonylpiperidine)carbonyl-4,5,6,7-tetrahydrobenzo(b)thiophen-2-amide trifluoroacetate salt

Prepared from 4-aminocarbonylpiperidine.
¹H NMR (d₃ acetonitrile): (Spectrum complicated by rotomers) 9.51-6.35 (various) 5.78-5.62 (1 H, m, α-CH), 4.15-1.18 (various aliphatic).
Hplc (Luna 2, Gradient 1): rt = 3.48 minutes.
LC/MS (Luna 2, Gradient 4): rt = 1.72 minutes, 574 (MH⁺).

### Example 34

### 3-(Aminomethyl)benzoyl-D/L-phenylglycine 4,5,6,7-tetrahydro-3-(piperazinecarbonyl)benzo(b)thiophen-2-amide bis-trifluoroacetate salt

Prepared from piperazine.
Hplc (Luna 2, Gradient 1): rt = 3.07 minutes.
LC/MS (Luna 2, Gradient 4): rt = 1.40 minutes, 532 (MH⁺).

### Example 35

### 3-(Aminomethyl)benzoyl-D/L-phenylglycine 3-(4-ethoxycarbonyl)piperazinecarbonyl-4,5,6,7-tetrahydrobenzo(b)thiophen-2-amide trifluoroacetate salt

Prepared from 4-ethoxycarbonylpiperazine.
¹H NMR (d₃ acetonitrile): (Spectrum complicated by rotomers) 9.22-9.08 & 7.85-6.72 (various) 5.78-5.68 (1 H, m, α-CH), 4.09-3.85 (4 H, m, CH₂CH₃ & ArCH₂NH₂), 3.51-2.10 (various aliphatic), 1.70-1.51 (4 H, m, CH₂CH₂CH₂CH₂), 1.13-1.00 (3 H, m, CH₂CH₃).
Hplc (Luna 2, Gradient 1): rt = 3.77 minutes.
LC/MS (Luna 2, Gradient 4): rt = 2.26 minutes, 604 (MH⁺).

### Example 36

### 3-(Aminomethyl)benzoyl-D/L-phenylglycine 4,5,6,7-tetrahydro-3-[(pyrid-4-ylethyl)aminocarbonyl]benzo (b)thiophen-2-amide trifluoroacetate salt

Prepared from pyrid-4-ylethylamine.
¹H NMR (d₆ DMSO): 9.32-9.23 (1 H, d, J 7 Hz, NH), 8.69 & 7.72 (2 x 2H, 2 x d, 2 x J 9 Hz, pyridyl), 8.25 (3 H, br s, NH₃⁺), 8.01-7.30 (11 H, m, 3 x NH, 8 x Ar), 5.91 (1 H, d, J 6 Hz, α-CH), 4.05 (2 H, br d, J 4 Hz, ArCH₂NH₂), 3.01 (2 H, t, J 6 Hz, NHCH₂CH₂) 2.60 (2 H, t, J 6 Hz, NHCH₂CH₂), CH₂CH₂CH₂CH₂ peaks obscured by solvent peak at 2.50ppm, 1.72-1.58 (4 H, m, CH₂CH₂CH₂CH₂).
Hplc (Luna 2, Gradient 1): rt = 3.32 minutes.
LC/MS (Luna 2, Gradient 4): rt = 1.67 minutes, 568 (MH⁺).

### Example 37

### 3-(Aminomethyl)benzoyl-D/L-phenylglycine 3-(1-ethyl-1H-pyrazol-5-yl)aminocarbonyl-9,5,6,7-tetrahydrobenzo(b)thiophen-2-amide trifluoroacetate salt

Prepared from 1-ethyl-*1H*-pyrazol-5-ylamine
¹H NMR (d₃ acetonitrile): 8.01-7.84 & 7.63-7.39 (3 H & 10 H, 2 x m, Ar, 3 x NH), 7.00 (3 H, br s, NH₃⁺), 6.34 (1 H, s, pyrazolyl C⁴-H), 5.81 (1 H, d, J 7 Hz, α-CH), 4.20-4.09 (4 H, m, CH₂CH₃ & ArCH₂NH₂), 2.85-2.77 & 2.64-2.56 (2 x 2 H, 2 x m, CH₂CH₂CH₂CH₂), 1.85-1.73 (4 H, m, CH₂CH₂CH₂CH₂), 1.30 (3 H, t, J 7 Hz, CH₂CH₃).
Hplc (Luna 2, Gradient 1): rt = 4.22 minutes.
LC/MS (Luna 2, Gradient 4): rt = 2.16 minutes, 557 (MH⁺).

For the synthesis of examples 38 - 56 the following general procedures were used for synthesis of appropriate 2-aminothiophenes.

### General Procedure A (two step route).

Cyclohexanone (or a substituted cyclohexanone, pyridone, cycloheptanone, etc) (10 mmol), malononitrile (or a suitably substituted acetonitrile such as ethyl cyanoacetate, methanesulfonylacetonitrile, etc) (10 mmol), acetic acid (8 mmol) and ammonium acetate (2 mmol) were dissolved in benzene (25 mL). The reaction vessel was equipped with a Dean-Stark collector and heated to reflux until water evolution ceased (typically 2-14 hours). The mixture was then cooled to room temperature, diluted to 100 mL with ethyl acetate, and extracted twice with sodium bicarbonate solution (sat., aq., 50 mL). Volatile components of the reaction mixture were then removed under reduced pressure to afford a crude olefin/mixture of olefins. The crude product was then dissolved in ethanol (20 mL), and sulfur (10 mmol) and morpholine (10 mmol) were added. The mixture was then heated to reflux until TLC indicated complete consumption of starting material (typically 30 mins - 22 hours). After cooling, the reaction mixture was diluted to 100 mL with ethyl acetate and extracted with hydrochloric acid (1N, 2 x 30 mL). The organic portion was then concentrated under reduced pressure to afford crude product. Where the product was above 80% pure by HPLC no purification was used, but in some cases the product was purified by flash column chromatography (SiO₂, typical solvent mixture EtOAc:Hexane 1:1).

### General Procedure B (one pot route).

A mixture of the ketone (10 mmol), the acetonitrile derivative (10 mmol) and sulphur (10 mmol) were dissolved in ethanol. The mixture was heated to reflux, and morpholine (15 mmol) was added dropwise over 2 hours. After a further 2 hours, the reaction mixture was cooled, diluted to 100 mL with ethyl acetate, and extracted with hydrochloric acid (1N, 2 x 25 mL). The solution was then concentrated under reduced pressure, and the crude product purified by flash column chromatography (SiO₂, typical solvent mixture EtOAc:Hexane 1:1).

### Example 38

### 3-(Aminomethyl)benzoyl-D/L-phenylglycine 3-cyano-4,5,6,7-tetrahydrobenzo(b)thiophen-2-amide

Prepared from 2-amino-3-cyano-4,5,6,7-tetrahydrobenzo(b)thiophene (synthesised using general procedure A).

To a solution of 2-amino-3-cyano-4,5,6,7-tetrahydrobenzo(b)thiophene (230 mg, 1.29 mmol) in DCM (4 mL) was added N-Boc-D-Phg (340 mg, 1.35 mmol) followed by PyBrOP (631 mg, 1.35 mmol). The mixture was cooled to 0°C and diisopropylethylamine (550 mg, 4.26 mmol) was added dropwise. The mixture was then allowed to warm to room temperature and stir overnight. The reaction was then quenched by the addition of hydrochloric acid (0.5 N, aq., 25 mL) and extracted with DCM (2 x 25 mL) The combined organic layers were then evaporated and the crude product purified by flash column chromatography (SiO₂, acetone:hexane 1:5) to afford the coupled product (146 mg) as a pale yellow solid. The solid was dissolved in DCM (5 mL) and TFA (2 mL) was added. After stirring for 2 hours, the reaction mixture was evaporated and taken up in DMF (5 mL). 3-(*N*-Boc-aminomethyl)benzoic acid (100 mg, 0.40 mmol) was then added, followed by EDC (200 mg, 1.04 mmol), DMAP (5 mg, cat.) and diisopropylethylamine (180 mg, 1.39 mmol). The reaction mixture was allowed to stir for 16 hours, diluted with ethyl acetate (50 mL), and then extracted with hydrochloric acid (1 N, aq., 25 mL), sodium bicarbonate (saturated aq., 25 mL) and water (25 mL). The solution was dried over magnesium sulfate, evaporated, and purified by flash column chromatography (SiO₂, acetone:hexane 1:4) to afford the coupled product (125 mg) as a white solid. The product was then dissolved in DCM (5 mL) and TFA (2 mL) was added. After stirring for 2 hours, the reaction mixture was evaporated, dissolved in water (50 mL) and lyophillised to afford 3-(aminomethyl)benzoyl-D/L-phenylglycine 3-cyano-4,5,6,7-tetrahydrobenzo(b)thiophen-2-amide as its trifluoroacetate salt (120 mg, 0.21 mmol, 17 %) as a white solid.
¹H NMR (d₃ acetonitrile): 9.80 (1 H, s, H-bonded NH), 7.90-7.71 & 7.58-7.27 (4 H & 6 H, 2 x m, NH & 9 x Ar), 6.81 (3 H, br s, NH₃⁺), 5.86 (1 H, d, J 6 Hz, α-CH), 4.09 & 4.05 (2 H, ABq, J 6 Hz, ArCH₂NH₂), 2.52-2.34 (4 H, m, CH₂CH₂CH₂CH₂), 1.75-1.61 (4 H, m, CH₂CH₂CH₂CH₂).
Hplc (Luna 2, Gradient 1): rt = 4.31 minutes.
LC/MS (Luna 2, Gradient 4): rt = 2.21 minutes, 445 (MH⁺).

Examples 39 - 56 were synthesised using the same method as example 38 but using the indicated amine, which was in turn synthesised using general procedure A or B.

### Example 39

### 3-(Aminomethyl)benzoyl-D/L-phenylglycine 3-(2-thenoyl)-9,5,6,7-tetrahydrobenzo(b)thiophen-2-amide trifluoroacetate salt.

Prepared from 2-amino-3-(2-thenoyl)-4,5,6,7-tetrahydrobenzo(b)thiophene (synthesised using general procedure A).
¹H NMR (d₃ acetonitrile): 10.21 (1 H, s, H-bonded NH), 8.11-7.23 (15 H, 4 x NH & 11 x Ar), 6.85 (1H, t, J 3 Hz, thiophene C(4)H), 5.65 (1 H, d, J 6 Hz, α-CH), 4.02 (2 H, s, ArCH₂NH₂), 2.57-2.18 (4 H, m, CH₂CH₂CH₂CH₂), 1.78-1.42 (4 H, m, CH₂CH₂CH₂CH₂).
Hplc (Luna 2, Gradient 1): rt = 4.62 minutes.
LC/MS (Luna 2, Gradient 4): rt = 2.37 minutes, 530 (MH⁺).

### Example 40

### 3-(Aminomethyl)benzoyl-D/L-phenylglycine 3-pyrrolidinoyl-4,5,6,7-tetrahydrobenzo(b)thiophen-2-amide trifluoroacetate salt

Prepared from 2-amino-3-pyrrolidinoyl-4,5,6,7-tetrahydrobenzo(b)thiophene (synthesised using general procedure A)
¹H NMR (d₃ acetonitrile) : (Spectrum complicated by rotomers) 9.31 (1 H, s, H-bonded NH), 8.06-6.85 (13 H, m, 4 x NH & 9 x Ar), 5.81 (1 H, d, J 7 Hz, α-CH), 4.15 (2 H, br d, J 7 Hz, ArCH₂NH₂), 3.46-1.60 (m, various aliphatic).
Hplc (Luna 2, Gradient 1): rt = 3.96 minutes.
LC/MS (Luna 2, Gradient 4): rt = 2.50 minutes, 517 (MH⁺).

### Example 41

### 3-(Aminomethyl)benzoyl-D/L-phenylglycine 3-dimethylaminocarbonyl-4,5,6,7-tetrahydrobenzo(b)thiophene-2-amide trifluoroacetate salt

Prepared from 2-amino-3-dimethylaminocarbonyl-4,5,6,7-tetrahydrobenzo(b)thiophene (synthesised using general procedure A)
¹H NMR (d₃ acetonitrile): (Spectrum complicated by rotomers) 9.51 (1 H, br s, H-bonded NH), 8.01-7.20 (13 H, m, 4 x NH & 9 x Ar), 5.65 (1 H, br s, α-CH), 4.15-3.80 (2 H, m, ArCH₂NH₂), 2.90-1.55 (various aliphatic).
Hplc (Luna 2, Gradient 1): rt = 3.92 minutes.
LC/MS (Luna 2, Gradient 4): rt = 2.02 minutes, 491 (MH⁺).

### Example 42

### 3-(Aminomethyl)benzoyl-DL-phenylglycine 3-ethoxycarbonyl-4,5,6,7-tetrahydro-6-oxabenzo(b)thiophene-2-amide trifluoroacetate salt

Synthesised using 3-ethoxycarbonyl-4,5,6,7-tetrahydro-6-oxabenzo(b)thiophene-2-amine (prepared according to general procedure A).
¹H NMR (d₃ acetonitrile): 7.95-7.75 & 7.46-7.24 (3 H & 8H, 2 x m, Ar & NH's), 6.71 (3 H, br s, NH₃⁺), 5.73 (1 H, d, J 6 Hz, α-CH), 4.42 (2 H, s, ring CH₂O), 4.10-3.97 (4 H, m, CH₂CH₃ & ArCH₂NH₂), 3.66 & 2.65 (2 x 2 H, 2 x t, 2 x J 7 Hz, CH₂CH₂), 1.10 (3 H, t, J 7 Hz, CH₃).
Hplc (Luna 2, Gradient 1): rt = 4.272 minutes.
LC/MS (Luna 2, Gradient 4): rt = 2.18 minutes, 494 (MH⁺).

### Example 43

### 3-(Aminomethyl)benzoyl-D/L-phenylglycine 3-ethoxycarbonyl-cyclohepta(b)thiophen-2-amide trifluoroacetate salt

Synthesised from 2-amino-3-ethoxycarbonyl cyclohepta[b]thiophene (prepared according to general procedure A)
¹H NMR (d₃ acetonitrile): 8.20 & 8.01-7.39 (1 H & 10 H, d, J 8 Hz & m, Ar & 2 x NH), 6.98 (3 H, br s, NH₃⁺), 5.91 (1 H, d, J 7 Hz, α-CH), 4.22-4.09 (4 H, m, CH₂CH₃ & ArCH₂NH₂), 3.02-2.93, 2.70-2.60, 1.82-1.71 & 1.65-1.50 (2 H, 2 H, 2 H & 4 H, aliphatic ring H's), 1.30-1.15 (3 H, m, CH₃).
Hplc (Luna 2, Gradient 1): rt = 5.24 minutes.
LC/MS (Luna 2, Gradient 4): rt = 2.63 minutes, 506 (MH⁺).

### Example 44

### 3-(Aminomethyl)benzoyl-D/L-phenylglycine 3-ethoxycarbonyl-6-methyl-4,5,6,7-tetrahydrobenzo(b)thiophen-2-amide trifluoroacetate salt

Synthesised as a mixture of diastereomers from 2-amino-3-ethoxycarbonyl-6-methyl-4,5,6,7-tetrahydrobenzothiophene (prepared according to general procedure A)
¹H NMR (d₃ acetonitrile): 8.02-7.85 & 7.62-7.38 (3 H & 8 H, 2 x m, Ar & 2 x NH), 6.83 (3 H, br s, NH₃⁺), 5.89 (1 H, d, J 7 Hz, α-CH), 4.29-4.13 (4 H, m, CH₂CH₃ & ArCH₂NH₂), 2.99-1.20 (7 H, m, aliphatic ring H's), 1.30-1.18 (3 H, m, CH₃), 1.08-0.99 (3 H, m, CHCH₃).
Hplc (Luna 2, Gradient 1): rt = 5.35 minutes.
LC/MS (Luna 2, Gradient 4): rt = 2.78 minutes, 506 (MH⁺).

### Example 45

### 3-(Aminomethyl)benzoyl-D/L-phenylglycine 3-ethoxycarbonyl-4,5,6,7-tetrahydro-6-thiabenzo(b)thiophene-2-amide trifluoroacetate salt

Synthesised using 3-ethoxycarbonyl-4,5,6,7-tetrahydro-6-thiabenzo(b)thiophene-2-amine (prepared according to general procedure A).
¹H NMR (d₃ acetonitrile): 8.01-7.85 & 7.65-7.38 (3 H & 8H, 2 x m, Ar & NH's), 6.81 (3 H, br s, NH₃⁺), 5.84 (1 H, d, J 6 Hz, α-CH), 4.30-4.15 (4 H, m, CH₂CH₃ & ArCH₂NH₂), 3.68 (2 H, s, ring CH₂S), 3.05-2.81 (4 H, m, CH₂CH₂), 1.25 (3 H, t, J 7 Hz, CH₃).
Hplc (Luna 2, Gradient 1): rt = 4.64 minutes.
LC/MS (Luna 2, Gradient 4): rt = 2.40 minutes, 510 (MH⁺).

### Example 46

### 3-(Aminomethyl)benzoyl-D/L-phenylglycine 3-ethoxycarbonyl-5-methyl-4,5,6,7-tetrahydrobenzo(b)thiophen-2-amide trifluoroacetate salt

Synthesised as a mixture of diastereomers from 2-amino-3-ethoxycarbonyl-5-methyl-4,5,6,7-tetrahydrobenzothiophene (prepared according to general procedure A)
¹H NMR (d₃ acetonitrile): 8.19-7.85 & 7.65-7.36 (3 H & 8 H, 2 x m, Ar & 2 x NH), 6.88 (3 H, br s, NH₃⁺), 5.90 (1 H, d, J 7 Hz, α-CH), 4.28-4.14 (4 H, m, CH₂CH₃ & ArCH₂NH₂), 3.00-1.22 (7 H, m, aliphatic ring H's), 1.30-1.19 (3 H, m, CH₃), 1.06 (3 H, d, J 7 Hz, CHCH₃).
Hplc (Luna 2, Gradient 1): rt = 5.20 minutes.
LC/MS (Luna 2, Gradient 4): rt = 2.61 minutes, 506 (MH⁺).

### Example 47

### 3-(Aminomethyl)benzoyl-D/Z-phenylglycine 3-ethoxycarbonyl-4-methyl-4,5,6,7-tetrahydrobenzo(b)thiophen-2-amide trifluoroacetate salt

Synthesised as a mixture of diastereomers from 2-amino-3-ethoxycarbonyl-4-methyl-4,5,6,7-tetrahydrobenzothiophene (prepared according to general procedure A)
¹H NMR (d₃ acetonitrile): 8.06-7.88 & 7.65-7.40 (3 H & 8 H, 2 x m, Ar & 2 x NH), 6.87 (3 H, br s, NH₃⁺), 5.94-5.86 (1 H, m, α-CH), 4.36-4.15 (4 H, m, CH₂CH₃ & ArCH₂NH₂), 3.36-3.24 (1 H, m, CHCH₃), 2.71-2.48 (2 H, m, CH₂CS), 1.91-1.62 (4 H, m, CH₂CH₂), 1.31-1.22 (3 H, m, CH₃), 1.18 & 1.14 (3 H, 2 x d, 2 x J 7 Hz, CHCH₃ for each diastereomer).
Hplc (Luna 2, Gradient 1): rt = 5.14 minutes.
LC/MS (Luna 2, Gradient 4): rt = 2.59 minutes, 506 (MH⁺).

### Example 48

### 3-(Aminomethyl)benzoyl-D/L-phenylglycine 3-ethoxycarbonyl-cyclopenta(b)thiophen-2-amide trifluoroacetate salt

Prepared from 2-amino-3-ethoxycarbonylcyclopenta(b)-thiophene (synthesised using general procedure A).
¹H NMR (d₃ acetonitrile): 8.00-7.78 & 7.52-7.30 (3 H & 8 H, 2 x NH & 9 x Ar), 6.86 (3 H, br s, NH₃⁺), 5.82 (1 H, d, J 7 Hz, α-CH), 4.20-4.08 (4 H, m, CH₂CH₃ & ArCH₂NH₂), 2.80-2.65 (4 H, m, CH₂CH₂CH₂), 2.25 (2 H, pentet, J 7 Hz, CH₂CH₂CH₂), 1.18 (3 H, t, J 7 Hz, CH₂CH₃).
Hplc (Luna 2, Gradient 1) : rt = 4.71 minutes.
LC/MS (Luna 2, Gradient 4): rt = 2.47 minutes, 478 (MH⁺).

### Example 49

### 3-(Aminomethyl)benzoyl-D/L-phenylglycine 3-ethoxycarbonyl-4,6,6-trimethylcyclopenta(b)thiophen-2-amide trifluoroacetate salt

Prepared as a mixture of diastereomers from 2-amino-3-ethoxycarbonyl-4,6,6-trimethylcyclopenta[b]thiophene (synthesised using general procedure A).
¹H NMR (d₃ acetonitrile): 8.10-7.10 (14 H, 5 x NH & 9 x Ar), 5.70-5.58 (1 H, m, α-CH), 4.10-3.99 (4 H, m, CH₂CH₃ & ArCH₂NH₂), 3.15-2.95 (1 H, m, CHCH₃), 2.40-2.10 (2 H, m, CH₂CH₂CH₂), 1.18-1.01 (3 H, m, CH₂CH₃).
Hplc (Luna 2, Gradient 1): rt = 5.60 minutes.
LC/MS (Luna 2, Gradient 4): rt = 2.79 minutes, 520 (MH⁺).

### Example 50

### 3-(Aminomethyl)benzoyl-D/L-phenylglycine 3-ethoxycarbonyl-4,5,6,7-tetrahydro-4,7-methanobenzo(b)thiophen-2-amide trifluoroacetate salt

Prepared as a mixture of diastereomers from 2-amino-3-ethoxycarbonyl-4,5,6,7-tetrahydro-4,7-methanobenzo(b)-thiophene (synthesised using general procedure A).
¹H NMR (d₃ acetonitrile): 8.30-7.26 (14 H, 5 x NH & 9 x Ar), 5.82-5.72 (1 H, m, α-CH), 4.25-4.06 (4 H, m, CH₂CH₃ & ArCH₂NH₂), 3.68-3.60 & 3.41-3.32 (2 x 1 H, 2 x m, 2 x CHCH₂), 1.90-1.18 (6 H, m, ring CH₂'s), 1.25-1.14 (3 H, m, CH₂CH₃).
Hplc (Luna 2, Gradient 1): rt = 5.12 minutes.
LC/MS (Luna 2, Gradient 4): rt = 2.60 minutes, 504 (MH⁺).

### Example 51

### 3-(Aminomethyl)benzoyl-D/L-phenylglycine 3-(pyrid-2-yl)-4,5,6,7-tetrahydrobenzo(b)thiophen-2-amide trifluoroacetate salt

Prepared from 2-amino-3-(pyrid-2-yl)-4,5,6,7-tetrahydrobenzo(b)thiophene (synthesised using general procedure A).
¹H NMR (d₆ DMSO): 9.40 (1 H, dd, J 9, 1 Hz, pyridyl C (6)H) , 8.30 (3 H, br s, NH₃⁺), 8.10-7.19 (14 H, 2 x NH & 12 x Ar), 5.97 (1 H, d, J 6 Hz, α-CH), 4.18-4.08 (2 H, m, ArCH₂NH₂), 2.75-2.60 (4 H, m, CH₂CH₂CH₂CH₂), 1.85-1.62 (4 H, m, CH₂CH₂CH₂CH₂).
Hplc (Luna 2, Gradient 1): rt = 3.85 minutes.
LC/MS (Luna 2, Gradient 4): rt = 2.44 minutes, 497 (MH⁺).

### Example 52

### 3-(Aminomethyl)benzoyl-D/L-phenylglycine 3-methanesulfonyl-4,5,6,7-tetrahydrobenzo(b)thiophen-2-amide trifluoroacetate salt

prepared from 2-amino-3-methanesulfonyl-4,5,6,7-tetrahydrobenzo(b)thiophene (synthesised using general procedure B).
¹H NMR (d₆ DMSO): 10.97 (1 H, s, H-bonded NH), 9.52 (1 H, d, J 6 Hz, NHCH), 8.31 (3 H, br s, NH₃⁺), 8.10-7.99 & 7.67-7.35 (2 H & 7 H, Ar), 5.99 (1 H, d, J 6 Hz, α-CH), 4.15-4.02 (2 H, m, ArCH₂NH₂), 3.21 (3 H, s, CH₃), 2.72-2.59 (4 H, m, CH₂CH₂CH₂CH₂), 1.80-1.67 (4 H, m, CH₂CH₂CH₂CH₂).
Hplc (Luna 2, Gradient 1): rt = 4.25 minutes.
LC/MS (Luna 2, Gradient 4): rt = 2.17 minutes, 498 (MH⁺).

### Example 53

### 3-(Aminomethyl)benzoyl-D/L-phenylglycine 3-methoxcarbonyl-4,5,6,7-tetrahydrobenzo(b)thiophen-2-amide trifluoroacetate salt

Prepared from 2-amino-3-methoxycarbonyl-4,5,6,7-tetrahydrobenzo(b)thiophene (synthesised using general procedure A).
¹H NMR (d₆ DMSO): 9.58 (1 H, d, J 6 Hz, NHCH), 8.42 (3 H, br s, NH₃⁺), 8.20-8.10 & 7.83-7.50 (2 H & 8 H, NH & Ar), 6.13 (1 H, d, J 6 Hz, α-CH), 4.29-4.18 (2 H, m, ArCH₂NH₂), 3.81 (3 H, s, CH₃), 2.85-2.61 (4 H, m, CH₂CH₂CH₂CH₂), 1.91-1.78 (4 H, m, CH₂CH₂CH₂CH₂).
Hplc (Luna 2, Gradient 1): rt = 4.71 minutes.
LC/MS (Luna 2, Gradient 4): rt = 2.49 minutes, 478 (MH⁺).

### Example 54

### 3-(Aminomethyl)benzoyl-D/L-phenylglycine 3-aminocarbonyl-4,5,6,7-tetrahydrobenzo(b)thiophen-2-amide trifluoroacetate salt

Prepared from 2-amino-3-aminocarbonyl-4,5,6,7-tetrahydrobenzo(b)thiophene (synthesised using general procedure A).
¹H NMR (d₆ DMSO): 9.39 (1 H, d, J 6 Hz, NHCH), 8.35 (3 H, br s, NH₃⁺), 8.05-7.98 & 7.62-7.30 (2 H & 8 H, NH & Ar), 5.83 (1 H, d, J 6 Hz, α-CH), 4.10-3.97 (2 H, m, ArCH₂NH₂), 3.81 (3 H, s, CH₃), 3.00-2.51 (4 H, m, CH₂CH₂CH₂CH₂), 1.82-1.70 (4 H, m, CH₂CH₂CH₂CH₂).
Hplc (Luna 2, Gradient 1): rt = 4.01 minutes.
LC/MS (Luna 2, Gradient 4): rt = 2.10 minutes, 463 (MH⁺).

### Example 55

### 3-(Aminomethyl)benzoyl-DL-phenylglycine 3-cyano-7-oxo-4,5,6,7-tetrahydrobenzo(b)thiophen-2-amide trifluoroacetate salt

Prepared from 2-amino-3-cyano-7-oxo-4,5,6,7-tetrahydrobenzo(b)thiophene (synthesised using general procedure B).
¹H NMR (d₆ DMSO): 9.14 (1 H, d, J 6 Hz, NHCH), 8.20 (3 H, br s, NH₃⁺), 7.99-7.26 (10 H, NH & Ar), 6.11 (1 H, d, J 6 Hz, α-CH), 4.03-3.92 (2 H, m, ArCH₂NH₂), 2.75-2.68 (2 H, m, CH₂CH₂CH₂CO), 2.05-1.92 (4 H, m, CH₂CH₂CH₂), CH₂CO peak not observed, presumably under water peak at 2.40ppm.
Hplc (Luna 2, Gradient 1): rt = 3.77 minutes.
LC/MS (Luna 2, Gradient 4): rt = 1.91 minutes, 459 (MH⁺).

### Example 56

### 3-(Aminomethyl)benzoyl-D/L-phenylglycine 6-aza-6-benzyloxycarbonyl-3-dimethylaminocarbonyl-4,5,6,7-tetrahydrobenzo(b)thiophene-2-amide trifluoroacetate salt

Synthesised using 6-aza-6-benzyloxycarbonyl-3-dimethylaminocarbonyl-4,5,6,7-tetrahydrobenzo(b)thiophene-2-amine (synthesised using general procedure B).
¹H NMR (d₃ acetonitrile): (Spectrum complicated by rotomers) 9.50 (1 H, br s, NH), 8.05-7.30 (10 H, NH & 9 x Ar), 6.90 (3 H, br s, NH₃⁺), 5.81 (1 H, d, J 7 Hz, α-CH), 5.11 (2 H, br s, CH₂Ph), 4.60-4.49 (2 H, m, ring CH₂N), 4.20-4.16 (2 H, m, ArCH₂NH₂), 3.71-3.58 (2 H, m, CH₂CH₂N), 3.01 & 2.89 (2 x 3 H, 2 x s, 2 x CH₃), 2.60-2.48 (2 H, m, CH₂CH₂N).
Hplc (Luna 2, Gradient 1): rt = 4.13 minutes.
LC/MS (Luna 2, Gradient 4): rt = 2.25 minutes, 626 (MH⁺).

Examples 57 - 61 were prepared using 3-(*N*-BOC-aminomethyl)benzoyl-D/L-phenylglycine 6-aza-3-dimethylamidocarbonyl-4,5,6,7-tetrahydrobenzo-(b)thiophene, made from an intermediate of the route to example 56, using standard deprotection and coupling methodology.

### Example 57

### 3-(Aminomethyl)benzoyl-D/L-phenylglycine 6-aza-3-dimethylaminocarbonyl-4,5,6,7-tetrahydrobenzo(b)thiophene 2-amide bis(trifluoroacetate) salt

¹H NMR (d₃ acetonitrile): (Spectrum complicated by rotomers) 10.12 (1 H, br s, NH), 8.60-7.25 (15 H, 6 x NH & 9 x Ar), 5.91-5.70 (1 H, m, α-CH), 4.30-4.14 (4 H, m, ring CH₂N & ArCH₂NH₂), 3.59-3.30 (2 H, m, CH₂CH₂N), 3.00-2.30 (8 H, m, 2 x CH₃ & CH₂CH₂N).
Hplc (Luna 2, Gradient 1): rt = 2.59 minutes.
LC/MS (Luna 2, Gradient 4): rt = 1.47 minutes, 492 (MH⁺).

### Example 58

### 3-(Aminomethyl)benzoyl-D/L-phenylglycine 6-acetyl-6-aza-3-dimethylaminocarbonyl-4,5,6,7-tetrahydrobenzo-(b)thiophene-2-amide trifluoroacetate salt

¹H NMR (d₃ acetonitrile): (Spectrum complicated by rotomers) 9.65-9.45 (1 H, br s, NH), 7.85-6.73 (13 H, 4 x NH & 9 x Ar), 5.70-5.56 (1 H, m, α-CH), 4.60-9.33 (2 H, m, ring CH₂N), 4.10-3.85 (2 H, m, ArCH₂NH₂), 3.69-3.31 (2 H, m, CH₂CH₂N), 2.90-2.30 (8 H, m, 2 x CH₃ & CH₂CH₂N), 1.95 (3 H, s, CH₃CO).
Hplc (Luna 2, Gradient 1): rt = 2.59 minutes.
LC/MS (Luna 2, Gradient 4): rt = 1.47 minutes, 492 (MH⁺).

### Example 59

### 3-(Aminomethyl)benzoyl-D/L-phenylglycine 6-aminoacetyl-6-aza-3-dimethylaminoearbonyl-4,5,6,7-tetrahydrobenzo(b)thiophene-2-amide trifluoroacetate salt

¹H NMR (d₃ acetonitrile): (Spectrum complicated by rotomers) 9.60-9.48 (1 H, br s, NH), 7.80-6.81 (16 H, 7 x NH & 9 x Ar), 5.60-5.49 (1 H, m, α-CH), 4.41 & 4.33 (2 x 1H, ABq, J 12 Hz, ring CH₂N), 3.95-3.83 (2 H, m, ArCH₂NH₂), 3.71-3.25 (4 H, m, NCH₂CO & CH₂CH₂N), 2.68-2.25 (8 H, m, 2 x CH₃ & CH₂CH₂N).
Hplc (Luna 2, Gradient 1): rt = 2.69 minutes.
LC/MS (Luna 2, Gradient 4): rt = 1.25 minutes, 549 (MH⁺).

### Example 60

### 3-(Aminomethyl)benzoyl-D/L-phenylglycine 6-aza-6-(3-methylbutanoyl)-3-dimethylaminocarbonyl-4,5,6,7-tetrahydrobenzo-(b)thiophene-2-amide trifluoroacetate salt

¹H NMR (d₃ acetonitrile): (Spectrum complicated by rotomers) 9.73-9.40 (1 H, m, NH), 7.99-7.32 (10 H, NH & 9 x Ar), 6.95 (3 H, br s, NH₃⁺), 5.72 (1 H, d, J 6 Hz, α-CH), 4.58 & 4.52 (2 x 1H, Abq, J 12 Hz, ring CH₂N), 4.13-4.01 (2 H, m, ArCH₂NH₂), 3.82-3.59 (2 H, m, CH₂CH₂N), 2.91-2.40 (9 H, m, 2 x CH₃, CH₂CH₂N & CHMe₂), 0.89-0.80 (6 H, m, CH(CH₃)₃).
Hplc (Luna 2, Gradient 1): rt = 3.72 minutes.
LC/MS (Luna 2, Gradient 4): rt = 1.94 minutes, 576 (MH⁺).

### Example 61

### 3-(Aminomethyl)benzoyl-D/L-phenylglycine 6-aza-6-methoxyacetyl-3-dimethylaminocarbonyl-4,5,6,7-tetrahydrobenzo(b)thiophene-2-amide trifluoroacetate salt

¹H NMR (d₃ acetonitrile): (Spectrum complicated by rotomers) 10.50-10.18 (1 H, br s, NH), 7.90-6.80 (13 H, 4 x NH & 9 x Ar), 5.70-5.52 (1 H, m, α-CH), 4.80-2.20 (16 H, m, ArCH₂NH₂, NCH₂CO & CH₂CH₂N, 2 x CH₃ & CH₂CH₂N).
Hplc (Luna 2, Gradient 1): rt = 3.17 minutes.
LC/MS (Luna 2, Gradient 4): rt = 1.68 minutes, 564 (MH⁺).

### Example 62

### 3-(Aminomethyl)benzoyl-D/L-4-(aminomethyl)phenylglycine 3-ethoxycarbonyl-4,5,6,7-tetrahydrobenzo(b)thiophen-2-amide bis(trifluoroacetate) salt.

Prepared from 2-amino-3-ethoxycarbonyl-4,5,6,7-tetrahydrobenzo(b)thiophene using the same method as example 1 but using D/L-4-(*N*-BOC-aminomethyl)-α-(*N-*benzyloxycarbonyl)phenylglycine, synthesised as described below.

### Methyl 4-bromophenylacetate

Thionyl chloride (18 mL, 0.25 mol) was added dropwise to a solution of 4-bromophenylacetic acid (50 g; 0.23 mol) in methanol (250 mL). The resulting mixture was stirred at room temperature for 1 hour before the methanol was removed *in vacuo.* Ethyl acetate (300 mL) was added and the resulting solution was washed with water (3 x 150 mL) and 1M aqueous NaHCO₃ (1 x 150 mL), dried (MgSO₄) and evaporated to give the ester (52.8 g; 100 %) as an orange oil which was used without further purification.
¹H NMR (CDCl₃): 7.38 ppm (2 H, d, *J =* 8.4 Hz, C(2)H and C(6)H) ; 7.09 (2 H, d, *J =* 8.4 Hz, C(3)H and C(5)H); 3.63 (3 H, s, OMe) ; 3.51 (2 H, s, CH₂).

### Methyl 4-cyanophenylacetate

Zinc cyanide (10.4 g, 0.088 mol) and tetrakis(triphenylphosphine)palladium(0) (5 g, 4.4 mmol) were added to a solution of methyl 4-bromophenylacetate (20 g, 0.088 mol) in dimethylformamide (150 mL). The resulting mixture was stirred at 80°C for 5 hours, then allowed to cool to room temperature. Toluene (500 mL) and 1 M aqueous ammonia (500 mL) were added, the layers were separated and the organic layer washed with brine (100 mL) and dried (MgSO₄). Evaporation of the solvents afforded an off-white solid, which was purified by silica gel chromatography to afford the nitrile as a white solid (11.3 g; 73 %).
¹H NMR (CDCl₃) : 7.65 ppm (2 H, d, *J* = 8.4 Hz, C(3)H and C(5)H); 7.42(2 H, d, *J* = 8.1 Hz, C(2)H and C(6)H); 3.74 (3H, s, OMe); 3.72 (2H, s, CH₂).

### 4-Cyanophenylacetic acid

A solution of methyl 4-cyanophenylacetate (23.9 g; 0.136 mol) in ethanol (250 mL) was stirred at room temperature and a solution of sodium hydroxide (6.0 g; 0.15 mol) in water (25 mL) was added. After 2 hours the ethanol was removed *in vacuo.* Ethyl acetate (300 mL) and 5% aqueous HCl (300 mL) were added and the layers were separated. The aqueous layer was extracted with ethyl acetate (300 mL) and the combined organic layers were dried (MgSO₄) and evaporated *in vacuo* to give the acid (21.6 g; 99 %) which was used without further purification.
¹H NMR (CDCl₃) : 7.57 ppm (2 H, d, *J* = 8.3 Hz, C(3)H and C(5)H); 7.34 (2 H, d, *J* = 8.2 Hz, C(2)H and C(6)H); 3.64 (2 H, s, CH₂).

### 4-(N-BOC-Aminomethyl)phenylacetic acid

A solution of 4-cyanophenylacetic acid (12.11 g, 0.075 mol) in water (163 mL) and concentrated aqueous ammonia (40 mL) was stirred at room temperature and Raney nickel (6.3 g) was added. The resulting suspension was stirred under a hydrogen atmosphere for 24 hours before the reaction mixture was filtered through celite and evaporated *in vacuo* to give crude 4-(aminomethyl)-phenylacetic acid (12.57 g; 100 %) as a pale blue solid.
A solution of the crude amino acid (12.57 g, 0.075 mol) in water (50 mL) and 1,4-dioxane (50 mL) was stirred at room temperature and sodium hydroxide (3 g, 0.075 mol) and di-^{t}butyl dicarbonate (16.4 g, 0.075 mol) were added simultaneously. After 24 hours the 1,4-dioxane was removed *in vacuo* and the aqueous layer was acidified with saturated aqueous citric acid (200 mL). The solution was extracted with ethyl acetate (3 x 150 mL) and the combined organic layers were dried (MgSO₄) and evaporated *in vacuo* to give the N-BOC-amine (17.6 g, 88 %) as a white solid which was used without further purification.
¹H NMR (CDCl₃): 7.00 ppm (4 H, m, Ar); 4.65 (1 H, br s, N-H); 4.09 (2 H, d, *J =* 6 Hz, CH₂NH) ; 3.43 (2H, s, CH₂); 1.25 (9H, s, ^{t}Bu).

### Methyl 4-(N-BOC-aminomethyl)phenylacetate

1-[3-(Dimethylamino)propyl]-3-ethylcarbodiimide hydrochloride (34.8 g, 0.18 mol) and 4-(*N,N*-dimethylamino)pyridine (220 mg, 1.8 mmol) were added to a solution of 4-(*N*-BOC-aminomethyl)phenylacetic acid (47.8 g, 0.18 mol) in methanol (200 ml). After stirring for 18 hours the methanol was removed *in vacuo* and the reaction mixture partitioned between ethyl acetate (200 mL) and saturated aqueous citric acid (200 mL). The organic phase was separated and washed with saturated aqueous NaHCO₃ (200 mL) and brine (200 mL), dried (MgSO₄ and evaporated to give the methyl ester (49.8 g; 99 %).
¹H NMR (CDCl₃) : 7.42 ppm (4 H, s, Ar); 5.02 (1 H, br s, N-H); 4.48 (2 H, d, *J* = 5.7 Hz, CH₂NH) ; 3.87 (3 H, s, OMe); 3.79 (2 H, s, CH₂); 1. 64 (9 H, s, ^{t}Bu).

### Methyl 4-(N-BOC-aminomethyl)-α-azidophenylacetate

A solution of methyl 4-(*N*-BOC-aminomethyl)phenylacetate (9.34 g; 0.033 mol) in THF (100 mL) was stirred under argon at -78°C and potassium bis(trimethylsilyl)amide (16.7 g, 0.084 mol) in THF (50 mL) was added. After stirring for 30 minutes, 2,4,6-triisopropylbenzenesulfonyl azide (31.1 g, 0.101 mol) was added as a solid. After 5 minutes, acetic acid (10 mL, 0.175 mol) was added and the reaction warmed to room temperature.
The reaction mixture was then partitioned between ethyl acetate (500 mL) and water (500 mL), separated and the organic layer dried (MgSO₄). Evaporation of the solvent and purification of the residue by silica gel chromatography afforded the azide (7.1 g, 67 %).
¹H NMR (CDCl₃): 7.28 ppm (4 H, s, Ar); 4.92 (1 H, s, CHN₃); 4.25 (2 H, s, CH₂NH); 3.69 (3 H, s, OMe); 1.38 (9 H, s, ^{t}Bu).

### Methyl 4-(N-BOC-aminomethyl)-α-aminophenylacetate

A solution of methyl 4-(*N*-BOC-aminomethyl)-α-azidophenylacetate (7.1 g, 0.022 mol) in ethyl acetate (50 mL) was stirred over palladium on carbon (5%). The reaction vessel was taken up to 250 p.s.i. with hydrogen for 17 hours. The reaction mixture was filtered through celite and evaporated *in vacuo* to give the amine (6.47 g, 100 %) as a pale solid.
¹H NMR (CDCl₃) : 7.20 ppm (2 H, m, Ar); 7.12 (2 H, m, Ar); 4.81 (1 H, br s, NH); 4.45 (1 H, s, CH); 4.18 (2 H, d, *J* = 6 Hz, CH₂NH); 3.54 (3 H, s, OMe); 2.09 (2 H, br s, NH₂); 1.30 (9 H, s, ^{t}Bu).

### Methyl 4-(N-BOC-aminomethyl)-α-(N-benzyloxycarbonylamino)phenylacetate

A solution of the amine (530 mg, 1.8 mmol) in tetrahydrofuran (15 mL) was treated with triethylamine (0.25 mL, 1.8 mmol) and benzyl chloroformate (0.26 mL, 1.8 mmol) and allowed to stir at room temperature for 1 hour. The reaction was diluted with ethyl acetate (40 mL), washed with brine (2 x 25 mL), dried (MgSO₄) and concentrated under reduced pressure to afford a yellow oil. The benzyloxycarbonyl ester was purified by flash chromatography on silica gel (ethyl acetate / hexane 1 : 1) to give a yellow solid (312 mg, 66 %).
¹H NMR (CDCl₃): 7.32 - 7.15 ppm (9 H, m, 9 Ar); 5.80 (1 H, br s, NH); 5.30 (1 H, d, *J* = 9.6 Hz, CH); 5.01 (2 H, s, CH₂Ph); 4.22 (2 H, d, *J* = 7.2 Hz, CH₂NHBoc); 3.63 (3 H, s, OCH3); 1.39 (9 H, s, ^{t}Bu).

### D/L-4-(N-BOC-aminomethyl)-α-(N-benzyloxycarbonyl)-phenylglycine

A solution of the ester (356 mg, 0.83 mmol) in tetrahydrofuran (15 mL) was treated with 1 M LiOH (1.7 mL, 1.7 mmol) and heated at reflux for 3 hours. The solvent was removed under reduced pressure and the residue diluted with water (20 mL).

The pH was reduced to 4 using 5 % aqueous HCl and the aqueous phase was extracted with ethyl acetate (3 x 20 mL). The combined organic extracts were dried (MgSO₄) and concentrated under reduced pressure to afford the acid as a yellow solid (273 mg, 79 %) which was carried forward without further purification.

### 3-(Aminomethyl)benzoyl-D/L-4-(aminomethyl)phenylglycine 3-ethoxycarbonyl-4,5,6,7-tetrahydrobenzo(b)thiophen-2-amide bis(trifluoroacetate) salt.

¹H NMR (d₃ acetonitrile): 8.50, 8.01, 7.75-6.88 (1 H, 1 H, 14 H, d, J 6 Hz, s, m, Ar & NH's), 5.81 (1 H, d, J 8 Hz, α-CH), 4.15-3.95 (6 H, m, CH₂CH₃ & 2 x ArCH₂NH₂), 2.65-2.45 (4 H, m, CH₂CH₂CH₂CH₂), 1.72-1.60 (4 H, m, CH₂CH₂CH₂CH₂), 0.84 (3 H, t, J 7 Hz, CH₂CH₃).
Hplc (Luna 2, Gradient 1): rt = 3.382 minutes.
LC/MS (Luna 2, Gradient 4): rt = 2.17 minutes, 521 (MH⁺). Examples 63 - 70 were prepared in a manner analogous to Example 16 except that the indicated amine was used in place of 2-aminobenzothiazole.

### Example 63

### 3-(Aminomethyl)benzoyl-D/L-phenylglycine 6-nitrobenzothiazol-2-amide trifluoroacetate salt

Prepared from 2-amino-6-nitrobenzothiazole.
¹H NMR (d₄ methanol): 8.62 (1 H, s, Ar); 8.09 (1 H, d, *J* = 7.2 Hz, Ar); 7.94 - 7.81 (2 H, m, Ar); 7.67 - 7.23 (8 H, m, Ar); 5.88 (1 H, s, CH); 4.10 (2 H, s, CH₂NH₂).
HPLC (Luna 2, Gradient 1): rt = 3.76 minutes.
LCMS (Luna 2, Gradient 4): rt = 1.91 minutes, 462 (MH)⁺.

### Example 64

### 3-(Aminomethyl)benzoyl-D/L-phenylglycine 6-ethoxycarbonyl-benzothiazol-2-amide trifluoroacetate salt.

Prepared from 2-amino-6-ethoxycarbonylbenzothiazole
¹H NMR (d₄ methanol) : 8.57 (1 H, s, Ar); 8.09 (1 H, d, *J =* 7.2 Hz, Ar); 7.97 (1 H, d, *J* = 7.2 Hz, Ar); 7.70 - 7.35 (7 H, m, Ar); 5.92 (1 H, s, CH); 4.38 (2 H, q, *J =* 7 Hz, CH₂CH₃); 4.18 (2 H, s, CH₂NH₂); 1.41 (3 H, t, *J =* 7 Hz, CH₂CH₃).
HPLC (Luna 2, Gradient 1): rt = 3.88 minutes.
LCMS (Luna 2, Gradient 4): rt = 2.07 minutes, 489 (MH)⁺.

### Example 65

### 3-(Aminomethyl)benzoyl-D/L-phenylglycine 4,7-dimethoxybenzothiazol-2-amide trifluoroacetate salt.

Prepared from 2-amino-4,7-dimethoxybenzothiazole (synthesised as described below).

### 2-Amino-4,7-dimethoxybenzothiazole

Sodium thiocyanate (830 mg, 10.2 mmol) and 2,5-dimethoxyaniline (1.56 g, 10.2 mmol) were stirred together in methanol (10 mL) at -5°C under argon. Bromine (262 µL, 5.1 mmol) was added dropwise and the solution was stirred for 2 hours. The reaction was partitioned between ethylacetate (100 mL) and water (100 mL). The organic phase was washed with brine (100 mL), dried (MgSO₄) and concentrated under reduced pressure. The aminobenzothiazole was purified by flash column chromatography, using acetone 1/3 hexane as eluent, to afford a colourless solid (668 mg, 31%).
¹H NMR (CDCl₃): 6.90 (1 H, s, Ar); 6.34 (1 H, s, Ar); 4.20 (2 H, br s, NH₂); 3.84 (6 H, s, 2 x OCH₃).

### 3-(Aminomethyl)benzoyl-D/L-phenylglycine 4,7-dimethoxybenzothiazol-2-amide trifluoroacetate salt.

¹H NMR (d₄ methanol): 8.16 (1 H, s, Ar); 7.72 - 7.62 (2 H, m, Ar); 7.60 - 7.36 (9 H, m, Ar); 5.93 (1 H, s, CH); 3.97 (2 H, s, CH₂NH₂); 3.90 (3 H, s, OCH₃) ; 3.83 (3 H, s, OCH₃).
HPLC (Luna 2, Gradient 1): rt = 4.23 minutes.
LCMS (Luna 2, Gradient 4): rt = 2.22 minutes, 477 (MH)⁺.

### Example 66

### 3-(Aminomethyl)benzoyl-D/L-phenylglycine 4-methoxy-7-methylbenzothiazol-2-amide trifluoroacetate salt.

Prepared from 2-amino-4-methoxy-7-methylbenzothiazole, which was synthesised in a similar manner to 2-amino-4,7-dimethoxybenzothiazole described in example 65.

### 2-Amino-4-methoxy-7-methylbenzothiazole

¹H NMR (CDCl₃) : 6.96 (1 H, s, Ar) ; 6.59 (1 H, s, Ar) ; 4.12 (2 H, br s, NH₂) ; 3.83 (3 H, s, OCH₃) ; 2.40 (3 H, s, CH₃).
LCMS (Luna 2, Gradient 4): rt = 3.10 minutes, 194 (MH)⁺.

### 3-(Aminomethyl)benzoyl-D/L-phenylglycine 4-methoxy-7-methylbenzothiazol-2-amide trifluoroacetate salt.

¹H NMR (d₄ methanol): 8.08 (1 H, s, Ar); 7.92 - 7.82 (2 H, m, Ar); 7.61 - 7.45 (5 H, m, Ar); 7.35 - 7.23 (3 H, m, Ar); 7.10 (1 H, s, Ar); 5.93 (1 H, s, CH); 4.09 (2 H, s, CH₂NH₂); 3.70 (3 H, s, OCH₃); 2.30 (3 H, s, CH₃).
HPLC (Luna 2, Gradient 1): rt = 3.88 minutes.
LCMS (Luna 2, Gradient 4): rt = 2.22 minutes, 461 (MH)⁺.

### Example 67

### 3-(Aminomethyl)benzoyl-D/L-phenylglycine 6-methyl- 4,5,6,7-tetrahydrobenzothiazol-2-amide trifluoroacetate salt.

Prepared from 2-amino-6-methyl-4,5,6,7-tetrahydrobenzothiazole (prepared as described below).

### 2-Chloro-4-methylcyclohexanone.

To a stirred mixture of 4-methylcyclohexanone (897 mg, 8 mmol), Mn(acac)₃ (28 mg, 0.08 mmol) and moist alumina (4 g) in dichloromethane (80 mL) under argon was added finely pulverised sodium chlorite (1.8 g, 16 mmol) and the mixture was stirred vigorously for 16 h. The mixture was filtered through celite and the residue was concentrated under reduced pressure. Purification by flash column chromatography using ethyl acetate / hexane (1:9) as eluent afforded the chlorocyclohexanone as a colourless liquid (230 mg, 20 %).
¹H NMR (CDCl₃) : 4.15 (1 H, m, CHCl); 2.97 - 2.85 (1 H, m, chex); 2.36 - 2.10 (3 H, m, chex); 2.00 - 1.87 (1 H, m, chex); 1.85 - 1.73 (1 H, m, chex); 1.45 - 1.28 (1 H, m, chex); 0.97 (3 H, d, J = 7 Hz, CHCH₃).

### 2-Amino-6-methyl-4,5,6,7-tetrahydrobenzothiazole.

A mixture of 2-chloro-4-methylcyclohexanone (230 mg, 1.42 mmol) and thiourea (119 mg, 1.56 mmol) in THF (10 mL) was heated at reflux for 16 h. The mixture was concentrated under reduced pressure and the residue was purified by flash column chromatography using methanol / dichloromethane (1:9) as eluent to afford the amine as a yellow oil (87 mg, 36 %).
¹H NMR (CDCl₃) : 4.60 (2 H, br s, NH₂); 2.44 - 2.20 (3 H, m, chex); 2.03 - 1.87 (1 H, m, chex); 1.76 - 1.53 (2 H, m, chex); 1.31 - 1.16 (1 H, m, chex); 0.86 (3 H, d, *J* = 7 Hz, CHCH₃).

### 3-(Aminomethyl)benzoyl-D/L-phenylglycine 6-methyl- 4,5,6,7-tetrahydrobenzothiazol-2-amide trifluoroacetate salt.

¹H NMR (d₄ methanol): 8.06 - 7.93 (2 H, m, Ar); 7.67 - 7.63 (1 H, d, *J* = 7.2 Hz, Ar); 7. 60 - 7.51 (3 H, m, Ar); 7.49 - 7.33 (3 H, m, Ar); 5.90 (1H, s, CH); 4.22 (2 H, s, CH₂NH₂); 3.89 - 2.51 (3 H, m, chex); 2.40 - 2.22 (1 H, m, chex); 2.09 - 1.88 (2 H, m, chex); 1. 61 - 1.40 (1 H, m, chex); 1.11 (3 H, d, *J =* 7 Hz, CHCH₃).
HPLC (Luna 2, Gradient 1): rt = 4.12 minutes.
LCMS (Luna 2, Gradient 4): rt = 2.14 minutes, 435 (MH)⁺.

### Example 68

### 3-(Aminomethyl)benzoyl-D/L-phenylglycine 5-ethyl-4,5,6,7-tetrahydrobenzothiazol-2-amide trifluoroacetate salt.

Prepared from 2-amino-5-ethyl-4,5,6,7-tetrahydrobenzo-thiazole (prepared in an analogous fashion to 2-amino-6-methyl-4,5,6,7-tetrahydrobenzothiazole described in example 67).

### 6-chloro-3-ethylcyclohexanone.

¹H NMR (CDCl₃): 4.32 - 4.24 (1 H, m, CHCl); 3.12 - 2.94 (1 H, m, chex); 2.46 - 2.29 (2 H, m, chex); 2.22 - 2.06 (2 H, m, chex); 1.96 - 1.83 (1 H, m, chex); 1.56 - 1.30 (3 H, m, chex, CH₂CH₃); 1.03 (3 H, m, J = 7 Hz, CH₂CH₃).

### 2-Amino-5-ethyl-4,5,6,7-tetrahydrobenzothiazole.

¹H NMR (CDCl₃) : 5.01 (2 H, br s, NH₂); 2. 64 - 2.36 (2 H, m, chex); 2.19 - 2.03 (1 H, m, chex); 1.93 - 1.75 (2 H, m, chex); 1.69 - 1.55 (1 H, m, chex); 1.46 - 1.26 (3 H, m, chex, CH₂CH₃); 0.90 (3 H, m, J = 7 Hz, CH₂CH₃).

### 3-(Aminomethyl)benzoyl-D/L-phenylglycine 5-ethyl-4,5,6,7-tetrahydrobenzothiazol-2-amide trifluoroacetate salt

¹H NMR (d₄ methanol): 7.89 - 7.79 (2 H, m, Ar); 7.55 (1 H, d, J = 7.2 Hz, Ar); 7.50 - 7.41 (3 H, m, Ar); 7.38 - 7.23 (3 H, m, Ar); 5.87 (1 H, s, CH); 4.09 (2H, s, CH₂NH₂); 2.80 - 2.40 (3 H, m, chex); 2.29 - 2.12 (1 H, m, chex); 1.97 - 1.85 (1 H, m, chex); 1.70 - 1.53 (1 H, m, chex); 1.47 - 1.30 (3 H, m, chex, CH₂CH₃); 0.89 (3 H, t, *J =* 7 Hz, CH₂CH₃).
HPLC (Luna 2, Gradient): rt = 4.5 minutes.
LCMS (Luna 2, Gradient 4): rt = 2.32 minutes, 449 (MH)⁺.

### Example 69

### 3-(Aminomethyl)benzoyl-D/L-phenylglycine 5-methyl- 4,5,6,7-tetrahydrobenzothiazol-2-amide trifluoroacetate salt.

Prepared from 2-amino-5-methyl-4,5,6,7-tetrahydrobenzothiazole (prepared in an analogous fashion to 2-amino-6-methyl 4,5,6,7-tetrahydrobenzothiazole, described in example 67).

### 6-Chloro-3-methylcyclohexanone.

¹H NMR (CDCl₃) : 4.13 (1 H, m, CHCl); 2.60 - 2.49 (1 H, m, chex); 2.28 - 1.99 (3 H, m, chex); 1.97 - 1.80 (1 H, m, chex); 1.75 - 1.50 (2 H, m, chex); 1.03 - 0.91 (3 H, m, CHCH₃).

### 2-Amino-5-methyl-4,5,6,7-tetrahydrobenzothiazole.

¹H NMR (CDCl₃) : 4.71 (2 H, br s, NH₂); 2. 62 - 2.49 (2 H, m, chex); 2.15 - 1.76 (4 H, m, chex); 1.50 - 1.31 (1 H, m, chex); 1.01 (3 H, d, *J* = 7 Hz, CHCH₃).

### 3-(Aminomethyl)benzoyl-DL-phenylglycine 5-methyl- 4,5,6,7-tetrahydrobenzothiazol-2-amide trifluoroacetate salt.

¹H NMR (d₄ methanol) : 8.00 - 7.88 (2 H, m, Ar); 7.73 - 7.36 (7 H, m, Ar); 5.90 (1 H, s, CH) ; 4.20 (2 H, s, CH₂NH₂); 2.96 - 2.61 (3 H, m, chex); 2.31 - 2.16 (1 H, m, chex); 2.07 - 1.84 (2 H, m, chex); 1. 60 - 1.04 (5 H, m, chex, CHCH₃).
HPLC (Luna 2, Gradient 1): rt = 4.20 minutes.
LCMS (Luna 2, Gradient 4): rt = 2.10 minutes, 435 (MH)⁺.

### Example 70

### 3-(Aminomethyl)benzoyl-D/L-phenylglycine 7-ethyl-4,5, 6,7-tetrahydrobenzothiazol-2-amide trifluoroacetate salt.

Prepared from 2-amino-7-ethylidenyl-4,5,6,7-tetrahydrobenzothiazole (prepared as described below); the ethylidene residue was reduced at an appropriate point in the synthesis using catalytic hydrogenation.

### 2-amino-7-oxo-4,5,6,7-tetrahydrobenzothiazole.

A trace of benzoyl peroxide was added to a mixture of 1,3-cyclohexanedione (2.24 g, 20 mmol), N-bromosuccinimide (3.56 g, 20 mmol) and thiourea (1.52 g, 20 mmol) in benzene (100 mL) and the mixture was heated at reflux for 3 hours. The solution was cooled, concentrated under reduced pressure and redissolved in saturated NaHCO₃ solution (100 mL). The aqueous solution was extracted with ethyl acetate (3 x 100 mL) and the combined organic extracts were concentrated under reduced pressure. The residue was purified by flash column chromatography using methanol / dichloromethane (1:9) as eluent to afford the ketone as a yellow powder (747 mg, 22 %).
¹H NMR (d₆ DMSO): 8.25 (2 H, s, NH₂); 2.88 - 2.80 (2 H, m, COCH₂); 2.58 - 2.49 (2 H, m, NCCH₂); 2.20 - 2.08 (2 H, m, CH₂CH₂CH₂).

### 2-amino-7-ethylidenyl-4,5,6,7-tetrahydrobenzothiazole

A solution of ethylmagnesium bromide (3 M in THF, 0.8 mL, 2.4 mmol) was added dropwise to a solution of the ketone (83 mg, 0.54 mmol) in THF (10 mL) at room temperature under argon and the solution was stirred for 1 hour. Aqueous NH₄Cl solution (1 mL) was added and the suspension was concentrated under reduced pressure. The residue was purified by flash column chromatography using methanol / dichloromethane (1:9) as eluent to afford the amine as a yellow solid (56 mg, 63 %).
¹H NMR (CDCl₃): 5.19 (1 H, q, *J* = 6.9 Hz, CHCH₃); 4.97 (2 H, br s, NH₂); 2.61 - 2.52 (2 H, m, CH=CCH₂); 2. 39 - 2.30 (2 H, m, NCCH₂); 1.84 - 1.73 (2 H, m, CH₂CH₂CH₂); 1.65 (3 H, d, *J* = 6.9 Hz, CHCH₃).

### 3-(Aminomethyl)benzoyl-D/L-phenylglycine 7-ethyl-4,5,6,7-tetrahydrobenzothiazol-2-amide trifluoroacetate salt.

¹H NMR (d₄ methanol): 7.92 - 7.84 (2 H, m, Ar); 7.60 - 7.28 (7 H, m, Ar); 5.77 (1 H, s, CH); 4.11 (2 H, s, CH₂NH₂); 2.74 - 2.60 (1 H, m, chex); 2.58 - 2.40 (2 H, m, chex); 2.03 - 1.86 (2 H, m, chex); 1.76 - 1.57 (2 H, m, chex); 1.53 - 1.36 (2 H, m, CH₂CH₃) ; 0.92 (3 H, t, *J =* 7 Hz, CH₂CH₃).
HPLC (Luna 2, Gradient 1): rt = 4.25 minutes.
LCMS (Luna 2, Gradient 4): rt = 2.22 minutes, 448 (MH)⁴.

### Example 71

### 3-(Aminomethyl)benzoyl-D/L-2-chlorophenylglycine 3-ethoxycarbonyl-4,5,6,7-tetrahydrobenzo[b]thiophene-2-amide trifluoroacetate salt

Prepared in the same manner as example 6 but using the protected amino acid *N*-^{t}butyloxycarbonyl-D/L-2-chlorophenylglycine (which was prepared as described below) instead of the protected phenylglycine.

### N-^{t}Butyloxycarbonyl-D/L-2-chlorophenylglycine

2,4-Dimethoxybenzylamine (3.00 mL, 3.34 g, 20 mmol), and 2-chlorobenzaldehyde (2.25 mL, 2.81 g, 20 mmol) were dissolved in DCM (20 mL). The reaction was stirred at ambient temperature for 2 hours. The reaction was then diluted with DCM (20 mL) and the organic extracts were dried over MgSO₄. The solution was concentrated under reduced pressure and the resulting mixture dissolved in DCM (20 mL). Acetic acid (1.15 mL, 1.20 g, 20 mmol) and ^{t}butylisonitrile (2.26 mL, 1.66 g, 20 mmol) were added and the reaction was stirred at ambient temperature for 48 hours. Trifluoroacetic acid (30 mL) and tetraethylsilane (5 mL) were added and the reaction was stirred at ambient temperature for a further 24 hours. The solution was concentrated under reduced pressure, and the crude reaction mixture was dissolved in hydrochloric acid (50 mL, 6N) and heated at reflux for 24 hours. After cooling, the reaction mixture was washed with ethyl acetate (3 x 25 mL). The aqueous layer was concentrated under reduced pressure to afford the crude amino acid. The amino acid was dissolved in NaHCO₃ (sat. aq., 50 mL) and washed with ethyl acetate (2 x 50 mL). A solution of di-^{t}butyl dicarbonate (8.73 g, 40 mmol) in dioxane (10 mL) was added and the resulting solution was stirred at ambient temperature for 12 hours. The reaction mixture was diluted to 100 mL with diethyl ether and the layers separated. The aqueous layer was acidified to pH 1 with HCl (6 N) and extracted with ethyl acetate (3 x 75 mL). Evaporation of the organic layer afforded the protected amino acid as an off-white solid, which was used without further purification.

### 3-(Aminomethyl)benzoyl-D/L-2-chlorophenylglycine 3-ethoxycarbonyl-4,5,6,7-tetrahydrobenzo[b]thiophene-2-amide trifluoroacetate salt

¹H NMR (d₆ DMSO): 9.80 (1H, d, J 7 Hz, NH), 8.45 (2H, br s, NH₂), 8.20 (2H, d, J 7 Hz, Ar), 7.90 (1H, d, J 6 Hz, Ar), 7.70 (4H, m, Ar, NH), 7.50 (2H, m, Ar), 6.45 (1H, d, J 6 Hz, NHCH), 4.25 (4H, m, CH₂CH3, CH₂NH₂), 2.90 (2H, br s, benzo[b]thiophenyl CH₂), 2.80 (2H, br s, benzo[b]thiophenyl CH₂), 1.90 (4H, br s, benzo[b]thiophenyl, 2x CH₂ ), 1.35 (3H, t, J 8 Hz, CH₂CH₃).
Hplc (Luna 2, Gradient 1): rt = 5.18 minutes
LC/MS (Luna 2, Gradient 4): rt = 2.64 minutes, 526 (MH)⁺

### Example 72

### 3-(Aminomethyl)benzoyl-D/L-2-chlorophenylglycine 3-cyano-4,5,6,7-tetrahydrobenzo(b)thiophene-2-amide trifluoroacetate salt

Prepared in a similar manner to example 38 but using the protected amino acid *N*-^{t}butyloxycarbonyl-D/L-2-chlorophenylglycine (described in example 71) instead of the protected phenylglycine.
¹H NMR (d₆ DMSO): 9.05 (1H, d, J 7 Hz, NH), 8.15 (2H, br s, NH₂), 7.90 (1H, s, Ar), 7.80 (1H, d, J 7 Hz, Ar), 7.50 (1H, m, Ar), 7.40 (2H, m, Ar), 7.30 (2H, m, Ar), 7.05 (1H, m, Ar), 6.15 (1H, d, J 6 Hz, CHNH), 4.00 (2H, d, J 6 Hz, CH₂NH₂), 2.50 (2H, br s, benzo[b]thiophenyl CH₂), 2.40 (2H, br s, benzo[b]thiophenyl CH₂), 1.60 (4H, br s, benzo[b]thiophenyl, 2x CH₂).
Hplc (Luna 2, Gradient 1): rt = 4.48 minutes
LC/MS (Luna 2, Gradient 4): rt = 2.33 minutes, 479 (MH)⁺

### Example 73

### 3-(Aminomethyl)benzoyl-D/L-2-chlorophenylglycine 4,5,6,7-tetrahydrobenzothiazol-2-amide hydrochloride salt

### 3-(BOC-Aminomethyl)benzoyl-D/L-N-(2,4-dimethoxybenzyl)-2-chlorophenylglycine 2-(methoxycarbonyloxy)-1,1-dimethylethylamide.

A mixture of 2-chlorobenzaldehyde (1.12 g, 8 mmol) and 2,4-dimethoxybenzylamine (1.2 mL, 8 mmol) in dichloromethane (5 mL) was stirred at room temperature for 1 hour. The solution was dried using sodium sulphate, filtered and evaporated *in vacuo.* The resulting oil was dissolved in methanol (30 mL), and 3-(N-Boc-aminomethyl)benzoic acid (2.0 g, 8 mmol) and (2-isocyano-2-methyl)propyl methyl carbonate (Tetrahedron, 55 (1999) 7411-7420) (1.26 g, 8 mmol) was added. The reaction mixture was then stirred and heated at 60°C for 3 days. The reaction mixture was absorbed onto silica and purified by flash chromatography, eluting with ethyl acetate / hexane. The desired fractions were collected and evaporated *in vacuo* to give a foam (3.3 g).
¹H NMR (CDCl₃) : 7.62 (1 H, bs, NH); 7.44 (2 H, m, Ar); 7.30 (3 H, m, Ar); 7.15 (3 H, m, Ar); 6.63 (1 H, br s, NHCMe₂); 6.35 (1 H, d, *J* = 10 Hz, Ar); 6.21 (1 H, s, Ar); 5.45 (1 H, s, CH2ClPh); 5.03 (1 H, m, NHBoc); 4.65 (1 H, d, *J =* 13 Hz, CH₂DMP); 4.53 (1 H, d, *J =* 13 Hz, CH₂DMP); 4. 3 - 4.15 (4 H, m, NHCH₂ & CH₂O); 3.76 (3 H, s, OMe); 3. 74 (3 H, s, OMe); 3. 62 (3 H, s, OMe); 1.43 (9 H, s, Boc); 1.25 (6 H, s, CH₂Me₂)

### 3-(BOC-Aminomethyl)benzoyl-D/L-N-(2,4-dimethoxybenxyl)-2-chlorophenylglycine

Potassium *tert*-butoxide (9.5 mL, 1.0 M in THF, 9.4 mmol) was added to a stirred solution of the methyl carbonate (3.3 g, 4.7mmol) in dry THF (40 mL). The reaction mixture was then allowed to stir at room temperature for 2 hours. The mixture was then acidified with HCl (*conc.* aq.). The resulting solid was filtered off and the yellow solution evaporated *in vacuo* to give an orange oil. This was purified by flash chromatograhy, eluting with ethyl acetate / hexane. The desired fractions were collected and evaporated to give the methyl ester as a white foam (1.8 g, 3.1 mmol).

A solution of the ester (1.8 g, 3.1 mmol) in THF (15 mL) was stirred and ethanol (15 mL) and water (5 mL) were added, followed by LiOH.H₂O (26 mg, 6.2 mmol). The solution was heated at 60°C for 16 hours. The reaction mixture was concentrated to remove the THF and ethanol. The residue was partitioned between water (70 mL) and ethyl acetate (40 mL). The aqueous solution was acidified with HCl (1 M), then extracted with ethyl acetate (100 mL). The dried extract (MgSO₄) was filtered and evaporated to give the acid as a white foam (1.29 g).
¹H NMR (CDCl₃) : 7.58 (1 H, m, Ar); 7.44 (2 H, m, Ar); 7.3 (3 H, m, Ar); 7.15 (3 H, m, Ar); 6.35 (1 H, d, *J =* 10 Hz, Ar); 6.21 (1 H, s, Ar); 5.66 (1 H, s, CH2ClPh); 5.18 (1 H, m, NHBoc); 4.59 (1 H, d, *J* = 13 Hz, CH₂DMP); 4.45 (1 H, d, *J* = 13 Hz, CH₂DMP); 4.28 (2 H, d, *J =* 5 Hz, NHCH₂); 3.72 (3H, s, OMe); 3.58 (3H, s, OMe); 1.44 (9H, s, Boc).

### 3-(Aminomethyl)benzoyl-D/L-2-chlorophenylglycine 4,5,6,7-tetrahydrobenzothiazol-2-amide hydrochloride salt

A solution of the above acid (250 mg, 0.44 mmol), 1-[3-(dimethylamino)propyl]-3-ethylcarbodiimide hydrochloride (101 mg, 0.53 mmol) and 1-hydroxy-7-azabenzotriazole (72 mg, 0.53 mmol) in dimethylformamide (20 mL) was stirred at room temperature and 4,5,6,7-tetrahydrobenzo[b]-thiazol-2-amine (synthesised as described in example 23) was added and the mixture allowed to stir overnight. The dimethyl formamide was removed under reduced pressure and the residue partitioned between water (20 mL) and ethyl acetate (30 mL). The ethyl acetate layer was then washed with HCl (1 M, 20 mL) and NaHCO₃ (sat. aq., 20 mL). The dried (MgSO₄) ethyl acetate layer was absorbed onto silica and purified by flash chromatography eluting with ethyl acetate / hexane. The desired fractions were collected and evaporated to give the amide as a yellow oil (127 mg).

The oil (127 mg, 0.18 mmol) was dissolved in dichloromethane and triethysilane (0.06 mL) was added, followed by trifluoroacetic acid (5 mL). After one hour the solvent was removed *in vacuo* and the residue purified on an SCX ionexchange column, eluting with 5% 2 N NH_{3/}MeOH in dichloromethne. The resulting oil was treated with ethereal HCl and isolated as the HCl salt.
¹H NMR (MeOH) : 7.88 (2 H, m, Ar); 7.58 (1 H, d, *J* = 9 Hz, Ar); 7.45 (2 H, m, Ar); 7.32 (3 H, m, Ar); 6.22 (1 H, s, NHCHAr); 4.1 (2 H, s, CH₂NH₂); 2.62 (2 H, br s, tetrahydrobenzothiazole C(7)H₂); 2.52 (2 H, br s, tetrahydrobenzothiazole C(4)H₂); 1.77 (4 H, br s, tetrahydrobenzothiazole C(5)H₂ and C(6)H₂).
Hplc (Luna 2, Gradient 1): rt = 3.95 minutes.
LC/MS (Luna 2, Gradient 4): rt = 2.22 minutes, 455 (MH)⁺.

### Example 74

### 3-(Aminomethyl)benzoyl-D/L-2-ethylthiazol-4-ylglycine 3-ethoxycarbonyl-4,5,6,7-tetrahydrobenzo[b]thiophene-2-amide trifluoroacetate salt

Prepared in a similar manner to example 6 but using the protected amino acid *N*-^{t} butyloxycarbonyl-D/L-2-ethylthiazol-4-ylglycine (prepared as described below) instead of the protected phenylglycine.

### N-t-butyloxycarbonyl-D/L-2-ethylthiazol-4-ylglycine

A solution of ethyl γ-chloro-α-oximinoacetoacetate (2.00 g, 10.3 mmol) and thiopropionamide (0.92g, 10.3 mmol) in dry benzene (15 mL) was heated at reflux. After 4 hours, the reaction mixture was poured onto NaHCO₃ (sat. aq., 50 mL). The resulting mixture was extracted with ethyl acetate (2 x 50 mL), and the combined extracts dried over MgSO₄ and evaporated under reduced pressure. Flash chromatography (ethyl acetate:hexane 1:4) then afforded impure ethyl α-oximino-2-ethylthiazole-4-acetate (0.83g). The crude oxime was then dissolved in methanol (25 mL) and formic acid (50% aq., 10 mL) was added. The mixture was cooled to 0°C and zinc dust (1.00 g, 15.3 mmol) was added portionwise over 30 minutes. The reaction mixture was allowed to warm to room temperature, and stirred for 6 hours. The solution was then filtered, basified to pH 9 with solid NaHCO₃, and extracted with ethyl acetate (3 x 80 mL). The combined extracts were then dried and evaporated to afford D/L-2-ethylthiazol-4-ylglycine ethyl ester (0.56 g, 2.6 mmol).
The ester (560 mg, 2.6 mmol) was dissolved in tetrahydrofuran (50 mL). Triethylamine (0.4 mL, 3.9 mmol) was added, followed by di-t-butyl dicarbonate (0.57 g, 2.6 mmol). After stirring at room temperature overnight the mixture was concentrated, water (20 mL) was added and the solution extracted with ethyl acetate (2 x 20 mL). The combined extracts were evaporated to afford N-t-butyloxycarbonyl-D/L-2-ethylthiazol-4-ylglycine ethyl ester (824 mg) as a golden oil. The oil was dissolved in methanol (25 mL) and sodium hydroxide (2 M aq., 5 mL) was added. After stirring at room temperature for 2 hours, the solution was concentrated, water (30 mL) was added, and the solution extracted with ethyl acetate (30 mL). The aqueous layer was then acidified to pH 4 with 2N HCl, and extracted with ethyl acetate (2 x 20 mL). The latter extracts were combined and evaporated to afford *N*-t-butyloxycarbonyl-D/L-2-ethylthiazol-4-ylglycine (450 mg) as a white solid.
¹H NMR (CDCl₃): 10.1 (1 H, br s, CO₂H), 7.20 (1 H, s, thiazole CH), 5.85 (1 H, br d, *J = 6 Hz*, NHBoc), 5.52 (1 H, br d, *J =* 6 Hz, α-CH), 3.05 (2 H, q, *J* = 5 Hz, CH₂CH₃), 1.49 (9 H, s, C (CH₃)₃), 1.42 (3 H, t, *J* = 5 Hz, CH₂CH₃).

### 3-(Aminomethyl)benzoyl-D/L-2-ethylthiazol-4-ylglycine 3-ethoxycarbonyl-4,5,6,7-tetrahydrobenzo[b]thiophene-2-amide trifluoroacetate salt

¹H NMR (d₃ acetonitrile): 8.05 (1H, s, Ar), 7.90 (1H, d, J 7 Hz, Ar), 7.60 (1H, d, J 7 Hz, Ar), 7.45 (1H, m, Ar), 7.35 (1H, s, Ar), 6.00 (1H, m, CHNH), 4.20 (2H, q, CH₂CH₃), 4.10 (2H, s, CH₂NH₂), 2.95 (2H, q, CH₂CH₃), 2.70 (2H, s, benzo[b]thiophenyl CH₂), 2.50 (2H, s, benzo[b]thiophenyl CH₂), 1.80 (4H, s, benzo[b]thiophenyl, 2x CH₂), 1.30 (3H, t, CH₃), 1.15 (3H, t, CH₃)
Hplc (Luna 2, Gradient 1): rt = 5.17 minutes
LC/MS (Luna 2 Gradient 4): rt = 2.69 minutes, 527 (MH)⁺

### Example 75

### 3-(Aminomethyl)benzoyl-D/L-2-ethylthiazol-4-ylglycine 3-cyano-4,5,6,7-tetrahydrobenzo[b]thiophene-2-amide trifluoroacetate salt

Prepared in a similar manner to that described for Example 38 but using the protected amino acid *N*-^{t}butyloxycarbonyl-D/L-2-ethylthiazol-4-ylglycine (described in example 74) instead of the protected phenylglycine.
¹H NMR (CDCl₃) : 8.30 (3H, m, Ar), 7.85 (1H, s, Ar) 7.65 (1H, d, J 6 Hz, Ar), 6.15 (1H, d, J 6 Hz, NHCH) , 3.90 (2H, s, NH₂CH₂), 2.95 (2H, q, CH₂CH₃), 2.35 (4H, br s, benzo[b]thiophenyl, 2x CH₂ ), 1.65 (4H, br s, benzo[b]thiophenyl, 2x CH₂), 1.25 (3H, t, CH₃CH₂)
Hplc (Luna 2, Gradient 1): rt = 4.46 minutes
LC/MS (Luna 2, Gradient 4): rt = 2.28 minutes, 480 (MH)⁺

### Example 76

### 3-(Aminomethyl)benzoyl-D/L-2-methylthiazol-4-ylglycine benzthiazol-2-amide

Prepared as described for example 16 but using the protected amino acid *N*-^{t}butyloxycarbonyl-D/L-2-methythiazol-4-ylglycine (which was prepared in the same way as that described for *N-*^{t}butyloxycarbonyl-D/L-2-ethylthiazol-4-ylglycine in example 74 but using thioacetamide instead of thiopropionamide) instead of the protected phenylglycine.
¹H NMR (d⁴ MeOH): 7.84 (2H, m, Ar) ; 7.7 (1 H, d, *J* = 9 Hz, Ar) ; 7.58 (1 H, d, *J* = 9 Hz, Ar); 7.52 - 7.38 (2 H, m, Ar); 7.35 (1 H, s, Ar) ; 7.26 (1 H, t, *J =* 6 Hz, Ar) ; 7. 14 (1 H, t, *J =* 6 Hz, Ar) ; 5.95 (1 H, s, CH-thiazole); 4.03 (2 H, s, CH₂NH₂); 2.57 (3 H, s, CH₃).
Hplc (Luna 2, Gradient 1): rt = 3.56 minutes.
LC/MS (Luna 2, Gradient 4): rt = 1.68 minutes, 438 (M⁺).

Examples 77-79 were synthesised according to the method of Example 38 using the indicated amine.

### Example 77

### 3-(Aminomethyl)benzoyl-D/L-phenylglycine 3-pyrid-4-yl-tetrahydrobenzo(b)thiophen-2-amide

Prepared from 2-amino-3-pyrid-4-yl-tetrahydrobenzo(b)thiophene (synthesised using general procedure A).
¹H NMR (CD₃CN) : 8.96 (1 H, br s, NH), 8.25 (2 H, d, J 8 Hz, pyridyl C¹H & C⁶H), 7.95-7.28 (12 H, NH & 11 x Ar), 5.52 (1 H, d, J 6 Hz, α-CH), 4.19-4.09 (2 H, m, ArCH₂NH₂), 2.75-2.65, 2.40-2.28, 1.84-1.73 & 1.72-1.59 (4 x 2 H, 4 x m, CH₂CH₂CH₂CH₂).
Hplc (Luna 2, Gradient 1): rt = 3.42 minutes.
LC/MS (Luna 2, Gradient 9): rt = 1.93 minutes, 497 (MH⁺).

### Example 78

### 3-(Aminomethyl)benzoyl-D/L-phenylglycine 3-tert-butylsulfonyl-tetrahydrobenzo(b)thiophen-2-amide

Prepared from 2-amino-3-*tert*-butylsulfonyl-tetrahydrobenzo(b)thiophene (synthesised using general procedure A).
¹H NMR (CD₃CN) : (peaks broadened by rotomers) 10.75 (1 H, s, H-bonded NH), 8.25-7.24 (11 H, m, Ar & 2 x NH), 5.74-5.66 (1 H, m, α-CH), 4.16-4.04 (2 H, m, ArCH₂NH₂), 2.56-2.38 (4H, m, CH₂CH₂CH₂CH₂), 1.91 (9 H, s, C(CH₃)₃), 1.80-1.66 (4 H, m, CH₂CH₂CH₂CH₂).
Hplc (Luna 2, Gradient 1): rt = 4.63 minutes.
LC/MS (Luna 2, Gradient 4): rt = 2.98 minutes, 540 (MH⁺).

### Example 79

### 3-(Aminomethyl)benzoyl-D/L-phenylglycine 3-phenylsulfonyl-tetrahydrobenzo(b)thiophen-2-amide

Prepared from 2-amino-3-phenylsulfonyl-tetrahydrobenzo(b)thiophene (synthesised using general procedure A).
¹H NMR (D₆ DMSO): 9.49 (1 H, d, J 8 Hz, NH), 8.18 (3 H, br s, NH₃⁺), 8.10-7.32 (16 H, m, Ar & 2 x NH), 6.08 (1 H, d, J 7 Hz, α-CH), 4.12-4.00 (2 H, m, ArCH₂NH₂), 1.70-1.64 (4 H, m, CH₂CH₂CH₂CH₂). CH₂CH₂CH₂CH₂ peaks obscured by DMSO peak around 2.5 ppm.
Hplc (Luna 2, Gradient 1): rt = 4.80 minutes.
LC/MS (Luna 2, Gradient 4): rt = 3.06 minutes, 560 (MH⁺).

### Example 80

### 3-(Aminomethyl)benzoyl-D/L-phenylglycine-tetrahydrobenzo(b)thiophen-2-amide trifluoroacetate salt

Prepared from 2-amino-4,5,6,7-tetrahydrothianaphthene synthesised as described below and the method of Example 1

### 2-Amino-4,5,6,7-tetrahydrothianaphthene

A solution of 4-keto-4,5,6,7-tetrahydrothianaphthene (910 mg, 5.98 mmol), hydrazine hydrate (619 mg, 12.4 mmol) and KOH (730 mg, 13.0 mmol) in ethylene glycol (4 mL) was heated to 170°C. After 6 hrs, the solution was cooled and EtOAc (100 mL) was added. The mixture was extracted with HCl (1N, aq., 2 x 50 mL) and concentrated in vacuo to afford crude 4,5,6,7-tetrahydrothianaphthene as a brown oil (670 mg). A portion of the oil (423 mg) was dissolved in acetic anhydride (1 mL), cooled to 0°C and fuming nitric acid (250 mg, 3.75 mmol) was added dropwise as a solution in acetic acid (2 mL). The reaction mixture was allowed to warm to room temperature, stirred for 2 hrs, and then poured onto ice (50 g). After allowing the ice to melt, EtOAc (50 mL) was added, and the solution extracted with NaHCO₃ (sat., aq., 50 mL) and HCl (1N, aq., 50 mL). The mixture was concentrated in vacuo and purified by flash chromatography (SiO₂, acetone:hexane 1:10), collecting the spot at 0.80, to afford 2-nitro-4,5,6,7-tetrahydrothianaphthene as a yellow oil (48 mg). The oil was then dissolved in MeOH (2 mL), 10% Pd/C (10 mg) added, and the reaction mixture was stirred for 18 hrs under an atmosphere of hydrogen. The mixture was then filtered through celite to afford 2-Amino-4,5,6,7-tetrahydrothianapthene (31 mg) as a yellow oil which was used in the subsequent synthesis without further purification.

### 3-(Aminomethyl)benzoyl-D/L-phenylglycine-tetrahydrobenzo(b)thiophen-2-amide trifluoroacetate salt

¹H NMR (d₃ acetonitrile): 9.58 (1 H, s, NH), 7.92-7.26 (10 H, Ar CH's & NH), 7.12 (3 H, br s, NH₃⁺), 6.24 (1 H, s, thiophene CH), 5.62(1 H, d, J 7 Hz, α-CH), 4.09 (2 H, br s, CH₂N), 2.52 & 2.39 (2 x 2H, 2 x t, 2 x J 6 Hz, CH₂CH₂CH₂CH₂), 1.74-1.56 (4 H, m, CH₂CH₂CH₂CH₂).
Hplc (Luna 2, Gradient 1): rt = 4.29 minutes.
LC/MS (Luna 2, Gradient 4): rt = 3.31 minutes, 420 (MH⁺).

Examples 81 -82 were synthesised according to the method of Example 1 using the indicated amine and the protected amino acid N-t-butyloxycarbonyl-2-trifluoromethyl-D/L-phenylglycine. The amino acid was prepared as described in Example 71 using 2-trifluoromethylbenzaldehyde instead of 2-chlorobenzaldehyde.

### Example 81

### 3-(Aminomethyl)benzoyl-D/L-(2-trifluoromethyl)-phenylglycine benzothiazol-2-amide trifluoroacetate salt

Prepared using 2-aminobenzothiazole
¹H NMR (d₃ acetonitrile): 7.96-7.30 (12 H, Ar), 6.52 (3 H, br s, NH₃⁺), 6.10 (1 H, d, J 6 Hz, α-CH), 4.04 & 3.97 (2 x 1 H, Abq, J 6 Hz, CH₂N).
Hplc (Luna 2, Gradient 1): rt = 4.19 minutes.
LC/MS (Luna 2, Gradient 4): rt = 2.60 minutes, 485 (MH⁺).

### Example 82

### 3-(Aminomethyl)benzoyl-D/L-(2-trifluoromethyl)-phenylglycine tetrahydrobenzothiazol-2-amide trifluoroacetate salt

Prepared using 2-aminotetrahydrobenzothiazole
¹H NMR (d₃ acetonitrile): 8.00-7.28 (12 H, Ar), 6.78 (3 H, br s, NH₃⁺), 6.12 (1 H, d, J 6 Hz, α-CH), 4.11-4.00 (2 H, m, CH₂N), 2.63-2.50 (4 H, m, CH₂CH₂CH₂CH₂), 1.80-1.69 (4 H, m, CH₂CH₂CH₂CH₂).
Hplc (Luna 2, Gradient 1) : rt = 4.16 minutes.
LC/MS (Luna 2, Gradient 4): rt = 2.58 minutes, 489 (MH⁺).

Examples 83-85 were synthesised according to the method of Example 38 using the indicated amine and the protected amino acid N-t-butyloxycarbonyl-2-trifluoromethyl-D/L-phenylglycine. The amino acid was prepared as described in Example 71 using 2-trifluorofnethylbenzaldehyde instead of 2-chlorobenzaldehyde.

### Example 83

### 3-(Aminomethyl)benzoyl-D/L-(2-trifluoromethyl)phenyl-glycine 3-methoxycarbonyl-tetrahydrobenzo(b)thiophen-2-amide trifluoroacetate salt

Prepared using 2-amino-3-methoxycarbonyl-tetrahydrobenzo(b)thiophen-2-amide (prepared according to general procedure A)
¹H NMR (d₃ acetonitrile) : 9.70 (1 H, d, J 8 Hz, NH), 8.22 (3 H, br s, NH₃⁺), 8.09-7.54 (9 H, Ar CH's & NH), 6.22 (1 H, d, J 7 Hz, α-CH), 4.20-4.09 (2 H, m, CH₂N), 3.74 (3 H, s, CO₂CH₃), 2.80-2.59 (4 H, m, CH₂CH₂CH₂CH₂), 1.82-1.69 (4 H, m, CH₂CH₂CH₂CH₂).
Hplc (Luna 2, Gradient 1): rt = 5.03 minutes.
LC/MS (Luna 2, Gradient 4): rt = 3.15 minutes, 546 (MH⁺).

### Example 84

### 3-(Aminomethyl)benzoyl-D/L-(2-trifluoromethyl)phenyl-glycine 3-methanesulfonyl-tetrahydrobenzo(b)thiophen-2-amide trifluoroacetate salt

Prepared using 2-amino-3-methanesulfonyl-tetrahydrobenzo(b)thiophen-2-amide (prepared according to general procedure A)
¹H NMR (d₃ acetonitrile): 8.09-7.67 (10 H, Ar CH's & 2 x NH), 6.99 (3 H, br s, NH₃⁺), 6.27 (1 H, d, J 7 Hz, α-CH), 4.37-4.24 (2 H, m, CH₂N), 3.15 (3 H, s, SO₂CH₃), 2.86-2.64 (4 H, m, CH₂CH₂CH₂CH₂), 1.97-1.84 (4 H, m, CH₂CH₂CH₂CH₂).
Hplc (Luna 2, Gradient 1): rt = 4.51 minutes.
LC/MS (Luna 2, Gradient 4): rt = 3.09 minutes, 566 (MH⁺).

### Example 85

### 3-(Aminomethyl)benzoyl-D/L-(2-trifluoromethyl)phenyl-glycine 3-cyanotetrahydrobenzo(b)thiophen-2-amide trifluoroacetate salt

Prepared using 2-amino-3-cyano tetrahydrobenzo(b)-thiophen-2-amide (prepared according to general procedure A)
¹H NMR (d₃ acetonitrile): 9.61 (1 H, s, NH), 7.91-7.24 (10 H, Ar CH's & NH), 6.80 (3 H, br s, NH₃⁺), 6.04 (1 H, d, J 7 Hz, α-CH), 4.05-3.94 (2 H, m, CH₂N), 2.45-2.24 (4 H, m, CH₂CH₂CH₂CH₂), 1.66-1.53 (4 H, m, CH₂CH₂CH₂CH₂).
Hplc (Luna 2, Gradient 1): rt = 4.64 minutes.
LC/MS (Luna 2, Gradient 4): rt = 3.32 minutes, 513 (MH⁺).

### Example 86

### 3-(Aminomethyl)benzoyl-D/L-2-methylthiazol-4-ylglycine 3-methanesulfonyl-tetrahydrobenzo(b)thiophen-2-amide

Prepared using the method of Example 38 using 2-amino-3-methanesulfonyl-tetrahydrobenzo(b)thiophene (prepared according to general procedure A) and the protected amino acid N-t-butyloxycarbonyl-2-methyl-thiazol-4-ylglycine. The amino acid was prepared using the procedure described in Example 76.
¹H NMR (CD₃CN): 8.23-7.30 (9 H, m, 5 x Ar, NH, NH₃⁺), 6.00 (1 H, d, J 6 Hz, α-CH), 4.16 (2 H, br s, ArCH₂NH₂), 3.02 (3 H, s, SO₂CH₃), 2.70-2.57 (7H, m, thiazolyl CH₃ & CH₂CH₂CH₂CH₂), 1.85-1.74 (4 H, m, CH₂CH₂CH₂CH₂).
Hplc (Luna 2, Gradient 1) : rt = 3.89 minutes.
LC/MS (Luna 2, Gradient 4): rt = 2.71 minutes, 519 (MH⁺).

### Example 87

### 3-(Aininomethyl)benzoyl-D/L-2-ethylthiazol-4-ylglycine 3-methanesulfonyl-tetrahydrobenzo(b)thiophen-2-amide

Prepared using the method of Example 38 using 2-amino-3-methanesulfonyl-tetrahydrobenzo(b)thiophene (prepared according to general procedure A) and the protected amino acid N-t-butyloxycarbonyl-2-ethyl-thiazol-4-ylglycine. The amino acid was prepared using the procedure described in Example 74.
¹H NMR (CD₃CN): 8.45 (1 H, br s, NH), 8.12-7.95 & 7.70-7.54 (2 H & 3 H, 2 x m, Ar CH's), 6.18-6.11 (1 H, m, α-CH), 4.28-4.19 (2 H, m, ArCH₂NH₂), 3.15 (2 H, q, CH₂CH₃), 3.10 (3 H, s, SO2Me), 2.78-2.66 (4H, m, CH₂CH₂CH₂CH₂), 1.90-1.79 (4 H, m, CH₂CH₂CH₂CH₂) 1.40 (3 H, t, J 7 Hz, CH₂CH₃).
Hplc (Luna 2, Gradient 1): rt = 4.24 minutes.
LC/MS (Luna 2, Gradient 4): rt = 2.86 minutes, 533 (MH⁺).

### Example 88

### 3-(Aminomethyl)benzoyl-(D/L)-2-chloro-3-pyridylglycine 4,5,6,7-tetrahydrobenzo(b)thiazol-2-amide trifluoroacetate

Prepared according to the procedure described for Example 73, substituting 2-chloronicotinaldehyde for 2-chlorobenzaldehyde.
¹H NMR (d₆ DMSO) 12.30 (1H, s, CONH-thiaz), 9.15 (1H, d, J=7Hz, ArCONH), 8.25 (1H, dd, J=5 & 2Hz, PyH), 8.00 (3H, br.s, NH₃⁺), 7.94 (1H, s, ArH), 7.78 (1H, d, J=7Hz, ArH), 7.52 (1H, d, J=8Hz, ArH), 7.46 (1H, d, J=8Hz, ArH), 7.38 (1H, d, J=8.5Hz, ArH), 7.32 (1H, m, PyH), 5.92 (1H, d, J=7Hz, α-H), 3.92 (2H, m, ArCH₂NH₃), 2.55 (1H, m, ArCH₂CH₂), 2.46 (2H, m, ArCH₂CH₂) 2.13 (1H, m, ArCH₂CH₂) and 1.59 (4H, m, 2 x ArCH₂CH₂).
HPLC (performed on a Shimadzu LC10AD gradient system, Luna 2, eluting with 20% B to 100% B over 16 minutes): rt = 5.30 minutes.
LC/MS (Luna 2, Gradient 4): rt = 1.98 minutes, 456/457 (MH⁺).

### Example 89

### 3-(Aminomethyl)benzoyl-(D/L)-2-chloro-3-pyridylglycine 3-cyano-4,5,6,7-tetrahydrobenzo(b)thiophen-2-amide trifluoroacetate

Prepared according to the procedure described for Example 73, substituting 2-chloronicotinaldehyde for 2-chlorobenzaldehyde and using 2-amino-3-cyano-4,5,6,7-tetrahydrobenzo(b)thiophene, prepared as described in Example 38 (general procedure A) in place of 2-amino-4,5,6,7-tetrahydrobenzo(b)thiazole.
¹H NMR (d₆ DMSO) 12.22 (1H, s, CONH-thiophene), 9.36 (1H, d, J=7Hz, ArCONH), 8.45 (1H, dd, J=5 & 2Hz, PyH), 8.16 (3H, br.s, NH₃⁺), 8.01 (1H, s, ArH), 7.95 (1H, d, J=8Hz, ArH), 7.68-7.60 (2H, m, ArH), 7.56 (1H, d, J=7.5Hz, ArH), 7.53-7.46 (1H, m, PyH), 6.24 (1H, d, J=7Hz, α-H), 4.09 (2H, br.m, ArCH₂NH₃), 2.73 (1H, m, ArCH₂CH₂), 2.60 (2H, m, ArCH₂CH₂) 2.27 (1H, m, ArCH₂CH₂) and 1.76 (4H, m, 2 x ArCH₂CH₂).
HPLC (performed on a Shimadzu LC10AD gradient system, Luna 2, eluting with 20% B to 100% B over 16 minutes): rt = 6.44 minutes.
LC/MS (Luna 2, Gradient 4): rt = 2.26 minutes, 480/482 (MH⁺).

### Example 90

### 3-(Aminomethyl)benzoyl-(D/L)-2-chloro-3-pyridylglycine 3-ethoxycarbonyl-4,5,6,7-tetrahydrobenzo[b]thiophen-2-amide trifluoroacetate

Prepared according to the procedure described for Example 73, substituting 2-chloronicotinaldehyde for 2-chlorobenzaldehyde and using ethyl 2-amino-4,5,6,7-tetrahydrobenzo(b)thiophene-3-carboxylate, prepared as described in Example 38 (general procedure A) in place of 2-amino-4,5,6,7-tetrahydrobenzo(b)thiazole.
¹H NMR (d₆ DMSO) 11.63 (1H, s, CONH-thiophene), 9.63 (1H, d, J=8Hz, ArCONH), 8.42 (1H, dd, J=5 & 2Hz, PyH), 8.13 (3H, br.s, NH₃⁺), 7.98 (1H, s, ArH), 7.89 (1H, dd, J=2&7Hz, ArH), 7.64 (1H, d, J=8Hz, ArH), 7.56 (1H, d, J=8Hz, ArH), 7.53-7.44 (1H, m, PyH), 6.20 (1H, d, J=8Hz, α-H), 4.13 (2H, q, J=7.5Hz, CO₂CH₂CH₃) 4.08 (2H, br.m, ArCH₂NH₃), 2.70 (1H, m, ArCH₂CH₂), 2.59 (2H, m, ArCH₂CH₂) 2.24 (1H, m, ArCH₂CH₂), 1.69 (4H, m, 2 x ArCH₂CH₂) and 1.16 (3H, t, J=7.5Hz, CO₂CH₂CH₃).
HPLC (performed on a Shimadzu LC10AD gradient system, Luna 2, eluting with 20% B to 100% B over 16 minutes): rt = 8.16 minutes.
LC/MS (Luna 2, Gradient 4): rt = 2.58 minutes, 527/529 (MH⁺).

### Example 91

### 3-(Aminomethyl)benzoyl-(D/L)-2-chloro-3-pyridylglycine 3-aminocarbonyl-4,5,6,7-tetrahydrobenzo(b)thiophen-2-amide trifluoroacetate

Prepared according to the procedure described for Example 73, substituting 2-chloronicotinaldehyde for 2-chlorobenzaldehyde and using 2-amino-4,5,6,7-tetrahydrobenzo[b]thiophene-3-carboxamide, prepared as described in Example 38 (general procedure A) in place of 2-amino-4,5,6,7-tetrahydrobenzo(b)thiazole.
¹H NMR (CD₃CN) 12.66 (1H, s, CONH-thiophene), 8.61 (1H, d, J=7Hz, ArCONH), 8.42 (1H, dd, J=5 & 2Hz, PyH), 8.35 (1H, s, ArH), 8.05 (1H, dd, J=2&8Hz, ArH), 7.91 (1H, d, J=8Hz, ArH), 7.90 (3H, br.s, NH₃⁺), 7.66 (1H, d, J=8Hz, ArH), 7.59 (1H, m, ArH), 7.43 (1H, dd, J=5&8Hz, PyH), 6.50 (2H, br.s, CONH₂) 6.33 (1H, d, J=8Hz, α-H), 4.31 (2H, br.s, ArCH₂NH₃), 2.75 (6H, m, ArCH₂CH₂), and 2.25 (2H, m, ArCH₂CH₂).
HPLC (performed on a Shimadzu LC10AD gradient system, Luna 2, eluting with 20% B to 100% B over 16 minutes): rt = 6.47 minutes.
LC/MS (Luna 2, Gradient 4): rt = 2.10 minutes, 498/500 (MH⁺).

### Example 92

### 3-(Aminomethyl)benzoyl-(D/L)-2-chloro-3-pyridylglycine 3-methylsulphonyl-4,5,6,7-tetrahydrobenzo(b)thiophen-2-amide trifluoroacetate

Prepared according to the procedure described for Example 73, substituting 2-chloronicotinaldehyde for 2-chlorobenzaldehyde and using 2-amino-3-methylsulphonyl-4,5,6,7-tetrahydrobenzo(b)thiophene, prepared as described in example 38 (general procedure A) in place of 2-amino-4,5,6,7-tetrahydrobenzo(b)thiazole.
¹H NMR (CD₃CN) 11.08 (1H partially exchanged, s, CONH-thiophene), 8.57 (1H partially exchanged, m, ArCONH), 8.33 (1H, dd, J=5 & 2Hz, PyH), 8.13 (1H, s, ArH), 7.95 (1H, dd, J=2&8Hz, ArH), 7.87 (1H, d, J=8Hz, ArH), 7.57 (1H, d, J=8Hz, ArH), 7.50 (3H, br.s, NH₃⁺), 7.49 (1H, m, ArH), 7.34 (1H, dd, J=2&5Hz, PyH), 6.22 (1H, m, α-H), 4.14 (2H, br.s, ArCH₂NH₃), 2.99 (3H, s, SO₂Me), 2.63 (4H, m, ArCH₂CH₂) and 1.75 (2H, m, ArCH₂CH₂).
HPLC (performed on a Shimadzu LC10AD gradient system, Luna 2, eluting with 20% B to 100% B over 16 minutes): rt = 6.82 minutes.
LC/MS (Luna 2, Gradient 4): rt = 2.20 minutes, 533/535 (MH⁺).

### Example 93

### 3-(Aminomethyl)benzoyl-D/L-phenylglycine 7-oxo-tetrahydrobenzothiazol-2-amide trifluoroacetate

Prepared according to the method of Example 16 using 2-amino-7-oxotetrahydrobenzothiazole, synthesised as described below.

### 2-amino-7-oxotetrahydrobenzothiazole.

A trace of benzoylperoxide was added to a mixture of 1,3-cyclohexanedione (2.24 g, 20 mmol), N-Bromosuccinimide (3.56 g, 20 mmol) and thiourea (1.52 g, 20 mmol) in benzene (100 ml) and the mixture was heated to reflux for 3 hours. The solution was cooled, concentrated under reduced pressure and redissolved in saturated NaHCO₃ solution (100 ml). The aqueous solution was extracted with ethylacetate (3 x 100 ml) and the combined organic extracts were concentrated under reduced pressure. The ketone was purified by flash column chromatography using methanol 1/9 dichloromethane as eluent to afford a yellow powder (747 mg, 22 %).
¹H NMR (d₆ DMSO): 8.25 (2 H, s, NH₂); 2.88 - 2.80 (2 H, m, COCH₂); 2.58 - 2.49 (2 H, m, NCCH₂); 2.20 - 2.08 (2 H, m, CH₂CH₂CH₂).

### 3-(Aminomethyl)benzoyl-D/L-phenylglycine 7-oxo-tetrahydrobenzothiazol-2-amide trifluoroacetate

¹H NMR (d₄ methanol): 7.90 - 7.74 (2 H, m, Ar); 7.67 - 7.14 (7 H, m, Ar); 5.79 (1 H, s, CH); 4.10 (2 H, s, CH₂NH₂); 2.89 - 2.70 (2 H, m, SCOCH₂); 2.61 - 2.40 (2 H, m, NCH₂CH₂); 2.12 - 1.97 (2 H, m, CH₂CH₂CH₂).
HPLC (Luna 2, Gradient 1): rt = 3.36 minutes.
LCMS (Luna 2, Gradient 4): rt = 2.50 minutes, 435 (M + H)⁺.

### Example 94

### 3-(Aminomethyl)benzoyl-D/h-phenylglycine 5-(hydroxymethyl)benzothiazol-2-amide

Prepared according to the procedure of Example 1 using 2-amino-5-ethoxycarbonyl-benzothiazole, synthesised as described below. The 5-ethoxy carbony group was reduced to the 5-hydroxymethyl group using Diisobutylaluminium hydride in dry THF prior to the final deprotection step.

### 2-amino-5-ethoxycarbonylbenzothiazole

A solution of ethyl-2-aminobenzoate (4.13 ml, 27.7 mmol) and sodium thiocyanate (2.25 g, 27.7 mmol) was stirred in methanol (50 ml) at -5 C. Bromine (0.712 ml, 13.9 mmol) was added and the solution was stirred at -5 C for 2 hours. The reaction was partitioned between ethyl acetate (250 ml) and water (250 ml). The organic phase was dried (MgSO₄) and concentrated under reduced pressure. The residue was purified by flash column chromatography using ethylacetate 3 / 7 hexane as eluent to afford the amino benzothiazole as a colourless solid (1.25 g, 20 %).
¹H NMR (CDCl₃): 7.48 (1 H, d, *J* = 7.5 Hz, Ar); 7.24 (1 H, s, Ar); 6.76 (1 H, d, *J* = 7.5 Hz, Ar); 4.21 (2 H, q, *J* = 7.2 Hz, OCH₂CH₃); 3.86 (2 H, br s, NH₂); 1.25 (3 H, t, *J* = 7.2 Hz, OCH₂CH₃).

### 3-(Aminomethyl)benzoyl-D/L-phenylglycine 5-(hydroxymethyl)benzothiazol-2-amide

¹H NMR (d₄ methanol) : 8.15 - 7.56 (12 H, m, Ar); 5.97 (1 H, s, CH) ; 4.30 (2 H, s, CH₂OH) ; 4.19 (2 H, s, CH₂NH₂).
HPLC (Luna 2, Gradient 1): rt = 3.38 minutes.
LCMS (Luna 2, Gradient 4): rt = 2.50 minutes, 447 (M + H)⁺.

### Example 95

### 3-(Aminomethyl)benzoyl-DL-phenylglycine 6-(hydroxymethyl)benzothiazol-2-amide

Prepared using the methods as described in Example 91 substituting ethyl-4-aminobenzoate in place of ethyl-3-aminobenzoate.
¹H NMR (d₄ methanol) : 7.97 - 7.87 (2 H, m, Ar); 7.81 (1 H, s, Ar); 7.70 - 7.31 (9 H, m, Ar); 5.90 (1 H, s, CH); 4.68 (2 H, s, CH₂OH); 4.17 (2 H, s, CH₂NH₂).
HPLC (Luna 2, Gradient 1): rt = 3.29 minutes.
LCMS (Luna 2, Gradient 4): rt = 2.58 minutes, 447 (M + H)⁺.

### Example 96

### 3-(Aminomethyl)benzoyl-DL-phenylglycine N-methyl-tetrahydrobenzothiazol-2-amide

Prepared according to the method of Example 16 using 2-methylamino-tetrahydrobenzothiazole, synthesised as described below

### 2-methylamino-tetrahydrobenzothiazole

A mixture of 2-chlorocyclohexanone (1.14 ml, 10 mmol) and methylthiourea (902 mg, 10 mmol) in tetrahydrofuran (30 ml) was heated to reflux for 6 hours. The solution was cooled to room temperature, concentrated under reduced pressure and the residue was purified by flash column chromatography using ethylacetate 1 / 2 hexane as eluent to afford the thiazole as a colourless solid (1.07 g, 63 %).
¹H NMR (CDCl₃) : 5.98 (1 H, br s, NH); 2.98 (3 H, s, HNCH₃) ; 2.68 - 2.53 (4 H, m, 2 x CH₂chex); 1.92 - 1.80 (4 H, m, 2 x CH₂chex).

### 3-(Aminomethyl)benzoyl-D/L-phenylglycine N-methyl-tetrahydrobenzothiazol-2-amide

¹H NMR (d₄ methanol): 7.93 - 7.81 (2 H, m, Ar) ; 7.62 - 7.26 (7 H, m, Ar); 6.27 (1 H, s, CH); 4.08 (2 H, s, CH₂NH₂); 3.51 (3 H, br s, HNCH₃): 2.66 - 2.50 (4 H, m, 2 x CH₂chex) ; 1.85 - 1.68 (4 H, m, 2 x CH₂chex).
HPLC (Luna 2, Gradient 1): rt = 4.22 minutes.
LCMS (Luna 2, Gradient 4): rt = 2.55 minutes, 435 (M + H)⁺.

### Example 97

### 3-(Aminomethyl)benzoyl-D/L-3-(methylsulfonyl)phenylglycine tetrahydrobenzothiazol-2-amide

Prepared as described in Example 73 using 3-methylthiobenzaldehyde in place of 2-chlorobenzaldehyde. The 3-methylthiogroup was oxidised to the 3-methylsulfonyl group using metachloroperbenzoic acid prior to the final coupling step.
¹H NMR (d⁴ MeOH): 8.07 (1H, s, Ar); 7.90 - 7.77 (4H, m, Ar); 7.62 - 7.42 (3H, m, Ar); 5.91 (1H, s, CHPh); 4.09 (1H, s, CH₂₋NH₂); 3.05 (3H, s, SO₂CH₃); 2.60 (2H, bs, CH₂); 2.51 (2H, bs, CH₂); 1.75 (4H, bs, CH₂CH₂).
Hplc (Luna 2, Gradient 1): rt = 3.685 (96%)
LC/MS (Luna 2, Gradient 4): rt = 1.98 minutes, 499 MH⁺

### Example 98

### 3-(Aminomethyl)benzoyl-D/L-3-(methanesulfonyl)phenyl-glycine 3-ethoxycarbonyl- 4,5,6,7-tetrahydrobenzo(b)-thiophen-2-amide trifluoroacetate

Prepared as described for Example 93 using ethyl 2-amino-4,5,6,7-tetrahydrobenzo(b)thiophene-3-carboxylate in place of 2-amino-tetrahydrobenzothiazole
¹H NMR (d⁴ MeOH): 8.09 (1H, s, Ar); 8.0 - 7.86 (3H, m, Ar); 7.83 - 7.77 (1H, m, Ar); 7.66 - 7.48 (3H, m, Ar); 6.08 (1H, s, CHPh); 4.16 (2H, m, CH₂CH₃); 4.11 (1H, s, CH₂NH₂); 3.08 (3H, s, SO₂CH₃); 2.68 (2H, bs, CH₂); 2.57 (2H, bs, CH₂); 1.70 (4H, bs, CH₂CH₂); 1.21 (3H, t, *J* = 7Hz, CH₂CH₃).
Hplc (Luna 2, Gradient 1): rt = 4.733 (96%)
LC/MS (Luna 2, Gradient 4): rt = 2.604 minutes, 570 MH⁺

### Example 99

### 3-(Aminomethyl)benzoyl-D/L-2-chlorophenylglycine 3-aminocarbonyl-4,5,6,7-tetrahydrobenzo(b)thiophen-2-amide trifluoroacetate

Prepared as described for Example 73, using 2-amino-3-aminocarbonyl-4,5,6,7-tetrahydrobenzo(b)thiophene in place of 2-aminotetrahydrobenzothiazole.
¹H NMR (d⁴ MeOH): 8.14 (2H, m, Ar); 7.80 - 7.56 (4H, m, Ar); 7.56 - 7.45 (2H, m, Ar); 6.43 (1H, s, CHPh); 4.32 (1H, s, CH₂-NH₂); 2.87 (2H, bs, CH₂) ; 2.82 (2H, bs, CH₂) ; 1.97 (4H, pent, *J* = 3*Hz*, CH₂CH₂).
Hplc (Luna 2, Gradient 1): rt = 4.138 (99%)
LC/MS (Luna 2, Gradient 4): rt = 2.25 minutes, 497/499 MH⁺

### Example 100

### 3-(Aminomethyl) benzoyl-D/L-2-ethylthiazol-4-ylglycine-3-aminocarbonyl-tetrahydrobenzo(b)thiophen-2-amide hydrochloride salt

Prepared using the method of Example 38 using 2-amino-3-cyano-tetrahydrobenzo(b)thiophene (prepared according to general procedure A) and the protected amino acid N-t-butyloxycarbonyl-2-ethyl-thiazol-4-ylglycine. The amino acid was prepared using the procedure described in Example 74. The 3-cyano group was hydrolysed to 3-aminocarbonyl using HCl/diethyl ether at the final deprotection step.
¹H NMR (d⁴ MeOH): 8.18 (1H, s, Ar); 8.13 (1H, d, *J =* 7.5 *Hz,* Ar); 7.82 (1H, s, Ar); 7.77 (1H, d, *J =* 7.5 *Hz,* Ar); 7.69 (1H, t, *J =* 7.5 *Hz,* Ar); 6.26 (1H, s, CHPh); 4.31 (1H, s, CH₂NH₂); 3.27 (2H, q, *J =* 7.5 *Hz,* CH₂CH₃); 2.82 (2H, bs, CH₂); 2.77 (2H, bs, CH₂); 1.93 (4H, bd, CH₂CH₂); 1.52 (3H, t, *J =* 7.5 *Hz,* CH₂CH₃)
Hplc (Luna 2, Gradient 1): rt = 3.82 (95%)
LC/MS (Luna 2, Gradient 4): rt = 2.73 minutes, 498 MH⁺

### Example 101

### 3-(Aminomethyl) benzoyl-D/L-2-(trifluoromethoxy)phenyl-glycine tetrahydrobenzothiazol-2-amide TFA salt

Prepared according to the method of Example 73, substituting 2-trifluoromethoxybenzaldehyde for 2-chlorobenzaldehyde.
¹H NMR (d⁴ MeOH): 7.9 (2H, m, Ar); 7.71 - 7.37 (6H, m, Ar); 6.29 (1H, s, CHPh) ; 4.20 (2H, s, CH₂NH₂) ; 2.72 (2H, bs, CH₂) ; 2.63 (2H, bs, CH₂) ; 1.88 (4H, s, 2 x CH₂).
Hplc (Luna 2, Gradient 1): rt = 4.11 (99%)
LC/MS (Luna 2, Gradient 4): rt = 2.88 minutes, 506 MH⁺

### Example 102

### 3-(Aminomethyl) benzoyl-D/L-2-(trifluoromethoxy)phenylglycine-1-(3-methoxycarbonyl)-tetrahydrobenzothiophen-2-amide TFA salt

Prepared according to the method of Example 73, substituting 2-trifluoromethoxybenzaldehyde for 2-chlorobenzaldehyde and 2-amino-3-methoxycarbonyl-tetrahydrobenzothiophene in place of 2-aminotetrahydrobenzothiazole.
¹H NMR (d⁴ MeOH) : 8.05 (1H, s, Ar); 8.00 (1H, s, Ar); 7.7 - 7.4 (6H, m, Ar); 6.3 (1H, s, CHPh); 4.21 (2H, s, CH₂NH₂); 3.77 (3H, s, CO₂CH₃) ; 2.77 (2H, bs, CH₂) ; 2.67 (2H, bs, CH₂) ; 1.84 (4H, s, 2 x CH₂).
Hplc (Luna 2, Gradient 1): rt = 5.027 (100%)
LC/MS (Luna 2, Gradient 4): rt = 3.13 minutes, 562 MH⁺

### Example 103

### 3-(Aminomethyl)benzoyl-D/L-2-benzylthiazol-4-ylglycine 4,5,6,7-tetrahydrobenzothiazol-2-amide trifluoroacetate salt

¹H NMR (d₆ DMSO): 8.90 (1 H, d, J 6 Hz, NH), 8.05 (3 H, br s, NH-3⁺), 7.91-7.10 (11 H, NH & Ar), 5.91 (1 H, d, J 6 Hz, α-CH), 4.20 (2 H, s, CH₂Ph), 4.01-3.90 (2 H, m, CH₂N), 1.71-1.59 (4 H, m, CH₂CH₂CH₂CH₂). Peaks due to CH₂CH₂CH₂CH₂ protons are obscured by the water (from d₆ DMSO) peak around 2.4ppm.
Hplc (Luna 2, Gradient 1): rt = 4.34 minutes.
LC/MS (Luna 2, Gradient 4): rt = 2.39 minutes, 518 (MH⁺).

The compounds exemplified hereinabove have been found to be inhibitors of tryptase by the method of Tapparelli et al., (1993) J. Biol. Chem., 268, 4734 to 4741, and to be selective for tryptase over other serine proteases tested.

The following compounds are synthesised in similar manners to those illustrated by Examples 1 - 103.

### 3-(Aminomethyl)benzoyl-D/L-2-chlorophenylglycine 3-methanesulfonyl-4,5,6,7-tetrahydrobenzo(b)thiophen-2-amide trifluoroacetate salt

### 3-(Aminomethyl)benzoyl-D/L-2-chlorophenylglycine 3-aminocarbonyl-4,5,6,7-tetrahydrobenzo(b)thiophen-2-amide trifluoroacetate salt (Example 99)

### 3-(Aminomethyl)benzoyl-D/L-2-chlorophenylglycine 3-(pyrid-2-yl)- 4,5,6,7-tetrahydrobenzo(b)thiophen-2-amide trifluoroacetate salt

### 3-(Aminomethyl)benzoyl-D/L-2-ethylthiazol-4-ylglycine 3-methanesulfonyl-9,5,6,7-tetrahydrobenzo(b)thiophen-2-amide trifluoroacetate salt (Example 87)

### 3-(Aminomethyl)benzoyl-D/L-2-ethylthiazol-4-ylglycine 3-aminocarbonyl-4,5,6,7-tetrahydrobenzo(b)thiophen-2-amide trifluoroacetate salt (Example 100)

### 3-(Aminomethyl)benzoyl-D/L-2-ethylthiazol-4-ylglycine 3-(pyrid-2-yl)- 4,5,6,7-tetrahydrobenzo(b)thiophen-2-amide trifluoroacetate salt

### 3-(Aminomethyl)benzoyl-D/L-2-chloro-3-pyridylglycine 3-methanesulfonyl-9,5,6,7-tetrahydrobenzo(b)thiophen-2-amide trifluoroacetate salt (Example 92)

### 3-(Aminomethyl)benzoyl-D/L-2-chloro-3-pyridylglycine 3-aminocarbonyl-9,5,6,7-tetrahydrobenzo(b)thiophen-2-amide trifluoroacetate salt (Example 91)

### 3-(Aminomethyl)benzoyl-D/L-2-chloro-3-pyridylglycine 3-cyano-4,5,6,7-tetrahydrobenzo(b)thiophen-2-amide trifluoroacetate salt (Example 89)

### 3-(Aminomethyl)benzoyl-D/L-2-chloro-3-pyridylglycine 3-(pyrid-2-yl)- 4,5,6,7-tetrahydrobenzo(b)thiophen-2-amide trifluoroacetate salt

### 3-(Aminomethyl)benzoyl-D/L-2-chloro-3-pyridylglycine 3-ethoxycarbonyl- 4,5,6,7-tetrahydrobenzo(b)thiophene-2-amide trifluoroacetate salt (Example 90)

### 3-(Aminomethyl)benzoyl-D/L-2-chlorophenylglycine 4,5,6,7-tetrahydrobenzothiazol-2-amide trifluoroacetate salt (Example 88)

### 3-(Aminomethyl)benzoyl-D/L-2-benzylthiazol-4-ylglycine 3-methanesulfonyl-4,5,6,7-tetrahydrobenzo(b)thiophen-2-amide trifluoroacetate salt

### 3-(Aminomethyl)benzoyl-D/L-2-benzylthiazol-4-ylglycine 3-aminocarbonyl-4,5,6,7-tetrahydrobenzo(b)thiophen-2-amide trifluoroacetate salt

### 3-(Aminomethyl)benzoyl-D/L-2-benzylthiazol-4-ylglycine 3-cyano-4,5,6,7-tetrahydrobenzo(b)thiophen-2-amide trifluoroacetate salt

### 3-(Aminomethyl)benzoyl-D/L-2-benzylthiazol-4-ylglycine 3-(pyrid-2-yl)- 4,5,6,7-tetrahydrobenzo(b)thiophen-2-amide trifluoroacetate salt

### 3-(Aminomethyl)benzoyl-D/L-2-benzylthiazol-4-ylglycine 3-ethoxycarbonyl- 4,5,6,7-tetrahydrobenzo(b)thiophene-2-amide trifluoroacetate salt.

### 3-(Aminomethyl)benzoyl-D/L-2-benzylthiazol-4-ylglycine 4,5,6,7-tetrahydrobenzothiazol-2-amide trifluoroacetate salt (Example 103).

### 3-(Aminomethyl)benzoyl-D/L-6-methylpyridin-2-ylglycine 3-methanesulfonyl-4,5,6,7-tetrahydrobenzo(b)thiophen-2-amide trifluoroacetate salt

### 3-(Aminomethyl)benzoyl-D/L-6-methylpyridin-2-ylglycine 3-aminocarbonyl-4,5,6,7-tetrahydrobenzo(b)thiophen-2-amide trifluoroacetate salt

### 3-(Aminomethyl)benzoyl-D/L-6-methylpyridin-2-ylglycine 3-cyano-4,5,6,7-tetrahydrobenzo(b)thiophen-2-amide trifluoroacetate salt

### 3-(Aminomethyl)benzoyl-D/L-6-methylpyridin-2-ylglycine 3-(pyrid-2-yl)- 4,5,6,7-tetrahydrobenzo(b)thiophen-2-amide trifluoroacetate salt

### 3-(Aminomethyl)benzoyl-D/L-6-methylpyridin-2-ylglycine 3-ethoxycarbonyl- 4,5,6,7-tetrahydrobenzo(b)thiophene-2-amide trifluoroacetate salt.

**3-(Aminomethyl)benzoyl-D/L-6-methylpyridin-2-ylglycine 4,5,6,7-tetrahydrobenzothiazol-2-amide trifluoroacetate salt.**

## Claims

1. A tryptase inhibitor compound of formula (I) 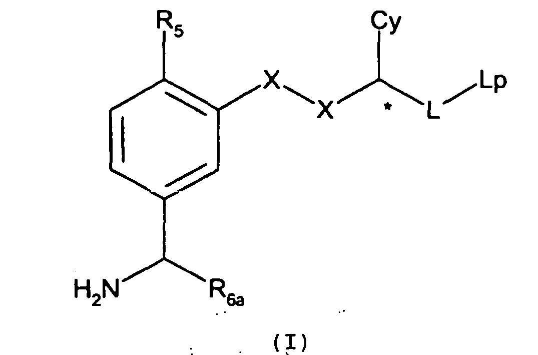 where:
R₅ represents amino, hydroxy, aminomethyl, hydroxymethyl or hydrogen;
R₆ₐ represents hydrogen or methyl;
X-X is -CONR₁ₐ-;
R₁ₐ represents hydrogen, (1-6C)alkyl or phenyl(1-6C)alkyl;
L is CONR_{1d}(CH₂)ₘ in which m is 0 or 1 and R_{1d} is hydrogen, (1-6C)alkyl or phenyl(1-6C)alkyl;
Cy is a saturated or unsaturated, mono or poly cyclic, homo or heterocyclic group, preferably containing 5 to 10 ring atoms and optionally substituted by one or more groups R₃ₐ- or R₃ᵢXᵢ-;
each R₃ₐ independently is hydrogen, hydroxyl, (1-6C) alkoxy, (1-6C) alkyl, (2-6C)alkenyl, (2-6C)alkynyl, (1-6C)alkanoyl, (1-6C) alkylaminoalkyl, hydroxy(1-6C)alkyl, carboxy, (1-6C) alkoxyalkyl, (1-6C) alkoxycarbonyl, (1-6C) alkylaminocarbonyl, amino(1-6C)alkyl, CONH₂, CH₂CONH₂, aminoacetyl, (1-6C)alkanoylamino, hydroxy(1-6C)alkanoylamino, amino(1-6C)alkanoylamino, (1-6C)alkylamino(1-6C)alkanoylamino, di(1-6C)alkylamino(1-6C)alkanoylamino, (1-6C) alkoxycarbonylamino, amino, halo, cyano, nitro, thiol,(1-6C) alkylthio, (1-6C) alkylsulphonyl, (1-6C) alkylsulphenyl, imidazolyl, hydrazido, (1-6C)alkylimidazolyl, (1-6C) alkylsulphonamido, (1-6C) alkylaminosulphonyl, aminosulphonyl, (1-6C) haloalkoxy, or (1-6C) haloalkyl;
X₁ is a bond, O, NR₁ₚ, CH₂, CO, CONR_{1P}, NR₁ₚCO, SO₂, NR₁ₚSO₂ or SO₂NR₁ₚ;
R₃ᵢ is phenyl or pyridyl;
R₁ₚ is as defined for R₁ₐ;
Lp is selected from 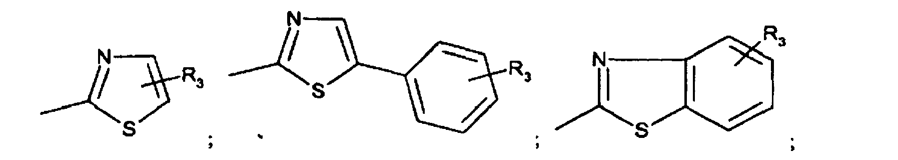 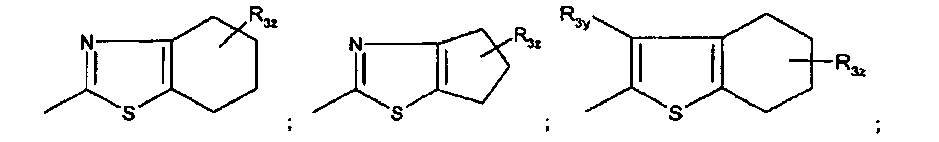 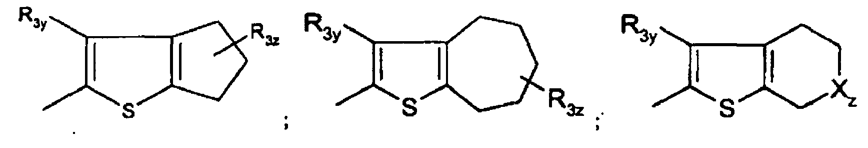 and 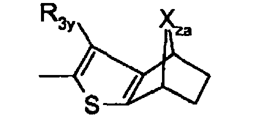 in which R₃ is an amino acid residue, N-(1-6C)alkylaminocarbonyl, N,N-di(1-6C)alkylaminocarbonyl, N-(1-6C)alkylaminoalkanoyl, N-(1-6C)alkanoylamino(1-6C)alkanonyl, C-hydroxyamino(1-6C)alkanoyl, hydroxy(2-6C)alkanoylamino(1-6C)alkanoyl, di(1-6C)alkylaminosulfonyl, hydrogen, hydroxyl, (1-6C)alkoxy, (1-6C)alkanoyloxy, (1-6C) alkyl, (2-6C)alkenyl (2-6C)alkynyl, (3-6C)alkenyloxycarbonyl, (1-6C)alkanoyl, amino(1-6C)alkyl, amido (CONH₂), amino(1-6C)alkanoyl, aminocarbonyl(1-5C)alkanoyl, hydroxy(1-6C)alkyl, carboxy, hydroxy(1-6C)alkanoyl, (1-6C)alkoxy(1-6C)alkanoyl, (1-6C)alkoxy(1-6C)alkyl, (1-6C)alkoxycarbonyl(1-5C)alkyl, (1-6C)alkoxycarbonyl, (1-6C)alkanoylamino, amino, halo, cyano, nitro, thiol, (1-6C) alkylthio, (1-6C)alkylsulfonyl, (1-6C)alkylsulphenyl or hydrazido;
R_{3y} represents R₃ or a group of formula
Rₖ-G₂-Xₐ-
in which G₂ is absent or represents (1-3C)alkanediyl, Xₐ is absent or represents O, S, SO, SO₂ NR_{L}, CO, OCO, COO, CONR_{L}, NR_{L}CO, SO₂NR_{L} or NR_{L}SO₂; Rₖ represents a carbocyclic or heterocyclic group, optionally substituted by R₃; and R_{L} represents hydrogen or (1-6C)alkyl;
R_{3z} is oxo or is as defined for R_{3y}, X_{za} is CH₂ and X_{z} is O, S or NR_{z} in which R_{z} has a value independently selected from a value for R_{3y};
or a physiologically tolerable salt thereof.

2. A compound as claimed in Claim 1, in which:
each R₃ₐ independently is hydrogen, hydroxyl, (1-6C) alkoxy, (1-6C) alkyl, (2-6C)alkenyl, (2-6C)alkynyl, (1-6C)alkanoyl, (1-6C) alkylaminoalkyl, hydroxy(1-6C)alkyl, carboxy, (1-6C) alkoxyalkyl,(1-6C) alkoxycarbonyl, (1-6C) alkylaminocarbonyl, amino(1-6C)alkyl, CONH₂, CH₂CONH₂, aminoacetyl, (1-6C)alkanoylamino, (1-6C) alkoxycarbonylamino, amino, halo, cyano, nitro, thiol, (1-6C) alkylthio, (1-6C) alkylsulphonyl, (1-6C) alkylsulphenyl, imidazolyl, hydrazido, (1-6C)alkylimidazolyl, (1-6C) alkylsulphonamido, (1-6C) alkylaminosulphonyl, aminosulphonyl, (1-6C) haloalkoxy, or (1-6C) haloalkyl;
Xᵢ is a bond, O, NH or CH₂; and R₃ᵢ is phenyl optionally substituted by R₃ₐ; and
Lp is selected from 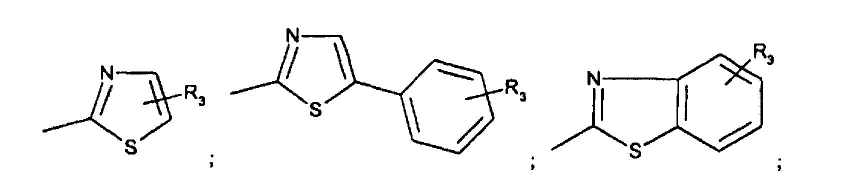 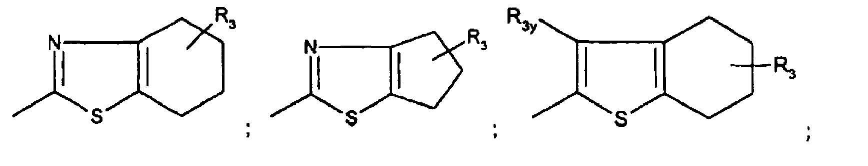 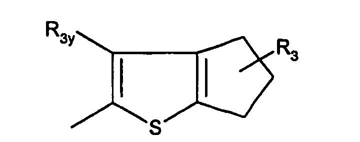 R₃ is an amino acid residue, N-(1-6C)alkylaminocarbonyl, N,N-di(1-6C)alkylaminocarbonyl, N-(1-6C)alkylaminoalkanoyl, N-(1-6C)alkanoylamino(1-6C)alkanonyl, C-hydroxyamino(1-6C)alkanoyl, hydroxy(2-6C)alkanoylamino(1-6C)alkanoyl,di(1-6C)alkrlaminosulfonyl, hydrogen, hydroxyl, (1-6C)alkoxy, (1-6C)alkanoyloxy, (1-6C) alkyl, (2-6C)alkenyl (2-6C)alkynyl, (3-6C)alkenyloxycarbonyl, (1-6C)alkanoyl, amino (1-6C) alkyl, amido (CONH₂), amino(1-6C)alkanoyl, aminocarbonyl(1-5C)alkanoyl, hydroxy(1-6C)alkyl, carboxy, hydroxy(1-6C)alkanoyl, (1-6C)alkoxy(1-6C)alkyl, (1-6C)alkoxycarbonyl(1-5C)alkyl, (1-6C)alkoxycarbonyl, (1-6C)alkanoylamino, amino, halo, cyano, nitro, thiol, (1-6C) alkylthio, (1-6C)alkylsulfonyl, (1-6C)alkylsulphenyl or hydrazido; and
R_{3y} represents R₃ or a group of formula
Rₖ-G₂-Xₐ-
in which G₂ represents a bond or (1-3C)alkanediyl, Xₐ represents a bond, CO, OCO, COO or NHCO, and Rₖ represents a carbocyclic or heterocyclic group, optionally substituted by R₃.

3. A compound as claimed in Claim 1 or Claim 2, in which R₅ is amino or hydrogen.

4. A compound as claimed in Claim 3, in which R₅ is hydrogen.

5. A compound as claimed in Claim 4, in which R₆ₐ is hydrogen.

6. A compound as claimed in any one of Claims 1 to 5, in which X-X is CONH.

7. A compound as claimed in any one of Claims 1 to 6, in which the alpha carbon atom (*) has the conformation that would result from construction from a D-α-aminoacid NH₂-CH(Cy)-COOH where the NH₂ represents part of X-X.

8. A compound as claimed in any one of Claims 1 to 7, in which Cy represents cycloalkyl, piperidinyl, phenyl, 3,4-methylenedioxyphenyl, furyl, thienyl, imidazolyl, oxazolyl, thiazolyl, pyridyl, pyrimidinyl, pyrazinyl, naphthyl, indolyl, indanyl, 3,4-dihydrobenzofuryl, benzofuryl or benzo[b]thienyl group, optionally substituted by R₃ₐ or R₃ᵢXᵢ.

9. A compound as claimed in Claim 8, in which Cy represents cyclohexyl, piperidin-4-yl, phenyl, 3,4-methylenedioxy-phenyl, fur-2-yl, thien-2-yl, thien-3-yl, imidazol-4-yl, oxazol-4-yl, oxazol-5-yl, thiazol-4-yl, thiazol-5-yl, pyrid-2-yl, pyrid-3-yl, pyrid-4-yl, pyrimidin-2-yl, pyrimidin-4-yl, pyrimidin-5-yl, pyrazin-2-yl, pyrazin-3-yl, naphth-1-yl, naphth-2-yl, indol-5-yl, indan-5-yl, 3,4-dihydrobenzofur-5-yl, benzofur-2-yl or benzo[b]thien-2-yl, optionally substituted by R₃ₐ or R₃ᵢXᵢ.

10. A compound as claimed in Claim 8 or Claim 9, in which R₃ₐ is hydrogen; hydroxyl; methoxy; ethoxy; isopropoxy; methyl; ethyl; isopropyl; acetyl; propanoyl; isopropanoyl; methylaminomethyl; dimethylaminomethyl; hydroxymethyl; carboxy; methoxymethyl; methoxycarbonyl; ethoxycarbonyl; methylaminocarbonyl; dimethylaminocarbonyl; aminomethyl; CONH₂; CH₂CONH₂; aminoacetyl; formylamino; acetylamino; hydroxyacetylamino, dimethylaminoacetylamino, methoxycarbonylamino; ethoxycarbonylamino; t-butoxycarbonylamino; amino; fluoro; chloro; bromo; cyano; nitro; thiol; methylthio; methylsulphonyl; ethylsulphonyl; methylsulphenyl; imidazol-4-yl; hydrazido; 2-methylimidazol-4-yl; methylsulphonylamido; ethylsulphonylamido; methylaminosulphonyl; ethylaminosulphonyl; aminosulphonyl; trifluoromethoxy or trifluoromethyl; and R₃ᵢXᵢ is phenyl, phenoxy, phenylamino or benzyl.

11. A compound as claimed in Claim 10, in which Cy is selected from cyclohexyl, piperidin-4-yl, 1-acetylpiperidin-4-yl, 1-propanoylpiperidin-4-yl, 1-isobutyrylpiperidin-4-yl, 1-aminoacetylpiperidin-4-yl, phenyl, 2-aminophenyl, 4-aminophenyl, 3-hydroxyphenyl, 4-methylphenyl, 2,4-dimethylphenyl, 3,6-dimethylphenyl, 4-ethylphenyl, 4-isopropylphenyl, 4-hydroxphenyl, 3-aminomethylphenyl, 4-aminomethylphenyl, 4-(H₂NCO)phenyl, 4-hydroxymethylphenyl, 3-hydroxymethylphenyl, 2-hydroxymethylphenyl, 4-carboxyphenyl, 4-isopropoxyphenyl, 2-chlorophenyl, 3,4-methylenedioxyphenyl, 4-phenylphenyl, 4-phenoxyphenyl, 5-methylfur-2-yl, imidazol-4-yl, 2-methylthiazol-4-yl, 2-aminothiazol-4-yl, 2-formylaminothiazol-4-yl, 2-aminothiazol-5-yl, 2-formylaminothiazol-5-yl, 2-phenylthiazol-4-yl, 4-aminopyrid-3-yl, 6-methylpyrid-2-yl, 3-amino-pyrid-4-yl, naphth-1-yl, naphth-2-yl, benzofur-2-yl, 2-methoxyphenyl, 3-methoxyphenyl, 4-methoxyphenyl, indan-5-yl, 2-methylthiophenyl, 3-methylthiophenyl, 3-methylsulfinylphenyl, 2-methylsulphonylphenyl, 3-methylsulphonylphenyl, 3-N,N-dimethylaminophenyl, 2,3-dihydrobenzofuran-5-yl, 3-bromophenyl, 3-cyanophenyl, 2-methoxycarbonylphenyl, 2-ethoxycarbonylphenyl, 2-methylphenyl, 2-fluorophenyl, 6-aminopyrid-3-yl, 2-ethylthiazol-4-yl, 2-benzylthiazol-4-yl, 2-methylsulfonamidothiazol-4-yl, 2-chloropyrid-3-yl, 2-hydroxyacetylaminothiazol-4-yl, 2-N,N-dimethylaminoacetyl-aminothiazol-4-yl, 2-trifluoromethoxyphenyl, 2-trifluoromethylphenyl, 3-chloropyrid-2-yl, 3-methylpyrid-2-yl, pyrazin-2-yl, pyrazin-3-yl, pyrimidin-2-yl and pyrimidin-3-yl.

12. A compound as claimed in any one of Claims 1 to 11, in which Cy is a group of formula 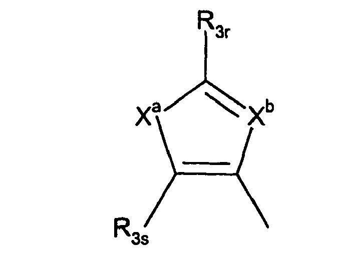 in which one of X^{a} and X^{b} is N and the other is NH or S, and each of R₃ᵣ and R₃ₛ is as defined for R₃ₐ.

13. A compound as claimed in Claim 12, in which X^{a} is S and X^{b} is N.

14. A compound as claimed in Claim 12 or Claim 13, in which R₃ₛ is hydrogen and R₃ᵣ is hydrogen, (1-6C)alkyl, amino, (1-6C)alkanoylamino, hydroxy(1-6C)alkanoylamino, N,N-di(1-6C)alkylaminoalkanoylamino, (1-6C)alkylsulfonylamino, phenyl or benzyl.

15. A compound as claimed in any one of Claims 1 to 14, in which Cy is pyrid-2-yl, pyrimidin-2-yl, pyrimidin-4-yl, pyrazin-2-yl, pyrazin-3-yl or oxazol-4-yl optionally substituted by R₃ₐ or R₃ᵢXᵢ.

16. A compound as claimed in any one of Claims 2 to 15, in which Cy is cycloalkyl, piperidinyl, phenyl, 3,4-methylenedioxyphenyl, furyl, thienyl, imidazolyl, thiazolyl, pyridyl, naphthyl, benzofuryl, or benzo[b]thienyl group, optionally substituted by R₃ₐ or R₃ᵢXᵢ in which Xᵢ is a bond, O, NH or CH₂ and R₃ᵢ is phenyl optionally substituted by R₃ₐ and each R₃ₐ independently is hydrogen, hydroxyl, (1-6C) alkoxy, (1-6C) alkyl, (1-6C)alkanoyl, (1-6C) alkylaminoalkyl, hydroxy(1-6C)alkyl, carboxy, (1-6C) alkoxyalkyl,(1-6C) alkoxycarbonyl, (1-6C) alkylaminocarbonyl, amino(1-6C)alkyl CONH₂, CH₂CONH₂, aminoacetyl, (1-6C) alkanoylamino, (1-6C) alkoxycarbonylamino, amino, halo, cyano, nitro, thiol, (1-6C) alkylthio, (1-6C) alkylsulphonyl, (1-6C) alkylsulphenyl, imidazolyl, hydrazido,(1-6C)alkylimidazolyl,(1-6C) alkylsulphonamido, (1-6C) alkylaminosulphonyl, aminosulphonyl, (1-6C) haloalkoxy, or (1-6C) haloalkyl.

17. A compound as claimed in Claim 16, in which Cy is cyclohexyl, piperidin-4-yl, phenyl, 3,4-methylenedioxyphenyl, fur-2-yl, thien-2-yl, thien-3-yl, imidazol-4-yl, thiazol-4-yl, thiazol-5-yl, pyrid-2-yl, pyrid-3-yl, pyrid-4-yl, naphth-1-yl, naphth-2-yl, benzofur-2-yl, or benzo[b]thien-2-yl group, optionally substituted by R₃ₐ or R₃ᵢXᵢ.

18. A compound as claimed in Claim 16 or Claim 17, in which R₃ₐ is hydrogen; hydroxyl; methoxy; ethoxy; isopropoxy; methyl; ethyl; isopropyl; acetyl; propanoyl; isopropanoy; methylaminomethyl; dimethylaminomethyl; hydroxymethyl; carboxy; methoxymethyl; methoxycarbonyl; ethoxycarbonyl; methylaminocarbonyl; dimethylaminocarbonyl; aminomethyl; CONH₂; CH₂CONH₂; aminoacetyl; formylamino; acetylamino; methoxycarbonylamino; ethoxycarbonylamino; t-butoxycarbonylamino; amino; fluoro; chloro; cyano; nitro; thiol; methylthio; methylsulphonyl; ethylsulphonyl; methylsulphenyl; imidazol-4-yl; hydrazido; 2-methylimidazol-4-yl; methylsulphonylamido; ethylsulphonylamido; methylaminosulphonyl; ethylaminosulphonyl; aminosulphonyl; trifluoromethoxy or trifluoromethyl; and R₃ᵢXᵢ is phenyl, phenoxy, phenylamino or benzyl.

19. A compound as claimed in Claim 18, in which Cy is cyclohexyl, piperidin-4-yl, 1-acetylpiperidin-4-yl, 1-propanoylpiperidin-4-yl, 1-isobutyrylpiperidin-4-yl, 1-aminoacetylpiperidin-4-yl, phenyl, 4-aminophenyl, 3-hydroxyphenyl, 4-methylphenyl, 2,4-dimethylphenyl, 3,6-dimethylphenyl, 4-ethylphenyl, 4-isopropylphenyl, 4-hydroxphenyl, 3-aminomethylphenyl, 4-aminomethylphenyl, 4-(H₂NCO)phenyl, 4-hydroxymethylphenyl, 3-hydroxymethylphenyl, 2-hydroxymethylphenyl, 4-carboxyphenyl, 4-isopropoxyphenyl, 2-chlorophenyl, 3,4-methylenedioxyphenyl, 4-phenylphenyl, 4-phenoxyphenyl, 5-methylfur-2-yl, imidazol-4-yl, 2-methylthiazol-4-yl, 2-aminothiazol-4-yl, 2-formylaminothiazol-4-yl, 2-aminothiazol-5-yl, 2-formylaminothiazol-5-yl, 2-phenylthiazol-4-yl, 4-aminopyrid-3-yl, 6-methylpyrid-2-yl, 3-amino-pyrid-4-yl, naphth-1-yl, naphth-2-yl, benzofur-2-yl or 3-methylbenzothien-2-yl.

20. A compound as claimed in any one of Claims 1 to 19, in which L represents CO, CONH, CONCH₃ or CONHCH₂.

21. A compound as claimed in Claim 20, in which L is CONH or CONCH₃.

22. A compound as claimed in any one of Claims 1 to 21, in which R₃ is selected from N-acetylalaninoyl; serinoyl; threoninoyl; aspartoyl; glutamoyl; N,N-dimethylaminocarbonyl; N,N-diethylaminocarbonyl; N-(1,3-dimethyl)butylaminocarbonyl; N-methyl-N-ethylaminocarbonyl; N-methylacetyl; 2-N-acetylaminoacetyl; 2-N-acetylaminopropanoyl; 2-N-(2-methylpropanoyl)aminoacetyl; 2-amino-3-hydroxypropanoyl; 2-amino-3-hydroxybutanoyl; 2-hydroxyacetylaminoacetyl; dimethylaminosulfonyl; hydrogen; hydroxyl; methoxy; acetoxy; methyl; ethyl; propyl; 2-propyl; 2,2-dimethylethyl; allyl; propynyl; allyloxycarbonyl; acetyl; propionyl; isobutyryl; aminomethyl; CONH₂; aminoacetyl; aminopropionyl; 2-aminopropionyl; aminocarbonylacetyl; hydroxymethyl; 1-hydroxyethyl; carboxy; 2-hydroxyacetyl; 2-hydroxypropanoyl; methoxyacetyl; methoxymethyl; methoxycarbonylmethyl; methoxycarbonyl; ethoxycarbonyl; formylamino; acetylamino; amino; chloro; cyano; nitro; thiol; methylthio; methylsulphonyl; ethylsulfonyl; methylsulphenyl; and hydrazido.

23. A compound as claimed in any one of Claims 2 to 22, in which R₃ is selected from N-acetylalaninoyl; serinoyl; threoninoyl; aspartoyl; glutamoyl; N-(1,3-dimethyl)butylamino-carbonyl; N-methyl-N-ethylaminocarbonyl; N-methylacetyl; 2-N-acetylaminoacetyl; 2-N-acetylaminopropanoyl; 2-N-(2-methylpropanoyl)aminoacetyl; 2-amino-3-hydroxypropanoyl; 2-amino-3-hydroxybutanoyl; 2-hydroxyacetylaminoacetyl; dimethylaminosulfonyl; hydrogen; hydroxyl; methoxy; acetoxy; methyl; ethyl; propyl; 2-propyl; 2,2-dimethylethyl; allyl; propynyl; allyloxycarbonyl; acetyl; propionyl; isobutyryl; aminomethyl; CONH₂; aminoacetyl; aminopropionyl; 2-aminopropionyl; aminocarbonylacetyl; hydroxymethyl; 1-hydroxyethyl; carboxy; 2-hydroxyacetyl; 2-hydroxypropanoyl; methoxymethyl; methoxycarbonylmethyl; methoxycarbonyl; ethoxycarbonyl; formylamino; acetylamino; amino; chloro; cyano; nitro; thiol; methylthio; methylsulphonyl; ethylsulfonyl; methylsulphenyl; and hydrazido.

24. A compound as claimed in any one of Claims 1 to 23, in which L represents CONR_{1d} and Lp represents

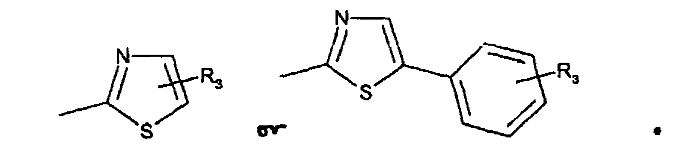

25. A compound as claimed in Claim 24, in which each R₃ is selected independently from hydrogen, amino, hydroxy, (1-6C)alkyl, (1-6C)alkanoyl, (1-6C)alkanoyloxy, (1-5C)alkoxycarbonyl(1-6C)alkyl, amino(1-6C)alkyl and cyano.

26. A compound as claimed in any one of Claims 1 to 24, in which L represents CONR_{1d} and Lp represents 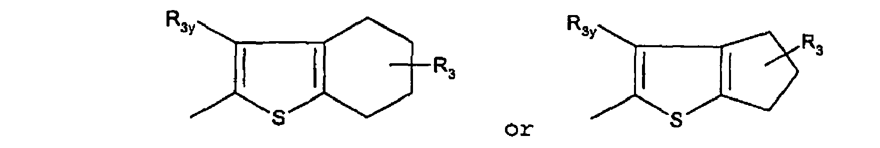 in which R_{3y} represents R₃ or a group of formula
Rₖ-G₂-Xₐ-
in which G₂ represents a bond or (1-3C)alkanedaiyl, Xₐ represents a bond, CO, OCO, COO or NHCO, and Rₖ represents a carbocyclic or heterocyclic group, optionally substituted by R₃.

27. A compound as claimed in Claim 26, in which Lp is selected from 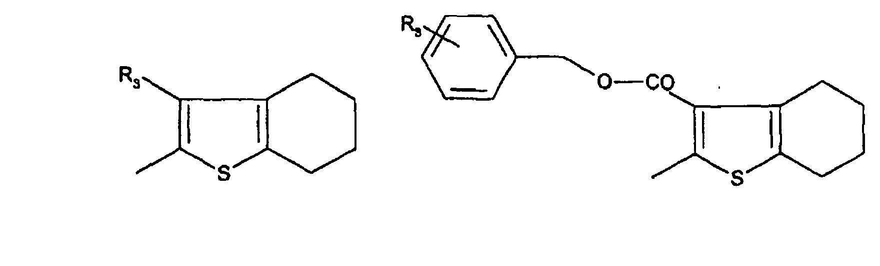 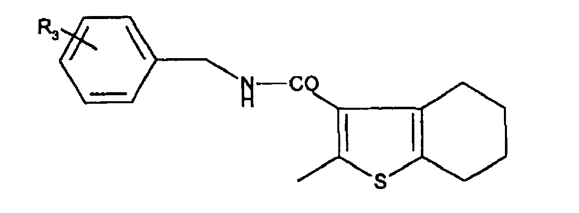 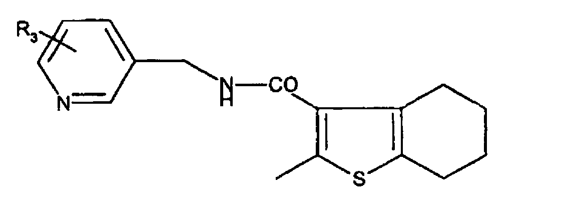 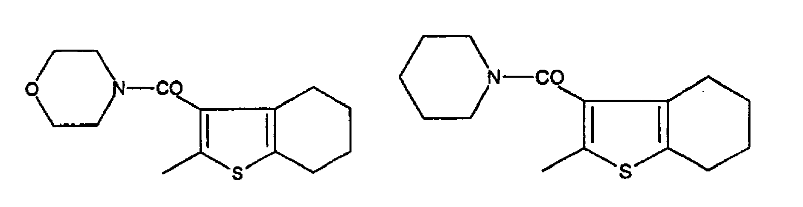 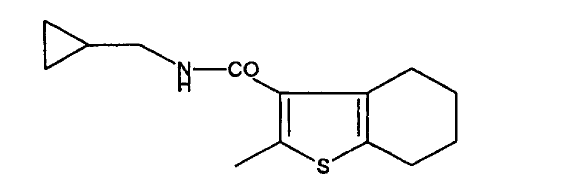 in which (i) when R₃ is present as a substituent at the 3-position of a 4,5,6,7-tetrahydrobenzothiophene group, it represents a carboxy group; a (1-6C)alkoxycarbonyl group; or a (1-6C)alkylaminocarbonyl group; and (ii) when R₃ is present as a substituent on a phenyl or pyridyl group, it is a hydrogen atom.

28. A compound as claimed in any one of Claims 1 to 24, in which L represents CONR_{1d} and Lp represents

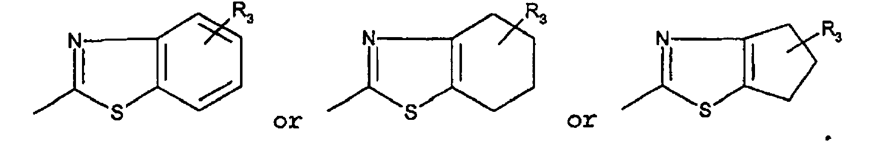

29. A compound as claimed in Claim 28, in which the heterocyclic group is substituted by one or two R₃ groups.

30. A compound as claimed in Claim 29, in which each R₃ group is selected from hydrogen, halogen, (1-6C)alkyl and (1-6C)alkoxy.

31. A compound as claimed in any one of Claims 1 to 24, in which L represents CONR_{1d} and Lp represents 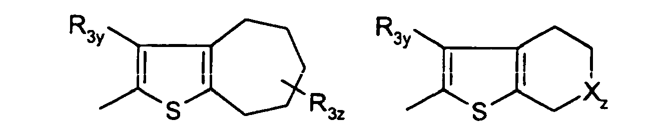 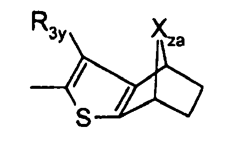 in which R_{3y}, R_{3z}, X_{z} and X_{za} are as defined hereinabove.

32. A compound as claimed in Claim 31, in R_{3y} is (1-6C)alkoxycarbonyl, N,N-dialkylaminocarbonyl or cyano; R_{3z} is hydrogen; X_{z} is O, S or NR_{z}, R_{z} is hydrogen, (1-6C) alkanoyl, amino(1-6C)alkanoyl, (1-6C)alkoxy(1-6C)alkanoyl or benzyloxycarbonyl; and X_{za} is CH₂.

33. A compound as claimed in Claim 32, in which Lp is 3-ethoxycarbonyl-tetrahydro-4H-cyclohepta(b)thien-2-yl, 3-ethoxycarbonyl-4,5-dihydro-5H-thieno[2,3-c]pyranyl, 3-ethoxycarbonyl-4,5-dihydro-5H-thieno[2,3-c]thiopyranyl, 3-dimethylaminocarbonyl-6-benzyloxycarbonyltetrahydrothieno[2,3-b]pyridin-2-yl, 3-dimethylaminocarbonyl-tetrahydrothieno[2,3-b]pyridin-2-yl, 3-dimethylaminocarbonyl-6-acetyltetrahydrothieno[2,3-b]pyridin-2-yl, 3-dimethylaminocarbonyl-6-aminoacetyltetrahydrothieno-[2,3-b]pyridin-2-yl, 3-dimethylaminocarbonyl-6-methoxyacetyl-tetrahydrothieno[2,3-b]pyridin-2-yl or 3-ethoxycarbonyl-tetrahydro-4,7-methanobenzo(b)thophen-2-yl.

34. A compound as claimed in any one of Claims 1 to 33, in which L is CONH and Lp is 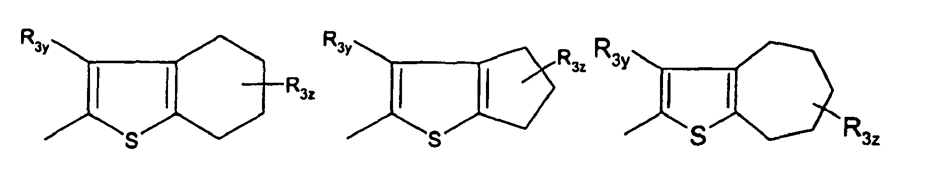 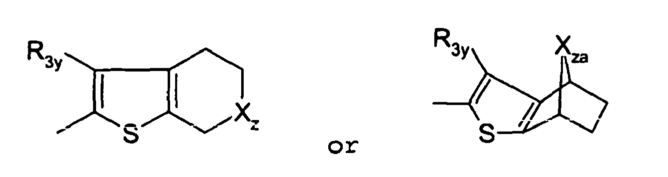 in which R_{3y} is selected from N,N-di-(1-6C)alkylaminocarbonyl, di(1-6C)alkylaminosulfonyl, (3-6C)alkenyloxycarbonyl, (1-6C)alkanoyl, hydroxy(1-6C)alkanoyl, (1-6C)alkoxy(1-6C)alkanoyl, (1-6C)alkoxycarbonyl, (1-6C)alkylsulfonyl, (1-6C)alkylsulphenyl, and Rₖ-G₂-Xₐ in which either Xₐ is CO, OCO, NR_{L}CO, (where R_{L} is (1-6C)alkyl), SO₂ or NR_{L}SO₂ and Rₖ and G₂ are as defined previously, or Xₐ and G₂ are both absent and Rₖ is pyrid-2-yl, thiazol-2-yl, thiazol-4-yl, pyrazin-2-yl, pyrazin-3-yl, pyrimidin-2-yl or pyrimidin-4-yl.

35. A pharmaceutical composition, which comprises a compound as claimed in any one of Claims 1 to 34 together with at least one pharmaceutically acceptable carrier or excipient.

36. A compound as claimed in any one of Claims 1 to 34 for use in therapy.

37. Use of a compound as claimed in any one of Claims 1 to 34 in the manufacture of a medicament for use in the treatment of the human or non-human body to combat a condition responsive to a tryptase inhibitor.

38. Use as claimed in Claim 37, in which said condition is selected from asthma, allergic rhinitis, eczema, psoriasis, atopic dermatitis, urticaria, rheumatoid arthritis, conjunctivitis, inflammatory bowel disease, neurogenic inflammation, atherosclerosis and cancer.

39. Use as claimed in Claim 38, in which said condition is asthma.

40. A compound of formula (IX)
Z₂-CH(Cy)-L-Lp (IX)
wherein Cy, L and Lp are as defined in any one of Claims 1 to 34 and Z₂ is NH₂ or a salt thereof but excluding the compounds phenylglycine 4-methyl-thiazol-2-amide and phenylglycine 5-methylthiazol-2-ylmethylamide.

## Patentansprüche

1. Tryptaseinhibitorverbindung der Formel (I) 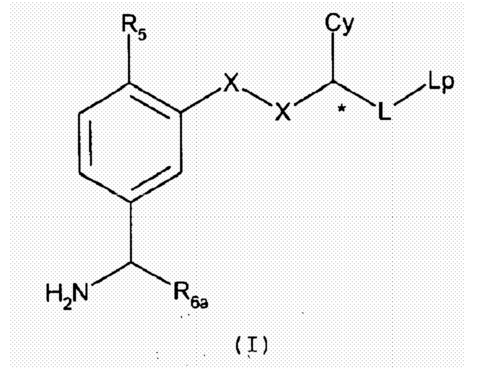 worin
R₅ für Amino, Hydroxy, Aminomethyl, Hydroxymethyl oder Wasserstoff steht,
R₆ₐ für Wasserstoff oder Methyl steht,
X-X für -CONR₁ₐ- steht,
R₁ₐ für Wasserstoff, C₁-C₆ Alkyl oder Phenyl-C₁-C₆-alkyl steht,
L für CONR_{1d}(CH₂)ₘ, steht, worin m für 0 oder 1 steht und R_{1d} für Wasserstoff, C₁-C₆ Alkyl oder Phenyl-C₁-C₆-alkyl steht,
Cy für eine gesättigte oder ungesättigte, mono- oder polycyclische, homo- oder heterocyclische Gruppe steht, die vorzugsweise 5 bis 10 Ringatome enthält und optional durch eine oder mehrere Gruppen R₃ₐ-oder R₃ᵢXᵢ- substituiert ist,
R₃ₐ jeweils unabhängig steht für Wasserstoff, Hydroxyl, C₁-C₆ Alkoxy, C₁-C₆ Alkyl, C₂-C₆ Alkenyl, C₂-C₆ Alkinyl, C₁-C₆ Alkanoyl, C₁-C₆ Alkylaminoalkyl, Hydroxy-C₁-C₆-alkyl, Carboxy, C₁-C₆ Alkoxyalkyl, C₁-C₆ Alkoxycarbonyl, C₁-C₆ Alkylaminocarbonyl, Amino-C₁-C₆-alkyl, CONH₂, CH₂CONH₂, Aminoacetyl, C₁-C₆ Alkanoylamino, Hydroxy-C₁-C₆-alkanoylamino, Amino-C₁-C₆-alkanoylamino, C₁-C₆ Alkylamino-C₁-C₆-alkanoylamino, Di-C₁-C₆-alkylamino-C₁-C₆-alkanoylamino, C₁-C₆ Alkoxycarbonylamino, Amino, Halogen, Cyano, Nitro, Thiol, C₁-C₆ Alklythio, C₁-C₆ Alkylsulfonyl, C₁-C₆ Alkylsulfenyl, Imidazolyl, Hydrazido, C₁-C₆ Alkylimidazolyl, C₁-C₆ Alkylsulfonamido, C₁-C₆ Alkylaminosulfonyl, Aminosulfonyl, C₁-C₆ Halogenalkoxy oder C₁-C₆ Halogenalkyl, und
Xᵢ für eine Bindung, O, NR₁ₚ, CH₂, CO, CONR₁ₚ, NR₁ₚCO, SO₂, NR₁ₚSO₂ oder SO₂NR₁ₚ steht,
R₃ᵢ für Phenyl oder Pyridyl steht,
R₁ₚ wie für R₁ₐ definiert ist,
Lp ausgewählt ist aus 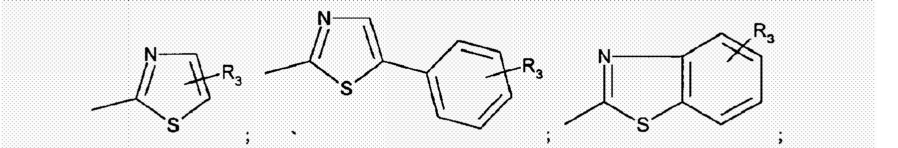 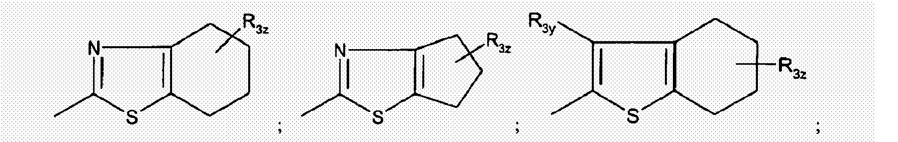 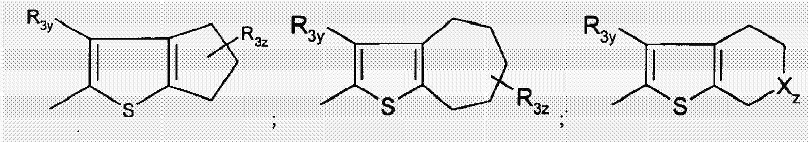 und 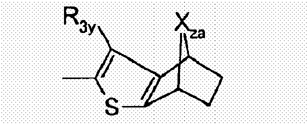 worin R₃ steht für einen Aminosäurerest, N-C₁-C₆ Alkylaminocarbonyl, N,N-Di-C₁-C₆-alkylaminocarbonyl, N-C₁-C₆ Alkylaminoalkanoyl, N-C₁-C₆ Alkanoylamino-C₁-C₆-alkanoyl, C-Hydroxyamino-C₁-C₆-alkanoyl, Hydroxy-C₂-C₆-alkanoylamino-C₁-C₆-alkanoyl, Di-C₁-C₆-alkylaminosulfonyl, Wasserstoff, Hydroxyl, C₁-C₆ Alkoxy, C₁-C₆ Alkanoyloxy, C₁-C₆ Alkyl, C₂-C₆ Alkenyl, C₂-C₆ Alkinyl, C₃-C₆ Alkenyloxycarbonyl, C₁-C₆ Alkanoyl, Amino-C₁-C₆-alkyl, Amido (CONH₂), Amino-C₁-C₆-alkanoyl, Aminocarbonyl-C₁-C₅-alkanoyl, Hydroxy-C₁-C₆-alkyl, Carboxy, Hydroxy-C₁-C₆-alkanoyl, C₁-C₆ Alkoxy-C₁-C₆-alkanoyl, C₁-C₆ Alkoxy-C₁-C₆-alkyl, C₁-C₆ Alkoxycarbonyl-C₁-C₅-alkyl, C₁-C₆ Alkoxycarbonyl, C₁-C₆ Alkanoylamino, Amino, Halogen, Cyano, Nitro, Thiol, C₁-C₆ Alkylthio, C₁-C₆ Alkylsulfonyl, C₁-C₆ Alkylsulfenyl oder Hydrazido,
R_{3y} für R₃ oder eine Gruppe der folgenden Formel steht
Rₖ - G₂ - Xₐ -
worin G₂ fehlt oder für C₁-C₃ Alkandiyl steht, Xₐ fehlt oder für O, S, SO, SO₂, NR_{L}, CO, OCO, COO, CONR_{L}, NR_{L}CO, SO₂NR_{L} oder NR_{L}SO₂ steht, Rₖ für eine carbocyclische oder heterocyclische Gruppe steht, die optional mit R₃ substituiert ist und R_{L} für Wasserstoff oder C₁-C₆ Alkyl steht,
R_{3z} für Oxo steht oder wie für R_{3y} definiert ist, X_{za} für CH₂ steht und X_{z} für O, S oder NR_{z} steht, worin R_{z} eine Bedeutung hat, die unabhängig aus einer Bedeutung für R_{3y} ausgewählt ist,
oder ein physiologisch tolerierbares Salz hiervon.

2. Verbindung nach Anspruch 1, worin R₃ₐ jeweils unabhängig ausgewählt ist aus Wasserstoff, Hydroxyl, C₁-C₆ Alkoxy, C₁-C₆ Alkyl, C₂-C₆ Alkenyl, C₂-C₆ Alkinyl, C₁-C₆ Alkanoyl, C₁-C₆ Alkylaminoalkyl, Hydroxy-C₁-C₆-alkyl, Carboxy, C₁-C₆ Alkoxyalkyl, C₁-C₆ Alkoxycarbonyl, C₁-C₆ Alkylaminocarbonyl, Amino-C₁-C₆-alkyl, CONH₂, CH₂CONH₂, Aminoacetyl, C₁-C₆ Alkanoylamino, C₁-C₆ Alkoxycarbonylamino, Amino, Halogen, Cyano, Nitro, Thiol, C₁-C₆ Alkylthio, C₁-C₆ Alkylsulfonyl, C₁-C₆ Alkylsulfenyl, Imidazolyl, Hydrazido, C₁-C₆ Alkylimidazolyl, C₁-C₆ Alkylsulfonamido, C₁-C₆ Alkylaminosulfonyl, Aminosulfonyl, C₁-C₆ Halogenalkoxy oder C₁-C₆ Halogenalkyl,
Xᵢ für eine Bindung, O, NH oder CH₂ steht und R₃ᵢ für Phenyl steht, das wahlweise durch R₃ₐ substituiert ist, und
Lp ausgewählt ist aus 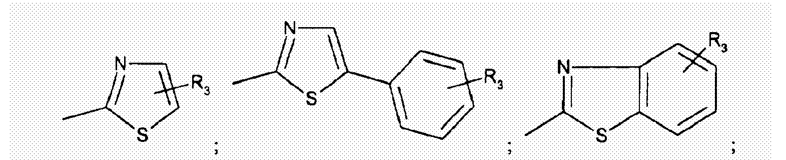 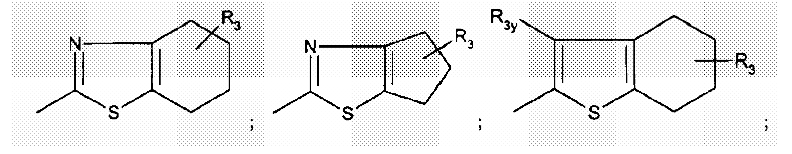 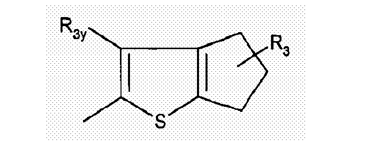
R₃ steht für einen Aminosäurerest, N-C₁-C₆ Alkylaminocarbonyl, N,N-Di-C₁-C₆-alkylaminocarbonyl, N-C₁-C₆ Alkylaminoalkanoyl, N-C₁-C₆ Alkanoylamino-C₁-C₆-alkanoyl, C-Hydroxyamino-C₁-C₆-alkanoyl, Hydroxy-C₂-C₆-alkanoylamino-C₁-C₆-alkanoyl, Di-C₁-C₆-alkylaminosulfonyl, Wasserstoff, Hydroxyl, C₁-C₆ Alkoxy, C₁-C₆ Alkanoyloxy, C₁-C₆ Alkyl, C₂-C₆ Alkenyl, C₂-C₆ Alkinyl, C₃-C₆ Alkenyloxycarbonyl, C₁-C₆ Alkanoyl, Amino-C₁-C₆-alkyl, Amido (CONH₂), Amino-C₁-C₆-alkanoyl, Aminocarbonyl-C₁-C₅-alkanoyl, Hydroxy-C₁-C₆-alkyl, Carboxy, Hydroxy-C₁-C₆-alkanoyl, C₁-C₆ Alkoxy-C₁-C₆-alkyl, C₁-C₆ Alkoxycarbonyl-C₁-C₅-alkyl, C₁-C₆ Alkoxycarbonyl, C₁-C₆ Alkanoylamino, Amino, Halogen, Cyano, Nitro, Thiol, C₁-C₆ Alkylthio, C₁-C₆ Alkylsulfonyl, C₁-C₆ Alkylsulfenyl oder Hydrazido, und
R_{3y} für R₃ oder eine Gruppe der folgenden Formel steht
Rₖ-G₂-Xₐ-
worin G₂ für eine Bindung oder C₁-C₃ Alkandiyl steht, Xₐ für eine Bindung, CO, OCO, COO, NHCO steht und Rₖ für eine carbocyclische oder heterocyclische Gruppe steht, die optional mit R₃ substituiert ist.

3. Verbindung nach Anspruch 1 oder Anspruch 2, worin R₅ für Amino oder Wasserstoff steht.

4. Verbindung nach Anspruch 3, worin R₅ für Wasserstoff steht.

5. Verbindung nach Anspruch 4, worin R₆ₐ für Wasserstoff steht.

6. Verbindung nach einem der Ansprüche 1 bis 5, worin X-X für CONH steht.

7. Verbindung nach einem der Ansprüche 1 bis 6, worin das alpha-Kohlenstoffatom (*) die Konformation aufweist, die aus der Konstruktion aus einer D-α-Aminosäure NH₂-CH(Cy)-COOH resultieren würde, worin NH₂ ein Teil von X-X ist.

8. Verbindung nach einem der Ansprüche 1 bis 7, worin Cy steht für eine Gruppe aus Cycloalkyl, Piperidinyl, Phenyl, 3,4-Methylendioxyphenyl, Furyl, Thienyl, Imidazolyl, Oxazolyl, Thiazolyl, Pyridyl, Pyrimidinyl, Pyrazinyl, Naphthyl, Indolyl, Indanyl, 3,4-Dihydrobenzofuryl, Benzofuryl oder Benzo[b]thienyl, die optional substituiert ist durch R₃ₐ oder R₃ᵢXᵢ.

9. Verbindung nach Anspruch 8, worin Cy steht für Cyclohexyl, Piperidin-4-yl, Phenyl, 3,4-Methylendioxyphenyl, Fur-2-yl, Thien-2-yl, Thien-3-yl, Imidazol-4-yl, Oxazol-4-yl, Oxazol-5-yl, Thiazol-4-yl, Thiazol-5-yl, Pyrid-2-yl, Pyrid-3-yl, Pyrid-4-yl, Pyrimidin-2-yl, Pyrimidin-4-yl, Pyrimidin-5-yl, Pyrazin-2-yl, Pyrazin-3-yl, Naphth-1-yl, Naphth-2-yl, Indol-5-yl, Indan-5-yl, 3,4-Dihydrobenzofur-5-yl, Benzofur-2-yl oder Benzo[b]-thien-2-yl, das optional substituiert ist durch R₃ₐ oder R₃ᵢXᵢ.

10. Verbindung nach Anspruch 8 oder Anspruch 9, worin R₃ₐ steht für Wasserstoff, Hydroxyl, Methoxy, Ethoxy, Isopropoxy, Methyl, Ethyl, Isopropyl, Acetyl, Propanoyl, Isopropanoyl, Methylaminomethyl, Dimethylaminomethyl, Hydroxymethyl, Carboxy, Methoxymethyl, Methoxycarbonyl, Ethoxycarbonyl, Methylaminocarbonyl, Dimethylaminocarbonyl, Aminomethyl, CONH₂, CH₂CONH₂, Aminoacetyl, Formylamino, Acetylamino, Hydroxyacetylamino, Dimethylaminoacetylamino, Methoxycarbonylamino, Ethoxycarbonylamino, t-Butoxycarbonylamino, Amino, Fluor, Chlor, Brom, Cyano, Nitro, Thiol, Methylthio, Methylsulfonyl, Ethylsulfonyl, Methylsulfenyl, Imidazol-4-yl, Hydrazido, 2-Methylimidazol-4-yl, Methylsulfonylamido, Ethylsulfonylamido, Methylaminosulfonyl, Ethylaminosulfonyl, Aminosulfonyl, Trifluormethoxy oder Trifluormethyl und R₃ᵢXᵢ für Phenyl, Phenoxy, Phenylamino oder Benzyl steht.

11. Verbindung nach Anspruch 10, worin Cy ausgewählt ist aus Cyclohexyl, Piperidin-4-yl,1-Acetylpiperidin-4-yl, 1-Propanoylpiperidin-4-yl, 1-Isobutyrylpiperidin-4-yl, 1-Aminoacetylpiperidin-4-yl, Phenyl, 2-Aminophenyl, 4-Aminophenyl, 3-Hydroxyphenyl, 4-Methylphenyl, 2,4-Dimethylphenyl, 3,6-Dimethylphenyl, 4-Ethylphenyl, 4-Isopropylphenyl, 4-Hydroxyphenyl, 3-Aminomethylphenyl, 4-Aminomethylphenyl, 4-(H₂NCO)Phenyl, 4-Hydroxymethylphenyl, 3-Hydroxymethylphenyl, 2-Hydroxymethylphenyl, 4-Carboxyphenyl, 4-Isopropoxyphenyl, 2-Chlorphenyl, 3,4-Methylendioxyphenyl, 4-Phenylphenyl, 4-Phenoxyphenyl, 5-Methylfur-2-yl, Imidazol-4-yl, 2-Methylthiazol-4-yl, 2-Aminothiazol-4-yl, 2-Formylaminothiazol-4-yl, 2-Aminothiazol-5-yl, 2-Formylaminothiazol-5-yl, 2-Phenylthiazol-4-yl, 4-Aminopyrid-3-yl, 6-Methylpyrid-2-yl, 3-Aminopyrid-4-yl, Naphth-1-yl, Naphth-2-yl, Benzofur-2-yl, 2-Methoxyphenyl, 3-Methoxyphenyl, 4-Methoxyphenyl, Indan-5-yl, 2-Methylthiophenyl, 3-Methylthiophenyl, 3-Methylsulfinylphenyl, 2-Methylsulfonylphenyl, 3-Methylsulfonylphenyl, 3-N,N-Dimethylaminophenyl, 2,3-Dihydrobenzofuran-5-yl, 3-Bromphenyl, 3-Cyanophenyl, 2-Methoxycarbonylphenyl, 2-Ethoxycarbonylphenyl, 2-Methylphenyl, 2-Fluorphenyl, 6-Aminopyrid-3-yl, 2-Ethylthiazol-4-yl, 2-Benzylthiazol-4-yl, 2-Methylsulfonamidothiazol-4-yl, 2-Chlorpyrid-3-yl, 2-Hydroxyacetylaminothiazol-4-yl, 2-N,N-Dimethylamino-acetylaminothiazol-4-yl, 2-Trifluormethoxyphenyl, 2-Trifluormethylphenyl, 3-Chlorpyrid-2-yl, 3-Methylpyrid-2-yl, Pyrazin-2-yl, Pyrazin-3-yl, Pyrimidin-2-yl und Pyrimidin-3-yl.

12. Verbindung nach einem der Ansprüche 1 bis 11, worin Cy für eine Gruppe der folgenden Formel steht 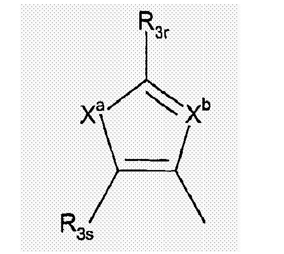 worin eines von X^{a} und X^{b} für N steht und das andere für NH oder S steht und R₃ᵣ und R₃ₛ jeweils wie für R₃ₐ definiert sind.

13. Verbindung nach Anspruch 12, worin X^{a} für S steht und X^{b} für N steht.

14. Verbindung nach Anspruch 12 oder Anspruch 13, worin R₃ₛ für Wasserstoff steht und R₃ᵣ für Wasserstoff, C₁-C₆ Alkyl, Amino, C₁-C₆ Alkanoylamino, Hydroxy-C₁-C₆-alkanoylamino, N,N-Di-C₁-C₆ Alkylaminoalkanoylamino, C₁-C₆ Alkylsulfonylamino, Phenyl oder Benzyl steht.

15. Verbindung nach einem der Ansprüche 1 bis 14, worin Cy für Pyrid-2-yl, Pyrimidin-2-yl, Pyrimidin-4-yl, Pyrazin-2-yl, Pyrazin-3-yl oder Oxazol-4-yl steht, das wahlweise durch R₃ₐ oder R₃ᵢXᵢ substituiert ist.

16. Verbindung nach einem der Ansprüche 2 bis 15, worin Cy für eine Gruppe aus Cycloalkyl, Piperidinyl, Phenyl, 3,4-Methylendioxyphenyl, Furyl, Thienyl, Imidazolyl, Thiazolyl, Pyridyl, Naphthyl, Benzofuryl oder Benzo[b]thienyl steht, die optional substituiert ist durch R₃ₐ oder R₃ᵢXᵢ, worin Xᵢ für eine Bindung, O, NH oder CH₂ steht and R₃ᵢ für Phenyl steht, das optional durch R₃ₐ substituiert ist, und R₃ₐ jeweils unabhängig steht für Wasserstoff, Hydroxyl, C₁-C₆ Alkoxy, C₁-C₆ Alkyl, C₁-C₆ Alkanoyl, C₁-C₆ Alkylaminoalkyl, Hydroxy-C₁-C₆-alkyl, Carboxy, C₁-C₆ Alkoxyalkyl, C₁-C₆ Alkoxycarbonyl, C₁-C₆ Alkylaminocarbonyl, Amino-C₁-C₆-alkyl, CONH₂, CH₂CONH₂, Aminoacetyl, C₁-C₆ Alkanoylamino, C₁-C₆ Alkoxycarbonylamino, Amino, Halogen, Cyano, Nitro, Thiol, C₁-C₆ Alkylthio, C₁-C₆ Alkylsulfonyl, C₁-C₆ Alkylsulfenyl, Imidazolyl, Hydrazido, C₁-C₆ Alkylimidazolyl, C₁-C₆ Alkylsulfonamido, C₁-C₆ Alkylaminosulfonyl, Aminosulfonyl, C₁-C₆ Halogenalkoxy oder C₁-C₆ Halogenalkyl.

17. Verbindung nach Anspruch 16, worin Cy steht für eine Gruppe aus Cyclohexyl, Piperidin-4-yl, Phenyl, 3,4-Methylendioxyphenyl, Fur-2-yl, Thien-2-yl, Thien-3-yl, Imidazol-4-yl, Thiazol-4-yl, Thiazol-5-yl, Pyrid-2-yl, Pyrid-3-yl, Pyrid-4-yl, Naphth-1-yl, Naphth-2-yl, Benzofur-2-yl oder Benzo[b]thien-2-yl, die optional substituiert ist durch R₃ₐ oder R₃ᵢXᵢ.

18. Verbindung nach Anspruch 16 oder Anspruch 17, worin R₃ₐ steht für Wasserstoff, Hydroxyl, Methoxy, Ethoxy, Isopropoxy, Methyl, Ethyl, Isopropyl, Acetyl, Propanoyl, Isopropanoyl, Methylaminomethyl, Dimethylaminomethyl, Hydroxymethyl, Carboxy, Methoxymethyl, Methoxycarbonyl, Ethoxycarbonyl, Methylaminocarbonyl, Dimethylaminocarbonyl, Aminomethyl, CONH₂, CH₂CONH₂, Aminoacetyl, Formylamino, Acetylamino, Methoxycarbonylamino, Ethoxycarbonylamino, t-Butoxycarbonylamino, Amino, Fluor, Chlor, Cyano, Nitro, Thiol, Methylthio, Methylsulfonyl, Ethylsulfonyl, Methylsulfenyl, Imidazol-4-yl, Hydrazido, 2-Methylimidazol-4-yl, Methylsulfonylamido, Ethylsulfonylamido, Methylaminosulfonyl, Ethylaminosulfonyl, Aminosulfonyl, Trifluormethoxy oder Trifluormethyl und R₃ᵢXᵢ für Phenyl, Phenoxy, Phenylamino oder Benzyl steht.

19. Verbindung nach Anspruch 18, worin Cy steht für Cyclohexyl, Piperidin-4-yl, 1-Acetylpiperidin-4-yl, 1-Propanoylpiperidin-4-yl, 1-Isobutyrylpiperidin-4-yl, 1-Aminoacetylpiperidin-4-yl, Phenyl, 4-Aminophenyl, 3-Hydroxyphenyl, 4-Methylphenyl, 2,4-Dimethylphenyl, 3,6-Dimethylphenyl, 4-Ethylphenyl, 4-Isopropylphenyl, 4-Hydroxyphenyl, 3-Aminomethylphenyl, 4-Aminomethylphenyl, 4-(H₂NCO)Phenyl, 4-Hydroxymethylphenyl, 3-Hydroxymethylphenyl, 2-Hydroxymethylphenyl, 4-Carboxyphenyl, 4-Isopropoxyphenyl, 2-Chlorphenyl, 3,4-Methylendioxyphenyl, 4-Phenylphenyl, 4-Phenoxyphenyl, 5-Methylfur-2-yl, Imidazol-4-yl, 2-Methylthiazol-4-yl, 2-Aminothiazol-4-yl, 2-Formylaminothiazol-4-yl, 2-Aminothiazol-5-yl, 2-Formylaminothiazol-5-yl, 2-Phenylthiazol-4-yl, 4-Aminopyrid-3-yl, 6-Methylpyrid-2-yl, 3-Aminopyrid-4-yl, Naphth-1-yl, Naphth-2-yl, Benzofur-2-yl oder 3-Methylbenzothien-2-yl.

20. Verbindung nach einem der Ansprüche 1 bis 19, worin L für CO, CONH, CONCH₃ oder CONHCH₂ steht.

21. Verbindung nach Anspruch 20, worin L für CONH oder CONCH₃ steht.

22. Verbindung nach einem der Ansprüche 1 bis 21, worin R₃ ausgewählt ist aus N-Acetylalaninoyl, Serinoyl, Threoninoyl, Aspartoyl, Glutamoyl, N,N-Dimethylaminocarbonyl, N,N-Diethylaminocarbonyl, N-(1,3-Dimethyl)butylaminocarbonyl, N-Methyl-N-ethylaminocarbonyl, N-Methylacetyl, 2-N-Acetylaminoacetyl, 2-N-Acetylaminopropanoyl, 2-N-(2-Methylpropanoyl)aminoacetyl, 2-Amino-3-hydroxypropanoyl, 2-Amino-3-hydroxybutanoyl, 2-Hydroxyacetylaminoacetyl, Dimethylaminosulfonyl, Wasserstoff, Hydroxyl, Methoxy, Acetoxy, Methyl, Ethyl, Propyl, 2-Propyl, 2,2-Dimethylethyl, Allyl, Propinyl, Allyloxycarbonyl, Acetyl, Propionyl, Isobutyryl, Aminomethyl, CONH₂, Aminoacetyl, Aminopropionyl, 2-Aminopropionyl, Aminocarbonylacetyl, Hydroxymethyl, 1-Hydroxyethyl, Carboxy, 2-Hydroxyacetyl, 2-Hydroxypropanoyl, Methoxyacetyl, Methoxymethyl, Methoxycarbonylmethyl, Methoxycarbonyl, Ethoxycarbonyl, Formylamino, Acetylamino, Amino, Chlor, Cyano, Nitro, Thiol, Methylthio, Methylsulfonyl, Ethylsulfonyl, Methylsulfenyl und Hydrazido.

23. Verbindung nach einem der Ansprüche 2 bis 22, worin R₃ ausgewählt ist aus N-Acetylalaninoyl, Serinoyl, Threoninoyl, Aspartoyl, Glutamoyl, N-(1,3-Dimethyl)butylaminocarbonyl, N-Methyl-N-ethylaminocarbonyl, N-Methylacetyl, 2-N-Acetylaminoacetyl, 2-N-Acetylaminopropanoyl, 2-N-(2-Methylpropanoyl)-aminoacetyl, 2-Amino-3-hydroxypropanoyl, 2-Amino-3-hydroxybutanoyl, 2-Hydroxyacetylaminoacetyl, Dimethylaminosulfonyl, Wasserstoff, Hydroxyl, Methoxy, Acetoxy, Methyl, Ethyl, Propyl, 2-Propyl, 2,2-Dimethylethyl, Allyl, Propinyl, Allyloxycarbonyl, Acetyl, Propionyl, Isobutyryl, Aminomethyl, CONH₂, Aminoacetyl, Aminopropionyl, 2-Aminopropionyl, Aminocarbonylacetyl, Hydroxymethyl, 1-Hydroxyethyl, Carboxy, 2-Hydroxyacetyl, 2-Hydroxypropanoyl, Methoxymethyl, Methoxycarbonylmethyl, Methoxycarbonyl, Ethoxycarbonyl, Formylamino, Acetylamino, Amino, Chlor, Cyano, Nitro, Thiol, Methylthio, Methylsulfonyl, Ethylsulfonyl, Methylsulfenyl und Hydrazido.

24. Verbindung nach einem der Ansprüche 1 bis 23, worin L für CONR_{1d} steht und Lp steht für

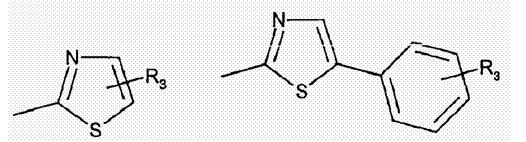

25. Verbindung nach Anspruch 24, worin R₃ jeweils unabhängig ausgewählt ist aus Wasserstoff, Amino, Hydroxy, C₁-C₆ Alkyl, C₁-C₆ Alkanoyl, C₁-C₆ Alkanoyloxy, C₁-C₅ Alkoxycarbonyl-C₁-C₆-alkyl, Amino-C₁-C₆-alkyl und Cyano.

26. Verbindung nach einem der Ansprüche 1 bis 24, worin L für CONR_{1d} steht und Lp steht für 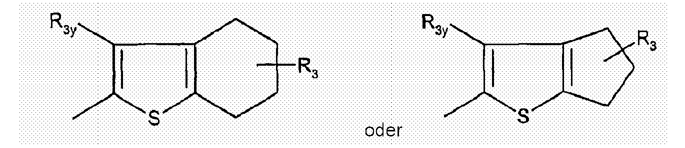 worin R_{3y} für R₃ oder eine Gruppe der folgenden Formel steht
Rₖ-G₂-Xₐ-
worin G₂ für eine Bindung oder C₁-C₃ Alkandiyl steht, Xₐ für eine Bindung, CO, OCO, COO oder NHCO steht und Rₖ für eine carbocyclische oder heterocyclische Gruppe steht, die optional mit R₃ substituiert ist.

27. Verbindung nach Anspruch 26, worin Lp ausgewählt ist aus 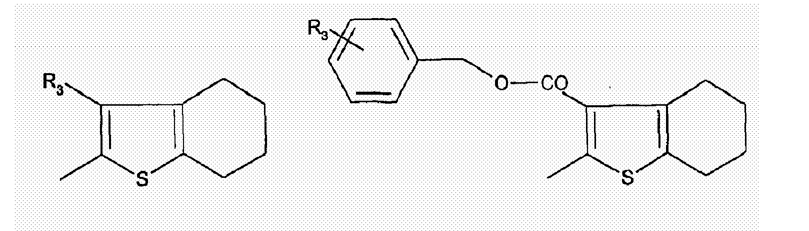 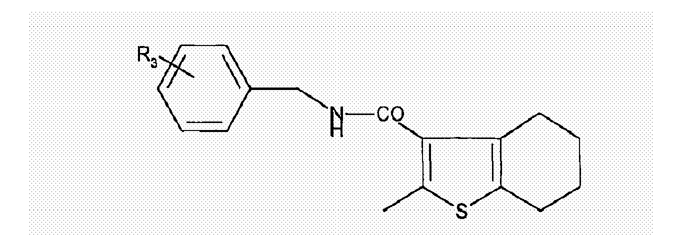 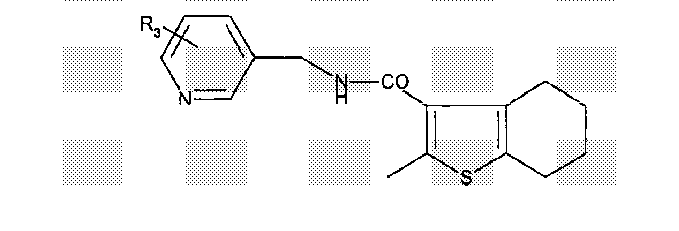 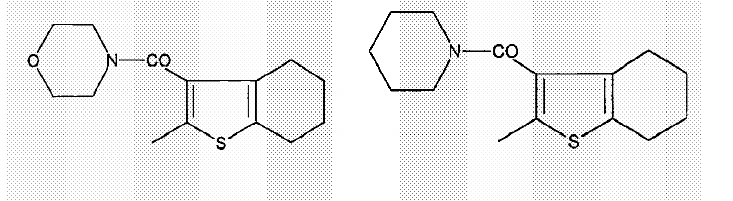 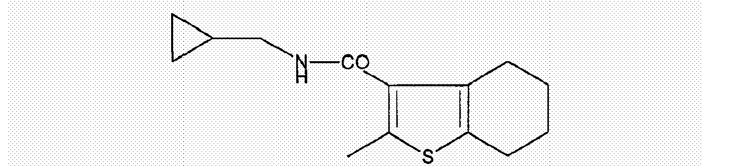 worin (i) wenn R₃ als Substituent an der Position 3 einer 4,5,6,7-Tetrahydrobenzothiophengruppe vorkommt, dann für eine Carboxygruppe, eine C₁-C₆ Alkoxycarbonylgruppe oder eine C₁-C₆ Alkylaminocarbonylgruppe steht und (ii) wenn R₃ als Substituent an einer Phenyl- oder Pyridylgruppe vorkommt, dann für ein Wasserstoffatom steht.

28. Verbindung nach einem der Ansprüche 1 bis 24, worin L für CONR_{1d} steht und Lp steht für

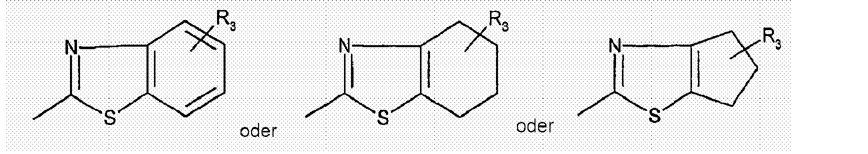

29. Verbindung nach Anspruch 28, worin die heterocyclische Gruppe durch eine oder zwei R₃ Gruppen substituiert ist.

30. Verbindung nach Anspruch 29, worin die R₃ Gruppe jeweils ausgewählt ist aus Wasserstoff, Halogen, C₁-C₆ Alkyl und C₁-C₆ Alkoxy.

31. Verbindung nach einem der Ansprüche 1 bis 24, worin L für CONR_{1d} steht und Lp steht für 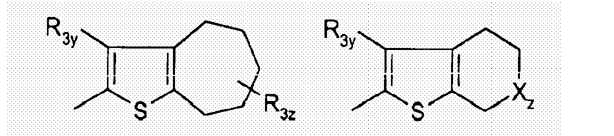 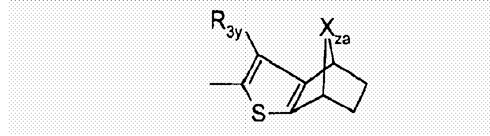 worin R_{3y}, R_{3z}, X_{z} und X_{za} wie oben definiert sind.

32. Verbindung nach Anspruch 31, worin R_{3y} für C₁-C₆ Alkoxycarbonyl, N,N-Dialkylaminocarbonyl oder Cyano steht, R_{3z} für Wasserstoff steht, X_{z} für O, S oder NR_{z} steht, R_{z} für Wasserstoff, C₁-C₆ Alkanoyl, Amino-C₁-C₆-alkanoyl, C₁-C₆ Alkoxy-C₁-C₆-alkanoyl oder Benzyloxycarbonyl steht und X_{za} für CH₂ steht.

33. Verbindung nach Anspruch 32, worin Lp steht für 3-Ethoxycarbonyltetrahydro-4H-cyclohepta(b)thien-2-yl, 3-Ethoxycarbonyl-4,5-dihydo-5H-thieno[2,3-c]pyranyl, 3-Ethoxycarbonyl-4,5-dihydro-5H-thieno-[2,3-c]thiopyranyl, 3-Dimethylaminocarbonyl-6-benzyloxycarbonyltetrahydrothieno[2,3-b]pyridin-2-yl, 3-Dimethylaminocarbonyltetrahydrothieno[2,3-b]pyridin-2-yl, 3-Dimethylaminocarbonyl-6-acetyltetrahydrothieno[2,3-b]pyridin-2-yl, 3-Dimethylaminocarbonyl-6-aminoacetyltetrahydrothieno[2,3-b]pyridin-2-yl, 3-Dimethylaminocarbonyl-6-methoxyacetyltetrahydrothieno[2,3-b]pyridin-2-yl oder 3-Ethoxycarbonyltetrahydro-4,7-methanobenzo[b]thiophen-2-yl.

34. Verbindung nach einem der Ansprüche 1 bis 33, worin L für CONH steht und Lp steht für 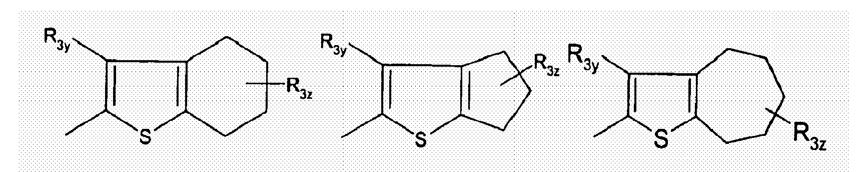 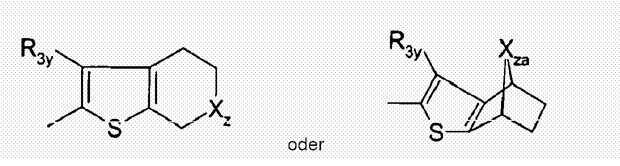 worin R_{3y} ausgewählt ist aus N,N-Di-C₁-C₆ Alkylaminocarbonyl, Di-C₁-C₆ Alkylaminosulfonyl, C₃-C₆ Alkenyloxycarbonyl, C₁-C₆ Alkanoyl, Hydroxy-C₁-C₆-alkanoyl, C₁-C₆ Alkoxy-C₁-C₆-alkanoyl, C₁-C₆ Alkoxycarbonyl, C₁-C₆ Alkylsulfonyl, C₁-C₆ Alkylsulfenyl und Rₖ - G₂ - Xₐ, worin entweder Xₐ für CO, OCO, NR_{L}CO (worin R_{L} für C₁-C₆ Alkyl steht), SO₂ oder NR_{L}SO₂ steht und R_{K} und G₂ wie vorher definiert sind oder Xₐ und G₂ beide fehlen und Rₖ für Pyrid-2-yl, Thiazol-2-yl, Thiazol-4-yl, Pyrazin-2-yl, Pyrazin-3-yl, Pyrimidin-2-yl oder Pyrimidin-4-yl steht.

35. Pharmazeutische Zusammensetzung, die eine Verbindung nach einem der Ansprüche 1 bis 34 zusammen mit zumindest einem pharmazeutisch annehmbaren Träger oder Hilfsstoff enthält.

36. Verbindung nach einem der Ansprüche 1 bis 34 zur Verwendung in der Therapie.

37. Verwendung einer Verbindung nach einem der Ansprüche 1 bis 34 zur Herstellung eines Arzneimittels zur Verwendung bei der Behandlung des menschlichen oder tierischen Körpers, um einen Zustand zu bekämpfen, der auf einen Tryptaseinhibitor anspricht.

38. Verwendung nach Anspruch 37, worin der Zustand ausgewählt ist aus Asthma, allergischer Rhinitis, Ekzem, Psoriasis, atoper Dermatitis, Urticaria, rheumatoider Arthritis, Konjunktivitis, entzündlicher Darmerkrankung, neurogener Entzündung, Artherosklerose und Krebs.

39. Verwendung nach Anspruch 38, worin der Zustand Asthma ist.

40. Verbindung der Formel (IX)
Z₂ - CH(Cy) - L - Lp (IX)
worin Cy, L und Lp wie in einem der Ansprüche 1 bis 34 definiert sind und Z₂ für NH₂ steht oder ein Salz hiervon, aber ausschließlich der Verbindungen Phenylglycin-4-methylthiazol-2-amid und Phenylglycin-5-methylthiazol-2-ylmethylamid.

## Revendications

1. Composé inhibiteur de la tryptase de formule (I) 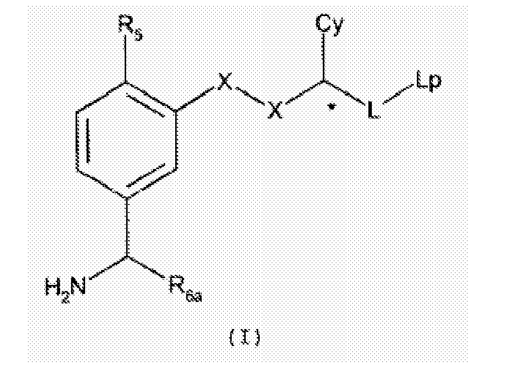 où :
R₅ représente un groupe amino, hydroxy, aminométhyle, hydroxyméthyle ou un atome d'hydrogène ;
R₆ₐ représente un atome d'hydrogène ou un groupe méthyle ;
X-X représente -CONR₁ₐ- ;
R₁ₐ représente un atome d'hydrogène, un groupe (C₁-C₆)alkyle ou phényl(C₁-C₆)alkyle;
L représente CONR_{1d}(CH₂)ₘ dans lequel m prend la valeur de 0 ou 1 et
R_{1d} représente un atome d'hydrogène, un groupe (C₁-C₆)alkyle ou phényl(C₁-C₆)alkyle ;
Cy représente un groupe saturé ou insaturé, mono ou polycyclique, homo ou hétérocyclique, contenant de préférence 5 à 10 atomes cycliques et éventuellement substitué par un ou plusieurs groupe(s) R₃ₐ- ou R₃ᵢXᵢ- ;
chaque R₃ₐ représente indépendamment un atome d'hydrogène, un groupe hydroxyle, (C₁-C₆)alcoxy, (C₁-C₆)alkyle, (C₂-C₆)alcényle, (C₂-C₆)alcynyle, (C₁-C₆)alcanoyle, (C₁-C₆)alkylaminoalkyle, hydroxy(C₁-C₆)alkyle, carboxy, (C₁-C₆)alcoxyalkyle, (C₁-C₆)alcoxycarbonyle, (Ci-C₆)alkylaminocarbonyle, amino(C₁-C₆)alkyle, CONH₂, CH₂CONH₂, aminoacétyle, (C₁-C₆)alcanoylamino, hydroxy(C₁-C₆)alcanoylamino, amino(C₁-C₆)alcanoylamino, (C₁-C₆)alkylamino(C₁-C₆)alcanoylamino, di(C₁-C₆)alkylamino (C₁-C₆)alcanoylamino, (C₁-C₆)alcoxycarbonylamino, amino, halogéno, cyano, nitro, thiol, (C₁-C₆)alkylthio, (C₁-C₆)alkylsulfonyle, (C₁-C₆)alkylsulfényle, imidazolyle, hydrazido, (C₁-C₆)alkylimidazolyle , (C₁-C₆)alkylsulfonamido, (C₁-C₆)alkylaminosulfonyle, aminosulfonyle, (C₁-C₆)halogénoalcoxy, ou (Ci-C₆)halogénoalkyle ;
Xᵢ représente une liaison, O, NR₁ₚ, CH₂, CO, CONR₁ₚ, NR₁ₚCO, SO₂, NR₁ₚSO₂ ou SO₂NR₁ₚ ;
R₃ᵢ représente un groupe phényle ou pyridyle ;
R₁ₚ est tel que défini pour R₁ₐ ;
Lp est choisi parmi 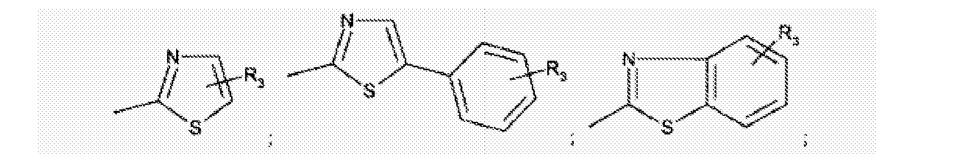 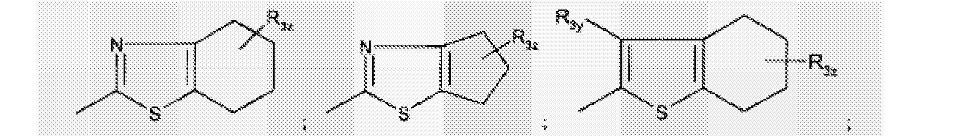 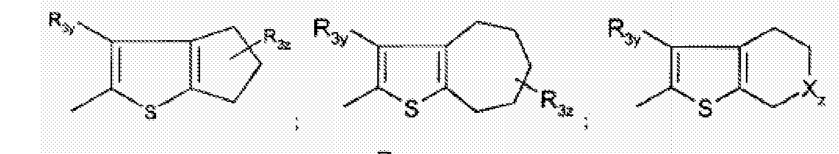 et 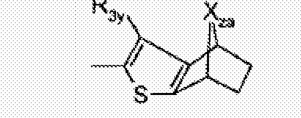 dans lesquelles R₃ représente un résidu d'acide aminé, un groupe N-(C₁-C₆)alkylaminocarbonyle, N,N-di(C₁-C₆)alkylaminocarbonyle, N-(C₁-C₆)alkylaminoalcanoyle, N-(C₁-C₆)alcanoylamino(C₁-C₆)alcanonyle, C-hydroxyamino(C₁-C₆)alcanoyle, hydroxy(C₂-C₆)alcanoylamino(C₁-C₆)alcanoyle, di(C₁-C₆)alkylaminosulfonyle, un atome d'hydrogène, un groupe hydroxyle, (Ci-C₆)alcoxy, (C₁-C₆)alcanoyloxy, (C₁-C₆)alkyle, (C₂-C₆)alcényle, (C₂-C₆)alcynyle, (C₃-C₆)alcényloxycarbonyle, (C₁-C₆)alcanoyle, amino(C₁-C₆)alkyle, amido (CONH₂), amino(C₁-C₆)alcanoyle, aminocarbonyl(C₁-C₅)alcanoyle, hydroxy(C₁-C₆)alkyle, carboxy, hydroxy(C₁-C₆)alcanoyle, (C₁-C₆)alcoxy(C₁-C₆)alcanoyle, (C₁-C₆)alcoxy(C₁-C₆)alkyle, (C₁-C₆)alcoxycarbonyl(C₁-C₅)alkyle, (C₁-C₆)alcoxycarbonyle, (C₁-C₆)alcanoylamino, amino, halogéno, cyano, nitro, thiol, (C₁-C₆)alkylthio, (C₁-C₆)alkylsulfonyle, (C₁-C₆)alkylsulfényle ou hydrazido ;
R₃y représente R₃ ou un groupe de formule
Rₖ-G₂-Xₐ-
dans laquelle G₂ est absent ou représente un groupe (C₁-C₃)alcanediyle, Xₐ est absent ou représente O, S, SO, SO₂, NR_{L}, CO, OCO, COO, CONR_{L}, NR_{L}CO, SO₂NR_{L} ou NR_{L}SO₂; Rₖ représente un groupe carbocyclique ou hétérocyclique, éventuellement substitué par R₃ ; et R_{L} représente un atome d'hydrogène ou un groupe (C₁-C₆)alkyle ;
R_{3z} représente un groupe oxo ou est tel que défini pour R_{3y}, X_{za} représente CH₂ et X_{z} représente O, S ou NR_{z} dans laquelle R_{z} a une valeur indépendamment choisie à partir d'une valeur pour R_{3y} ;
ou un sel physiologiquement tolérable de celui-ci.

2. Composé tel que revendiqué dans la revendication 1, dans lequel :
chaque R₃ₐ représente indépendamment un atome d'hydrogène, un groupe hydroxyle, (C₁-C₆)alcoxy, (C₁-C₆)alkyle, (C₂-C₆)alcényle, (C₂-C₆)alcynyle, (C₁-C₆)alcanoyle, (C₁-C₆)alkylaminoalkyle, hydroxy(C₁-C₆)alkyle, carboxy, (C₁-C₆)alcoxyalkyle, (C₁-C₆)alcoxycarbonyle, (Ci-C₆)alkylaminocarbonyle, amino(C₁-C₆)alkyle, CONH₂, CH₂CONH₂, aminoacétyle, (C₁-C₆)alcanoylamino, (C₁-C₆)alcoxycarbonylamino, amino, halogéno, cyano, nitro, thiol, (C₁-C₆)alkylthio, (C₁-C₆)alkylsulfonyle, (C₁-C₆)alkylsulfényle, imidazolyle, hydrazido, (C₁-C₆)alkylimidazolyle, (C₁-C₆)alkylsulfonamido, (C₁-C₆)alkylaminosulfonyle, aminosulfonyle, (Ci-C₆)halogénoalcoxy, ou (C₁-C₆)halogénoalkyle ;
Xᵢ représente une liaison, O, NH ou CH₂ ; et R₃ᵢ représente un groupe phényle éventuellement substitué par R₃ₐ ; et
Lp est choisi parmi 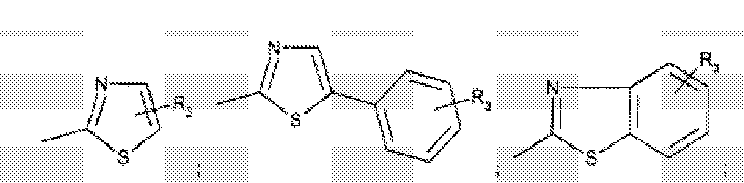 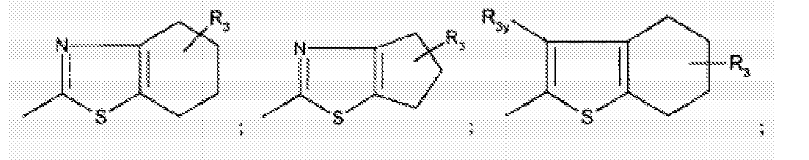 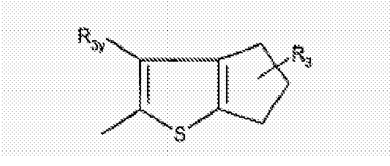
R₃ représente un résidu d'acide aminé, un groupe N-(C₁-C₆)alkylaminocarbonyle, N,N-di(C₁-C₆)alkylaminocarbonyle, N-(C₁-C₆)alkylaminoalcanoyle, N-(C₁-C₆)alcanoylamino(C₁-C₆)alcanonyle, C-hydroxyamino(C₁-C₆)alcanoyle, hydroxy(C₂-C₆)alcanoylamino(C₁-C₆)alcanoyle, di(C₁-C₆)alkylaminosulfonyle, un atome d'hydrogène, un groupe hydroxyle, (C₁-C₆)alcoxy, (C₁-C₆)alcanoyloxy, (C₁-C₆)alkyle, (C₂-C₆)alcényle, (C₂-C₆)alcynyle, (C₃-C₆)alcényloxycarbonyle, (C₁-C₆)alcanoyle, amino(C₁-C₆)alkyle, amido (CONH₂), amino(C₁-C₆)alcanoyle, aminocarbonyl(C₁-C₅)alcanoyle, hydroxy(C₁-C₆)alkyle, carboxy, hydroxy(C₁-C₆)alcanoyle, (C₁-C₆)alcoxy(C₁-C₆)alkyle, (C₁-C₆)alcoxycarbonyl(C₁-C₅)alkyle, (C₁-C₆)alcoxycarbonyle, (C₁-C₆)alcanoylamino, amino, halogéno, cyano, nitro, thiol, (C₁-C₆)alkylthio, (C₁-C₆)alkylsulfonyle, (Ci-C₆)alkylsulfényle ou hydrazido ; et
R₃y représente R₃ ou un groupe de formule
Rₖ-G₂-Xₐ-
dans laquelle G₂ représente une liaison ou (C₁-C₃)alcanediyle, Xₐ représente une liaison, CO, OCO, COO ou NHCO, et Rₖ représente un groupe carbocyclique ou hétérocyclique, éventuellement substitué par R₃.

3. Composé tel que revendiqué dans la revendication 1 ou la revendication 2, dans lequel R₅ représente un groupe amino ou un atome d'hydrogène.

4. Composé tel que revendiqué dans la revendication 3, dans lequel R₅ représente un atome d'hydrogène.

5. Composé tel que revendiqué dans la revendication 4, dans lequel R₆ₐ représente un atome d'hydrogène.

6. Composé tel que revendiqué dans l'une quelconque des revendications 1 à 5, dans lequel X-X représente CONH.

7. Composé tel que revendiqué dans l'une quelconque des revendications 1 à 6, dans lequel l'atome de carbone en position alpha (^{*}) a la conformation qui résulterait de la construction à partir d'un D-α-acide aminé NH₂-CH(Cy)-COOH où le groupe NH₂ représente une partie de X-X.

8. Composé tel que revendiqué dans l'une quelconque des revendications 1 à 7, dans lequel Cy représente un groupe cycloalkyle, pipéridinyle, phényle, 3,4-méthylènedioxyphényle, furyle, thiényle, imidazolyle, oxazolyle, thiazolyle, pyridyle, pyrimidinyle, pyrazinyle, naphtyle, indolyle, indanyle, 3,4-dihydrobenzofuryle, benzofuryle ou benzo[b]thiényle, éventuellement substitué par R₃ₐ ou R₃ᵢXᵢ.

9. Composé tel que revendiqué dans la revendication 8, dans lequel Cy représente un groupe cyclohexyle, pipéridin-4-yle, phényle, 3,4-méthylènedioxy-phényle, fur-2-yle, thièn-2-yle, thièn-3-yle, imidazol-4-yle, oxazol-4-yle, oxazol-5-yle, thiazol-4-yle, thiazol-5-yle, pyrid-2-yle, pyrid-3-yle, pyrid-4-yle, pyrimidin-2-yle, pyrimidin-4-yle, pyrimidin-5-yle, pyrazin-2-yle, pyrazin-3-yle, napht-1-yle, napht-2-yle, indol-5-yle, indan-5-yle, 3,4-dihydrobenzofur-5-yle, benzofur-2-yle ou benzo[b]thièn-2-yle, éventuellement substitué par R₃ₐ ou R₃ᵢXᵢ.

10. Composé tel que revendiqué dans la revendication 8 ou la revendication 9, dans lequel R₃ₐ représente un atome d'hydrogène ; un groupe hydroxyle ; méthoxy ; éthoxy ; isopropoxy ; méthyle; éthyle ; isopropyle; acétyle ; propanoyle ; isopropanoyle ; méthylaminométhyle ; diméthylaminométhyle ; hydroxyméthyle ; carboxy ; méthoxyméthyle ; méthoxycarbonyle ; éthoxycarbonyle ; méthylaminocarbonyle ; diméthylaminocarbonyle ; aminométhyle ; CONH₂ ; CH₂CONH₂ ; aminoacétyle ; formylamino ; acétylamino ; hydroxyacétylamino ; diméthylaminoacétylamino ; méthoxycarbonylamino; éthoxycarbonylamino; *t*-butoxycarbonylamino ; amino; fluoro; chloro; bromo ; cyano ; nitro ; thiol; méthylthio; méthylsulfonyle; éthylsulfonyle, méthylsulfényle, imidazol-4-yle; hydrazido ; 2-méthylimidazol-4-yle ; méthylsulfonylamido; éthylsulfonylamido; méthylaminosulfonyle, éthylaminosulfonyle; aminosulfonyle; trifluorométhoxy ou trifluorométhyle; et R₃ᵢXᵢ représente un groupe phényle, phénoxy, phénylamino ou benzyle.

11. Composé tel que revendiqué dans la revendication 10, dans lequel Cy est choisi parmi un groupe cyclohexyle, pipéridin-4-yle, 1-acétylpipéridin-4-yle, 1-propanoylpipéridin-4-yle, 1-isobutyrylpipéridin-4-yle, 1-aminoacétylpipéridin-4-yle, phényle, 2-aminophényle, 4-aminophényle, 3-hydroxyphényle, 4-méthylphényle, 2,4-diméthylphényle, 3,6-diméthylphényle, 4-éthylphényle, 4-isopropylphényle, 4-hydroxyphényle, 3-aminométhylphényle, 4-aminométhylphényle, 4-(H₂NCO)phényle, 4-hydroxyméthylphényle, 3-hydroxyméthylphényle, 2-hydroxyméthylphényle, 4-carboxyphényle, 4-isopropoxyphényle, 2-chlorophényle, 3,4-méthylènedioxyphényle, 4-phénylphényle, 4-phénoxyphényle, 5-méthylfur-2-yle, imidazol-4-yle, 2-méthylthiazol-4-yle, 2-aminothiazol-4-yle, 2-formylaminothiazol-4-yle, 2-aminothiazol-5-yle, 2-formylaminothiazol-5-yle, 2-phénylthiazol-4-yle, 4-aminopyrid-3-yle, 6-méthylpyrid-2-yle, 3-aminopyrid-4-yle, napht-1-yle, napht-2-yle, benzofur-2-yle, 2-méthoxyphényle, 3-méthoxyphényle, 4-méthoxyphényle, indan-5-yle, 2-méthylthiophényle, 3-méthylthiophényle, 3-méthylsulfinylphényle, 2-méthylsulfonylphényle, 3-méthylsulfonylphényle, 3-N,N-diméthylaminophényle, 2,3-dihydrobenzofuran-5-yle, 3-bromophényle, 3-cyanophényle, 2-méthoxycarbonylphényle, 2-éthoxycarbonylphényle, 2-méthylphényle, 2-fluorophényle, 6-aminopyrid-3-yle, 2-éthylthiazol-4-yle, 2-benzylthiazol-4-yle, 2-méthylsulfonamidothiazol-4-yle, 2-chloropyrid-3-yle, 2-hydroxyacétylaminothiazol-4-yle, 2-N,N-diméthylaminoacétylaminothiazol-4-yle, 2-trifluorométhoxyphényle, 2-trifluorométhylphényle, 3-chloropyrid-2-yle, 3-méthylpyrid-2-yle, pyrazin-2-yle, pyrazin-3-yle, pyrimidin-2-yle et pyrimidin-3-yle.

12. Composé tel que revendiqué dans l'une quelconque des revendications 1 à 11, dans lequel Cy représente un groupe de formule 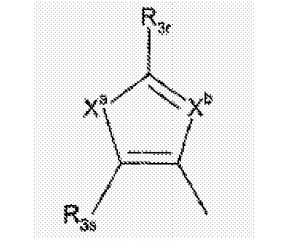 dans laquelle l'un parmi X^{a} et X^{b} représente N et l'autre représente NH ou S, et chacun de R₃ᵣ et R₃ₛ est tel que défini pour R₃ₐ.

13. Composé tel que revendiqué dans la revendication 12, dans lequel X^{a} représente S et X^{b} représente N.

14. Composé tel que revendiqué dans la revendication 12 ou la revendication 13, dans lequel R₃ₛ représente un atome d'hydrogène et R₃ᵣ représente un atome d'hydrogène, un groupe (C₁-C₆)alkyle, amino, (Ci-C₆)alcanoylamino, hydroxy(C₁-C₆)alcanoylamino, N,N-di(C₁-C₆)alkylaminoalcanoylamino, (C₁-C₆)alkylsulfonylamino, phényle ou benzyle.

15. Composé tel que revendiqué dans l'une quelconque des revendications 1 à 14, dans lequel Cy représente un groupe pyrid-2-yle, pyrimidin-2-yle, pyrimidin-4-yle, pyrazin-2-yle, pyrazin-3-yle ou oxazol-4-yle éventuellement substitué par R₃ₐ ou R₃ᵢXᵢ.

16. Composé tel que revendiqué dans l'une quelconque des revendications 2 à 15, dans lequel Cy représente un groupe cycloalkyle, pipéridinyle, phényle, 3,4-méthylènedioxyphényle, furyle, thiényle, imidazolyle, thiazolyle, pyridyle, naphtyle, benzofuryle, ou benzo[b]thiényle, éventuellement substitué par R₃ₐ ou R₃ᵢXᵢ dans lequel Xᵢ représente une liaison, O, NH ou CH₂ et R₃ᵢ représente un groupe phényle éventuellement substitué par R₃ₐ et chaque R₃ₐ représente indépendamment un atome d'hydrogène, un groupe hydroxyle, (C₁-C₆)alcoxy, (C₁-C₆)alkyle, (C₁-C₆)alcanoyle, (Ci-C₆)alkylaminoalkyle, hydroxy(C₁-C₆)alkyle, carboxy, (C₁-C₆)alcoxyalkyle, (C₁-C₆)alcoxycarbonyle, (C₁-C₆)alkylaminocarbonyle, amino(C₁-C₆)alkyle, CONH₂, CH₂CONH₂, aminoacétyle, (C₁-C₆)alcanoylamino, (C₁-C₆)alcoxycarbonylamino, amino, halogéno, cyano, nitro, thiol, (C₁-C₆)alkylthio, (C₁-C₆)alkylsulfonyle, (Ci-C₆)alkylsulfényle, imidazolyle, hydrazido, (C₁-C₆)alkylimidazolyle, (Ci-C₆)alkylsulfonamido, (C₁-C₆)alkylaminosulfonyle, aminosulfonyle, (C₁-C₆)halogénoalcoxy, ou (C₁-C₆)halogénoalkyle.

17. Composé tel que revendiqué dans la revendication 16, dans lequel Cy représente un groupe cyclohexyle, pipéridin-4-yle, phényle, 3,4-méthylènedioxyphényle, fur-2-yle, thièn-2-yle, thièn-3-yle, imidazol-4-yle, thiazol-4-yle, thiazol-5-yle, pyrid-2-yle, pyrid-3-yle, pyrid-4-yle, napht-1-yle, napht-2-yle, benzofur-2-yle, ou benzo[b]thièn-2-yle, éventuellement substitué par R₃ₐ ou R₃ᵢXᵢ.

18. Composé tel que revendiqué dans la revendication 16 ou la revendication 17, dans lequel R₃ₐ représente un atome d'hydrogène ; un groupe hydroxyle ; méthoxy ; éthoxy ; isopropoxy ; méthyle ; éthyle ; isopropyle, acétyle ; propanoyle ; isopropanoyle ; méthylaminométhyle ; diméthylaminométhyle ; hydroxyméthyle ; carboxy ; méthoxyméthyle ; méthoxycarbonyle ; éthoxycarbonyle ; méthylaminocarbonyle ; diméthylaminocarbonyle ; aminométhyle ; CONH₂ ; CH₂CONH₂ ; aminoacétyle ; formylamino ; acétylamino ; méthoxycarbonylamino ; éthoxycarbonylamino ; *t-*butoxycarbonylamino ; amino ; fluoro ; chloro ; cyano ; nitro ; thiol ; méthylthio ; méthylsulfonyle ; éthylsulfonyle ; méthylsulfényle ; imidazol-4-yle ; hydrazido ; 2-méthylimidazol-4-yle ; méthylsulfonylamido ; éthylsulfonylamido ; méthylaminosulfonyle ; éthylaminosulfonyle ; aminosulfonyle ; trifluorométhoxy ou trifluorométhyle ; et R₃ᵢXᵢ représente un groupe phényle, phénoxy, phénylamino ou benzyle.

19. Composé tel que revendiqué dans la revendication 18, dans lequel Cy représente un groupe cyclohexyle, pipéridin-4-yle, 1-acétylpipéridin-4-yle, 1-propanoylpipéridin-4-yle, 1-isobutyrylpipéridin-4-yle, 1-aminoacétylpipéridin-4-yle, phényle, 4-aminophényle, 3-hydroxyphényle, 4-méthylphényle, 2,4-diméthylphényle, 3,6-diméthylphényle, 4-éthylphényle, 4-isopropylphényle, 4-hydroxyphényle, 3-aminométhylphényle, 4-aminométhylphényle, 4-(H₂NCO)phényle, 4-hydroxyméthylphényle, 3-hydroxyméthylphényle, 2-hydroxyméthylphényle, 4-carboxyphényle, 4-isopropoxyphényle, 2-chlorophényle, 3,4-méthylènedioxyphényle, 4-phénylphényle, 4-phénoxyphényle, 5-méthylfur-2-yle, imidazol-4-yle, 2-méthylthiazol-4-yle, 2-aminothiazol-4-yle, 2-formylaminothiazol-4-yle, 2-aminothiazol-5-yle, 2-formylaminothiazol-5-yle, 2-phénylthiazol-4-yle, 4-aminopyrid-3-yle, 6-méthylpyrid-2-yle, 3-aminopyrid-4-yle, napht-1-yle, napht-2-yle, benzofur-2-yle ou 3-méthylbenzothièn-2-yle.

20. Composé tel que revendiqué dans l'une quelconque des revendications 1 à 19, dans lequel L représente CO, CONH, CONCH₃ ou CONHCH₂.

21. Composé tel que revendiqué dans la revendication 20, dans lequel L représente CONH ou CONCH₃.

22. Composé tel que revendiqué dans l'une quelconque des revendications 1 à 21, dans lequel R₃ est choisi parmi le groupe N-acétylalaninoyle; sérinoyle; thréoninoyle; aspartoyle ; glutamoyle ; N-N-diméthylaminocarbonyle ; N,N-diéthylaminocarbonyle ; N-(1,3-diméthyl)butylaminocarbonyle ; N-méthyl-N-éthylaminocarbonyle; N-méthylacétyle ; 2-N-acétylaminoacétyle; 2-N-acétylaminopropanoyle; 2-N-(2-méthylpropanoyl)aminoacétyle ; 2-amino-3-hydroxypropanoyle; 2-amino-3-hydroxybutanoyle ; 2-hydroxyacétylaminoacétyle; diméthylaminosulfonyle; hydrogène ; hydroxyle ; méthoxy ; acétoxy ; méthyle ; éthyle ; propyle ; 2-propyle ; 2,2-diméthyléthyle ; allyle ; propynyle; allyloxycarbonyle; acétyle, propionyle; isobutyryle ; aminométhyle; CONH₂; aminoacétyle; aminopropionyle ; 2-aminopropionyle ; aminocarbonylacétyle ; hydroxyméthyle ; 1-hydroxyéthyle ; carboxy ; 2-hydroxyacétyle ; 2-hydroxypropanoyle ; méthoxyacétyle ; méthoxyméthyle ; méthoxycarbonylméthyle ; méthoxycarbonyle ; éthoxycarbonyle ; formylamino ; acétylamino ; amino ; chloro ; cyano ; nitro ; thiol ; méthylthio ; méthylsulfonyle ; éthylsulfonyle ; méthylsulfényle ; et hydrazido.

23. Composé tel que revendiqué dans l'une quelconque des revendications 2 à 22, dans lequel R₃ est choisi parmi un groupe N-acétylalaninoyle ; sérinoyle ; thréoninoyle ; aspartoyle ; glutamoyle ; N-(1,3-diméthyl)butylaminocarbonyle ; N-méthyl-N-éthylaminocarbonyle ; N-méthylacétyle ; 2-N-acétylaminoacétyle ; 2-N-acétylaminopropanoyle ; 2-N-(2-méthylpropanoyl)aminoacétyle ; 2-amino-3-hydroxypropanoyle ; 2-amino-3-hydroxybutanoyle ; 2-hydroxyacétylaminoacétyle ; diméthylaminosulfonyle ; hydrogène ; hydroxyle ; méthoxy ; acétoxy ; méthyle ; éthyle ; propyle ; 2-propyle ; 2,2-diméthyléthyle ; allyle ; propynyle ; allyloxycarbonyle ; acétyle ; propionyle ; isobutyryle ; aminométhyle ; CONH₂ ; aminoacétyle ; aminopropionyle ; 2-aminopropionyle ; aminocarbonylacétyle ; hydroxyméthyle ; 1-hydroxyéthyle ; carboxy ; 2-hydroxyacétyle ; 2-hydroxypropanoyle ; méthoxyméthyle ; méthoxycarbonylméthyle ; méthoxycarbonyle ; éthoxycarbonyle ; formylamino ; acétylamino ; amino ; chloro ; cyano ; nitro ; thiol ; méthylthio ; méthylsulfonyle ; éthylsulfonyle ; méthylsulfényle ; et hydrazido.

24. Composé tel que revendiqué dans l'une quelconque des revendications 1 à 23, dans lequel L représente CONR_{1d} et Lp représente

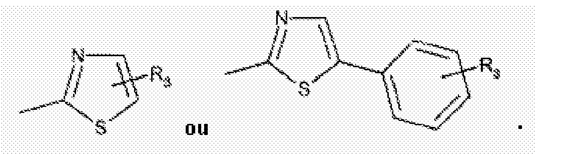

25. Composé tel que revendiqué dans la revendication 24, dans lequel chaque R₃ est indépendamment choisi parmi un atome d'hydrogène, un groupe amino, hydroxy, (C₁-C₆)alkyle, (C₁-C₆)alcanoyle, (C₁-C₆)alcanoyloxy, (C₁-C₆)alcoxycarbonyl(C₁-C₆)alkyle, amino(C₁-C₆)alkyle et cyano.

26. Composé tel que revendiqué dans l'une quelconque des revendications 1 à 24, dans lequel L représente CONR_{1d} et Lp représente 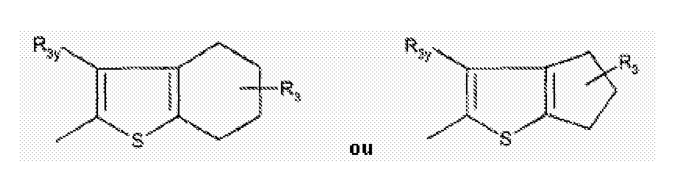 dans lesquelles R_{3y} représente R₃ ou un groupe de formule
Rₖ-G₂-Xₐ-
dans laquelle G₂ représente une liaison ou un groupe (C₁-C₃)alcanediyle, Xₐ représente une liaison, CO, OCO, COO ou NHCO, et Rₖ représente un groupe carbocyclique ou hétérocyclique, éventuellement substitué par R₃.

27. Composé tel que revendiqué dans la revendication 26, dans lequel Lp est choisi parmi 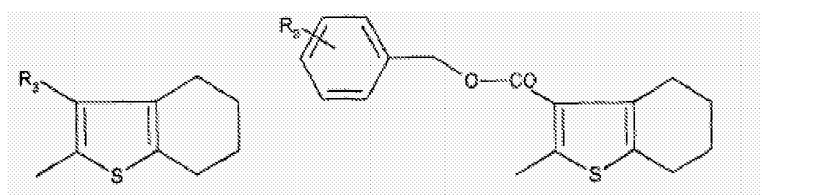 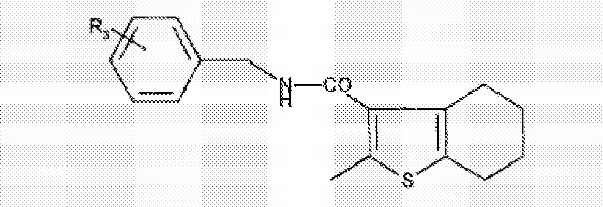 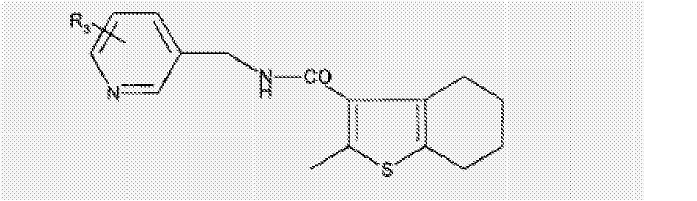 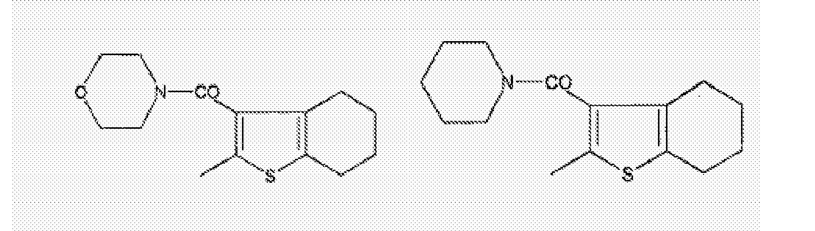 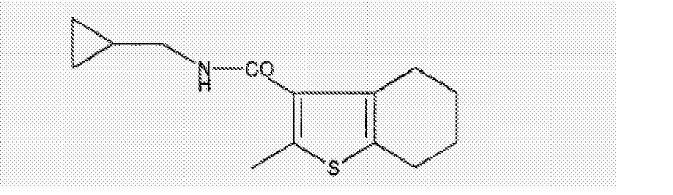 dans lesquelles (i) lorsque R₃ est présent comme substituant à la position 3 d'un groupe 4,5,6,7-tétrahydrobenzothiophène, il représente un groupe carboxy ; un groupe (C₁-C₆)alcoxycarbonyle; ou un groupe (Ci-C₆)alkylaminocarbonyle ; et (ii) lorsque R₃ est présent comme substituant sur un groupe phényle ou pyridyle, il représente un atome d'hydrogène.

28. Composé tel que revendiqué dans l'une quelconque des revendications 1 à 24, dans lequel L représente CONR_{1d} et Lp représente

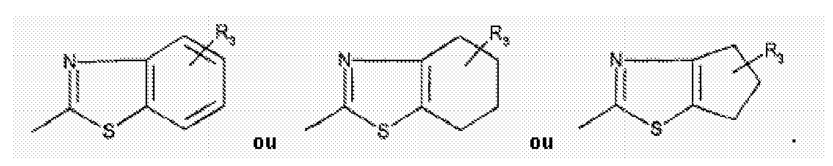

29. Composé tel que revendiqué dans la revendication 28, dans lequel le groupe hétérocyclique est substitué par un ou deux groupe(s) R₃.

30. Composé tel que revendiqué dans la revendication 29, dans lequel chaque groupe R₃ est choisi parmi un atome d'hydrogène, un atome d'halogène, un groupe (C₁-C₆)alkyle et un groupe (C₁-C₆)alcoxy.

31. Composé tel que revendiqué dans l'une quelconque des revendications 1 à 24, dans lequel L représente CONR_{1d} et Lp représente 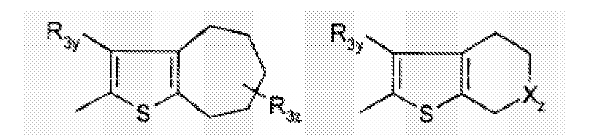 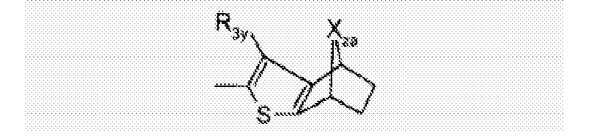 dans lesquelles R_{3y}, R_{3z}, X_{z} et X_{za} sont tels que définis ci-dessus.

32. Composé tel que revendiqué dans la revendication 31, dans lequel R_{3y} représente un groupe (C₁-C₆)alcoxycarbonyle, N,N-dialkylaminocarbonyle ou cyano ; R_{3z} représente un atome d'hydrogène ; X_{z} représente O, S ou NR_{z}, R_{z} représente un atome d'hydrogène, un groupe (C₁-C₆)alcanoyle, amino(C₁-C₆)alcanoyle, (C₁-C₆)alcoxy(C₁-C₆)alcanoyle ou benzyloxycarbonyle ; et X_{za} représente CH₂.

33. Composé tel que revendiqué dans la revendication 32, dans lequel Lp représente un groupe 3-éthoxycarbonyl-tétrahydro-4H-cyclohepta(b)thièn-2-yle, 3-éthoxycarbonyl-4,5-dihydro-5H-thiéno[2,3-c]pyranyle, 3-éthoxycarbonyl-4,5-dihydro-5H-thiéno[2,3-c]thiopyranyle, 3-diméthylaminocarbonyl-6-benzyloxycarbonyltétrahydrothiéno[2,3-b]pyridin-2-yle, 3-diméthylaminocarbonyl-tétrahydrothiéno[2,3-b]pyridin-2-yle, 3-diméthylaminocarbonyl-6-acétyltétrahydrothiéno[2,3-b]pyridin-2-yle, 3-diméthylaminocarbonyl-6-aminoacétyltétrahydrothiéno-[2,3-b]pyridin-2-yle, 3-diméthylaminocarbonyl-6-méthoxyacétyl-tétrahydrothiéno[2,3-b]pyridin-2-yle ou 3-éthoxycarbonyl-tétrahydro-4,7-méthanobenzo(b)thiophèn-2-yle.

34. Composé tel que revendiqué dans l'une quelconque des revendications 1 à 33, dans lequel L représente CONH et Lp représente 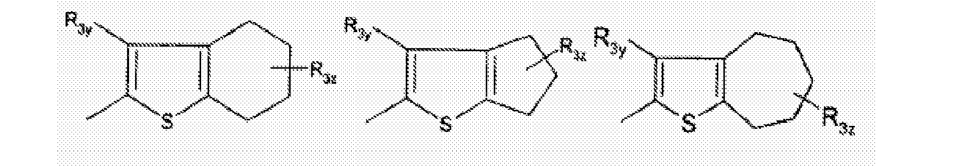 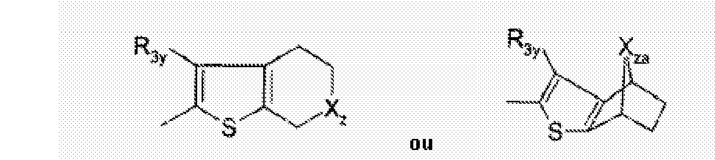 dans lesquelles R_{3y} est choisi parmi un groupe N,N-di-(C₁-C₆)alkylaminocarbonyle, di(C₁-C₆)alkylaminosulfonyle, (C₃-C₆)alcényloxycarbonyle, (C₁-C₆)alcanoyle, hydroxy(C₁-C₆)alcanoyle, (Ci-C₆)alcoxy(C₁-C₆)alcanoyle, (C₁-C₆)alcoxycarbonyle, (C₁-C₆)alkylsulfonyle, (C₁-C₆)alkylsulfényle, et Rₖ-G₂-Xₐ dans laquelle soit Xₐ représente CO, OCO, NR_{L}CO, (où R_{L} représente un groupe (C₁-C₆)alkyle), SO₂ ou NR_{L}SO₂ et Rₖ et G₂ sont tels que définis précédemment, soit Xₐ et G₂ sont tous les deux absents et Rₖ représente un groupe pyrid-2-yle, thiazol-2-yle, thiazol-4-yle, pyrazin-2-yle, pyrazin-3-yle, pyrimidin-2-yle ou pyrimidin-4-yle.

35. Composition pharmaceutique, qui comprend un composé tel que revendiqué dans l'une quelconque des revendications 1 à 34 conjointement à au moins un support ou excipient pharmaceutiquement acceptable.

36. Composé tel que revendiqué dans l'une quelconque des revendications 1 à 34 pour l'utilisation en thérapie.

37. Utilisation d'un composé tel que revendiqué dans l'une quelconque des revendications 1 à 34, dans la fabrication d'un médicament pour l'utilisation dans le traitement du corps humain ou non humain pour combattre un état sensible à un inhibiteur de la tryptase.

38. Utilisation telle que revendiquée dans la revendication 37, dans laquelle ledit état est choisi parmi l'asthme, la rhinite allergique, l'eczéma, le psoriasis, la dermatite atopique, l'urticaire, l'arthrite rhumatoïde, la conjonctivite, l'affection intestinale inflammatoire, l'inflammation neurogène, l'athérosclérose et le cancer.

39. Utilisation telle que revendiquée dans la revendication 38, dans laquelle ledit état est l'asthme.

40. Composé de formule (IX)
Z₂-CH(Cy)-L-Lp (IX)
dans laquelle Cy, L et Lp sont tels que définis dans l'une quelconque des revendications 1 à 34 et Z₂ représente NH₂ ou un sel de celui-ci mais en excluant les composés 4-méthyl-thiazol-2-amide de phénylglycine et 5-méthylthiazol-2-yl-méthylamide de phénylglycine.
